# EUROPEAN PATENT SPECIFICATION

(11) **EP 2 341 943 B1**
(45) Date of publication and mention of the grant of the patent: **07.11.2018**
(21) Application number: 09814902.4
(22) Date of filing: 17.09.2009
(51) Int. Cl.: C07H 21/02, A61K 31/713, C12N 15/113

(54) **COMPOSITIONS AND METHODS FOR THE SPECIFIC INHIBITION OF GENE EXPRESSION BY DSRNA POSSESSING MODIFICATIONS**
ZUSAMMENSETZUNGEN UND VERFAHREN ZUR SPEZIFISCHEN HEMMUNG DER GENEXPRESSION DURCH DSRNA-BESITZENDE MODIFIKATIONEN
COMPOSITIONS ET PROCÉDÉS POUR INHIBER SPÉCIFIQUEMENT DE L'EXPRESSION D'UN GÈNE PAR MODIFICATIONS DU TRAITEMENT DE L'ARNDS

(30) Priority: 22.09.2008 US 136741 P; 22.09.2008 US 136736 P
(43) Date of publication of application: 13.07.2011
(73) Proprietor: Dicerna Pharmaceuticals, Inc., Cambridge, MA 02140 (US)
(72) Inventor: BROWN, Bob, Littleton ,MA 01460 (US)
(74) Representative: Soames, Candida Jane
(86) International application number: PCT/US2009/005214
(87) International publication number: WO 2010/033225

(56) References cited:
- WO-A1-2008/049078
- US-A1- 2006 009 402
- US-A1- 2006 009 402
- SCHNEIDER C. ET AL.: "A molecular dynamics simulation study of coaxial stacking in RNA", JOURNAL OF BIOMOLECULAR STRUCTURE AND DYNAMICS, vol. 18, no. 3, December 2000 (2000-12), pages 345-352, XP008161347, ISSN: 0739-1102
- VLASSOV A.V. ET AL.: "shRNAs targeting Hepatitis C: effects of sequence and structural features, and comparision with siRNA", OLIGONUCLEOTIDES, vol. 17, no. 2, 2007, pages 223-236, XP055035981, ISSN: 1545-4576, DOI: 10.1089/oli.2006.0069
- ZENG Y. AND CULLEN B.R.: "Structural requirements for pre-MicroRNA binding and nuclear export by Exportin 5", NUCLEIC ACIDS RESEARCH, OXFORD UNIVERSITY PRESS, SURREY, GB, vol. 32, no. 16, 2004, pages 4776-4785, XP002989681, ISSN: 0305-1048, DOI: 10.1093/NAR/GKH824
- NAGEL R ET AL: "Substrate recognition by a eukaryotic RNase III: the double-stranded RNA-binding domain of Rnt1p selectively binds RNA containing a 5'-AGNN-3' tetraloop", RNA, COLD SPRING HARBOR LABORATORY PRESS, US, vol. 6, no. 8, 2000, pages 1142-1156, XP008150370, ISSN: 1355-8382
- PAOLO FRUSCOLONI ET AL: "Exonucleolytic degradation of double-stranded RNA by an activity in Xenopus laevis germinal vesicles", PROCEEDINGS OF THE NATIONAL ACADEMY OF SCIENCES, NATIONAL ACADEMY OF SCIENCES, US, vol. 100, no. 4, 18 February 2003 (2003-02-18), pages 1639-1644, XP008150373, ISSN: 0027-8424, DOI: 10.1073/PNAS.252777499 [retrieved on 2003-02-10]
- HAIHONG WU ET AL: "Structural basis for recognition of the AGNN tetraloop RNA fold by the double-stranded RNA-binding domain of Rnt1p RNase III", PROCEEDINGS OF THE NATIONAL ACADEMY OF SCIENCES, NATIONAL ACADEMY OF SCIENCES, US, vol. 101, no. 22, 2004, pages 8307-8312, XP008150375, ISSN: 0027-8424, DOI: 10.1073/PNAS.0402627101 [retrieved on 2004-05-18]
- HARBORTH J ET AL: "Sequence, chemical, and structural variation of small interfering RNAs and short hairpin RNAs and the effect on mammalian gene silencing", ANTISENSE & NUCLEIC ACID DRUG DEVELOPMENT, MARY ANN LIEBERT, INC., NEW YORK, US, vol. 13, no. 2, 2003, pages 83-105, XP002284355, ISSN: 1087-2906, DOI: 10.1089/108729003321629638
- LEUSCHNER P J F ET AL: "Cleavage of the siRNA passenger strand during RISC assembly in human cells", EMBO REPORTS, XX, XX, vol. 7, no. 3, 2006, pages 314-320, XP002467845,
- MATRANGA ET AL.: 'Passenger-Strand Cleavage Facilitates Assembly of siRNA into Ago2- Containing RNAi Enzyme Complexes.' CELL, [Online] vol. 123, no. 4, 18 November 2005, pages 607 - 620, XP002484663 Retrieved from the Internet: <URL:<http://download.cell.com/mmcs/journal s/0092-8674/PI IS0092867405009220.mmc1.pdf>).>
- NAGEL ET AL.: 'Substrate recognition by a eukaryotic RNase III: the double-stranded RNA- binding domain of Rnt1p selectively binds RNA containing a 5'-AGNN-3' tetraloop.' RNA. vol. 6, no. 8, August 2000, pages 1142 - 1156, XP008150370
- FRUSCOLONI ET AL.: 'Exonucleolytic degradation of double-stranded RNA by an activity in Xenopus laevis germinal vesicles.' PNAS vol. 100, no. 4, 18 February 2003, pages 1639 - 1644, XP008150373
- WU ET AL.: 'Structural basis for recognition of the AGNN tetraloop RNA fold by the double- stranded RNA-binding domain of Rnt1p RNase III.' PNAS vol. 101, no. 22, 01 June 2004, pages 8307 - 8312, XP008150375
- LEUSCHNER ET AL.: 'Cleavage of the siRNA passenger strand during RISC assembly in human cells.' EMBO REPORTS vol. 7, no. 3, March 2006, pages 314 - 320, XP002467845
- HARBORTH ET AL.: 'Sequence, Chemical, and Structural Variation of Small Interfering RNAs and Short Hairpin RNAs and the Effect on Mammalian Gene Silencing.' ANTISENSE NUCLEIC ACID DRUG DEV. vol. 13, no. 2, April 2003, pages 83 - 105, XP002284355

## Description

### CROSS-REFERENCE TO RELATED APPLICATIONS

The present application is related to and claims priority under 35 U.S. C. §1 19(e) to U.S. provisional patent application No. 61/136,736, filed September 22, 2008, and to provisional patent application No. 61/136,741, filed September 22, 2008.

### BACKGROUND OF THE INVENTION

Suppression of gene expression by double-stranded RNA (dsRNA) has been demonstrated in a variety of systems including plants (post-transcriptional gene suppression; Napoli et al., 1990), fungi (quelling; Romano and Marcino, 1992), and nematodes (RNA interference; Fire et al., 1998). Double-stranded RNA (dsRNA) is significantly more stable than single-stranded RNA (ssRNA). For example, WO2007107162 to Wengel et al. describes a dsRNA. The difference in the stability of dsRNA and ssRNA is pronounced in the intracellular environment (Raemdonck et al., 2006). However, unmodified siRNAs are rapidly degraded in serum, which is a fairly nuclease rich environment. Chemical modification can significantly stabilize the siRNA and improve potency both in vitro and in vivo. Extensive medicinal chemistry has been done over the past 20 years for applications where synthetic nucleic acids are used for experimental or therapeutic applications in vivo, such as in the antisense and ribozyme fields, and hundreds of compounds have been tested in a search for modifications that improve nuclease stability, increase binding affinity, and sometimes also improve pharmacodynamic properties of synthetic nucleic acids (Matteucci, 1997; Manoharan, 2002; Kurreck, 2003). Many of these modifications have already been tested and found to have utility as modifiers for use in traditional 21mer siRNAs. Several reviews have provided summaries of recent experience with 21mer siRNAs and chemical modifications (Zhang et al., 2006; Nawrot and Sipa, 2006; Rana, 2007). Modification patterns have also been tested or optimized for use in longer RNAs, such as Dicer-substrate siRNAs (DsiRNAs; Collingwood et al., 2008).

The invention provides compositions useful in RNAi for inhibiting gene expression and provides methods for their use. In addition, the invention provides RNAi compositions and methods designed to maximize potency, enhance Dicer processing, improve stability while evading the immune system and are not toxic. Additionally, various embodiments of the invention are suited for high throughput, small scale synthesis to meet research needs as well as large scale manufacturing for therapeutic applications. These and other advantages of the invention, as well as additional inventive features, will be apparent from the description of the invention provided herein.

WO2008/049078 directed to meroduplex RNA molecules that contain at least three nucleotide strands, the molecules comprising a nick or a gap.

### SUMMARY OF THE INVENTION

As described below, the present invention features compositions for inhibiting the expression or activity of a gene in a mammalian cell. The composition is defined in claim 1 with optional features being provided in the claims dependent from claim 1.

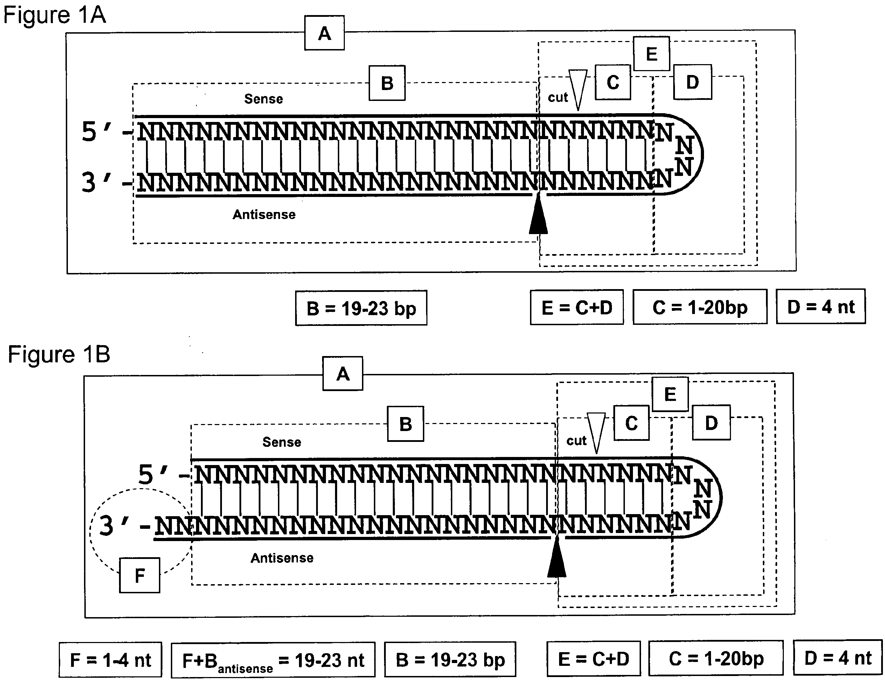

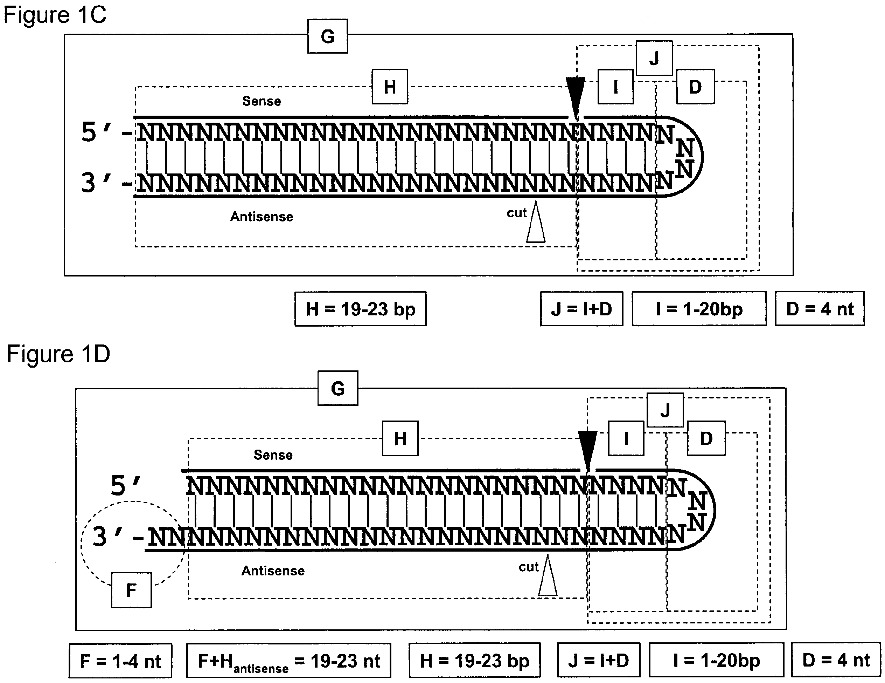

The invention provides an isolated double stranded RNA (dsRNA) containing a sense strand and an antisense strand, where the sense and antisense strands form a duplex in Region B; the sense strand contains a Region E at the 3' terminus and the Region E contains a tetraloop; and the dsRNA contains a discontinuity between the 3' terminus of the sense strand and the 5' terminus of the antisense strand (see Figure 1).

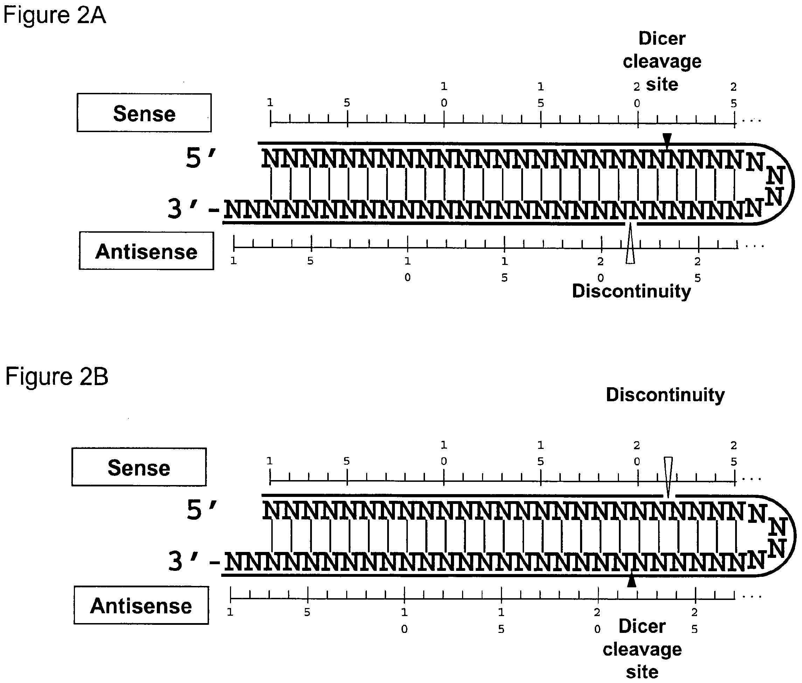

In another aspect, the invention provides an isolated double stranded RNA (dsRNA) containing a sense strand, and an antisense strand where the sense and antisense strands form a duplex in Region H; the antisense strand contains a Region J at the 5' terminus and the loop in the Region J contains a tetraloop; and the dsRNA contains a discontinuity between the 3' terminus of the sense strand and the 5' terminus of the antisense strand (see Figure 2).

The composition may be used for reducing expression of a target gene in a cell, involving contacting a cell with an isolated double stranded RNA (dsRNA) in an amount effective to reduce expression of a target gene in a cell in comparison to a reference dsRNA, where the dsRNA contains a sense strand and an antisense strand; the sense and antisense strands form a duplex in Region B; the sense strand contains a Region E at the 3 ' terminus and the Region E contains a tetraloop; the dsRNA contains a discontinuity between the 3' terminus of the sense strand and the 5' terminus of the antisense strand; and the antisense strand duplexes to a target RNA along at least 19 nucleotides of the length of the antisense strand.

The invention may provide a pharmaceutical composition for reducing expression of a target gene in a cell of a subject containing an isolated double stranded RNA (dsRNA) in an amount effective to reduce expression of a target gene in a cell in comparison to a reference dsRNA, and a pharmaceutically acceptable carrier, where the dsRNA contains a sense strand and an antisense strand; the sense and antisense strands form a duplex in Region B;
the sense strand contains a Region E at the 3 ' terminus and the Region E contains a tetraloop; the dsRNA contains a discontinuity between the 3' terminus of the sense strand and the 5' terminus of the antisense strand; and the antisense strand duplexes to a target RNA along at least 19 nucleotides of the length of the antisense strand.

In yet another aspect, the invention provides an isolated double stranded RNA (dsRNA) containing a first oligonucleotide strand that is 35-39 nucleotides in length, where nucleotides 1 1-16 from the 3' terminus form a duplex with nucleotides 1-6 from the 3' terminus and where nucleotides 7-10 from the 3' terminus form a tetraloop; and a second oligonucleotide strand that is 16 nucleotides shorter in length than the first oligonucleotide strand, and where all the nucleotides beginning from the 3 ' terminus of the second nucleotide strand form a duplex with the same number of nucleotides beginning at the 5' terminus of the first oligonucleotide strand.

In still another aspect, the invention provides an isolated double stranded RNA (dsRNA) containing a first oligonucleotide strand that is 37-41 nucleotides in length, where nucleotides 1 1-16 from the 3' terminus form a duplex with nucleotides 1-6 from the 3'
terminus and where nucleotides 7-10 from the 3' terminus form a tetraloop; and a second oligonucleotide strand that is 14 nucleotides shorter in length than the first oligonucleotide strand, and where all but the last 2 nucleotides from the 3' terminus of the second nucleotide strand form a duplex with the same number of nucleotides beginning at the 5' terminus of the first oligonucleotide strand.

In yet another aspect, the invention provides an isolated double stranded RNA (dsRNA) containing a first oligonucleotide strand that is 37-41 nucleotides in length, where nucleotides 1 1-16 from the 3' terminus form a duplex with nucleotides 1-6 from the 3' terminus and where nucleotides 7-10 from the 3' terminus form a tetraloop; and a second oligonucleotide strand that is 16 nucleotides shorter in length than the first oligonucleotide strand, and where all the nucleotides beginning from the 3' terminus of the second nucleotide strand form a duplex with the same number of nucleotides beginning at the 5' terminus of the first oligonucleotide strand.

In still another aspect, the invention provides an isolated double stranded RNA (dsRNA) containing a first oligonucleotide strand that is 37-41 nucleotides in length, where nucleotides 1 1-16 from the 3' terminus form a duplex with nucleotides 1-6 from the 3' terminus and where nucleotides 7-10 from the 3' terminus form a tetraloop; and a second oligonucleotide strand that is 18 nucleotides shorter in length than the first oligonucleotide strand, and where all but the last 2 nucleotides from the 3' terminus of the second nucleotide strand form a duplex with the same number of nucleotides beginning at the 5' terminus of the first oligonucleotide strand.

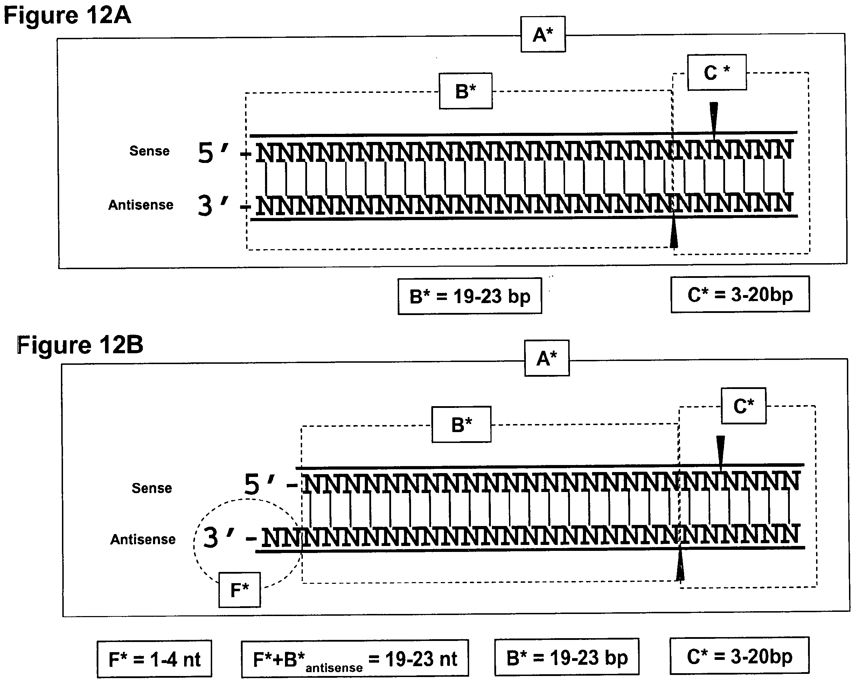

In an additional aspect, the disclosure provides an isolated double stranded RNA (dsRNA) containing a sense strand and an antisense strand, where the sense and antisense strands form a duplex that is 22-43 base pairs in length; and the dsRNA contains one or more modified nucleotides at any of positions B*1 on the sense strand, B* 1 on the antisense strand, C*1-C*3 on the sense strand, or C*1-C*3 on the antisense strand (see Figures 12-17).

The invention may provide a pharmaceutical composition for reducing expression of a target gene in a cell of a subject containing an isolated double stranded RNA in an amount effective to reduce expression of a target gene in a cell in comparison to a reference dsRNA and a pharmaceutically acceptable carrier, where the dsRNA contains a sense strand and an antisense strand; the sense and antisense strands form a duplex that is 22-43 base pairs in length; the dsRNA contains one or more modified nucleotides at any of positions B* 1 on the sense strand, B* 1 on the antisense strand, C*1-C*3 on the sense strand, or C*1-C*3 on the antisense strand; the antisense strand duplexes to a target RNA along at least 19 nucleotides of the length of the antisense strand.

The invention provides compositions and methods for the specific inhibition of gene expression. Embodiments of aspects of the invention are defined herein. Other features and advantages of the invention will be apparent from the detailed description, and from the claims.

### BRIEF DESCRIPTION OF THE DRAWINGS

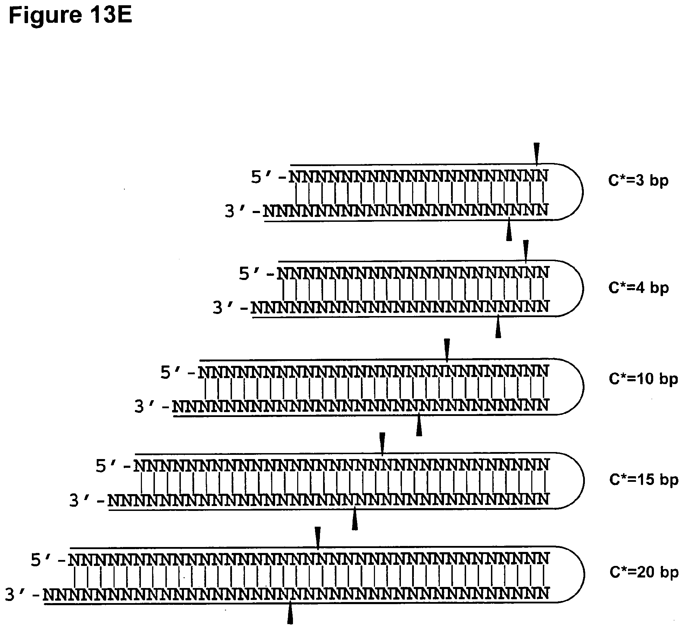

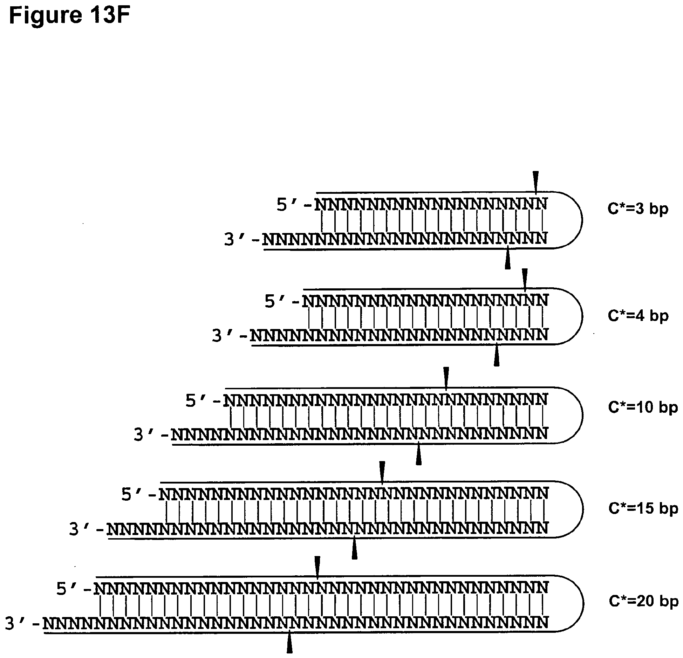

Figures IA, IB, 1C, and ID show the structures of "nicked dsRNAs" Figure IA shows a "nicked antisense" dsRNA containing a sense strand with an extended loop (Region E) at the 5' terminus, a duplex region formed by the antisense strand with the single stranded portion of the sense strand (Region B). When the dsRNA is duplexed, a discontinuity exists where Dicer cleaves the antisense strand (shown by black arrow). Figure 1B shows a "nicked antisense" dsRNA containing a sense strand with an extended loop (Region E) at the 5' terminus, a duplex region formed by the antisense strand with the single stranded portion of the sense strand (Region B), and a 3' overhang on the antisense strand (Region F, shown by dashed
   circle). When the dsRNA is duplexed, a discontinuity exists where Dicer cleaves the antisense strand (shown by black arrow). Figure 1C shows a "nicked sense" dsRNA containing a sense strand with an extended loop (Region J) at the 3' terminus, and a duplex region formed by the antisense strand with the single stranded portion of the sense strand (Region H). When the dsRNA is duplexed, a discontinuity exists where Dicer cleaves the sense strand (shown by black arrow). The nucleotide immediately proximal to the Dicer cleavage site must be a ribonucleotide (gray). Figure 1D shows a "nicked sense" dsRNA containing a sense strand with an extended loop (Regoin J) at the 3' terminus, and a duplex region formed by the antisense strand with the single stranded portion of the sense strand (Region H), and a 3' overhang on the antisense strand (Region F shown by dashed circle). When the dsRNA is duplexed, a discontinuity exists where Dicer cleaves the sense strand (black arrow). The nucleotide immediately proximal to the Dicer cleavage site must be a ribonucleotide (gray).
Figures 2A and 2B depict how positions of nucleotides are calculated from the 5' and 3' termini of the sense and antisense strands of the nicked dsRNAs of the invention. Figure 2A depicts how positions of nucleotides are calculated from the 5' and 3' termini of the sense and antisense strands when the sense strand has an extended loop with a tetraloop (i.e., the discontinuity is on the same side of the dsRNA as the antisense strand). The Dicer cleavage site on the sense strand is shown (short, black arrow). Figure 2B depicts how positions of nucleotides are calculated from the 5' and 3' termini of the sense and antisense strands when the antisense strand has an extended loop with a tetraloop (i.e., the discontinuity is on the same side of the dsRNA as the sense strand). The Dicer cleavage site on the antisense strand is shown (short, black arrow).

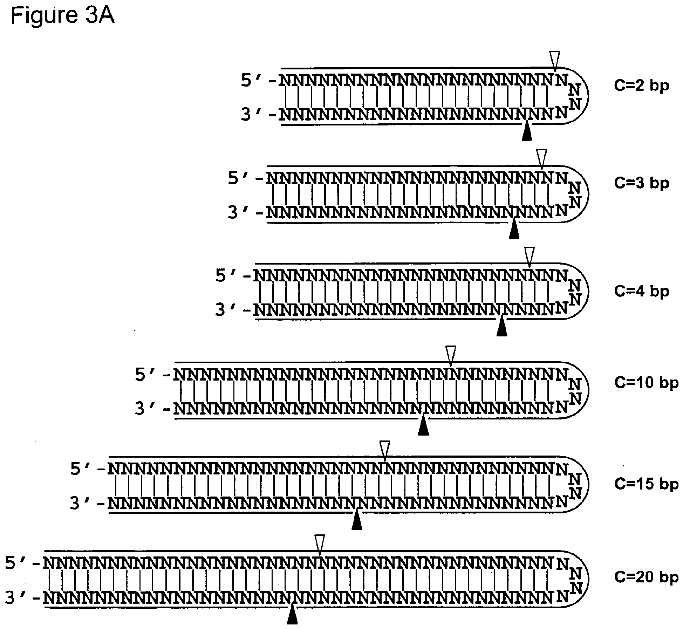

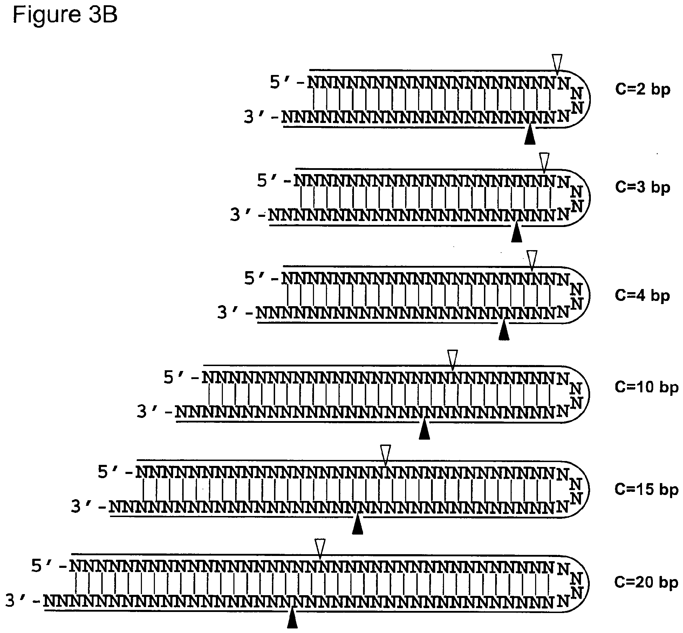

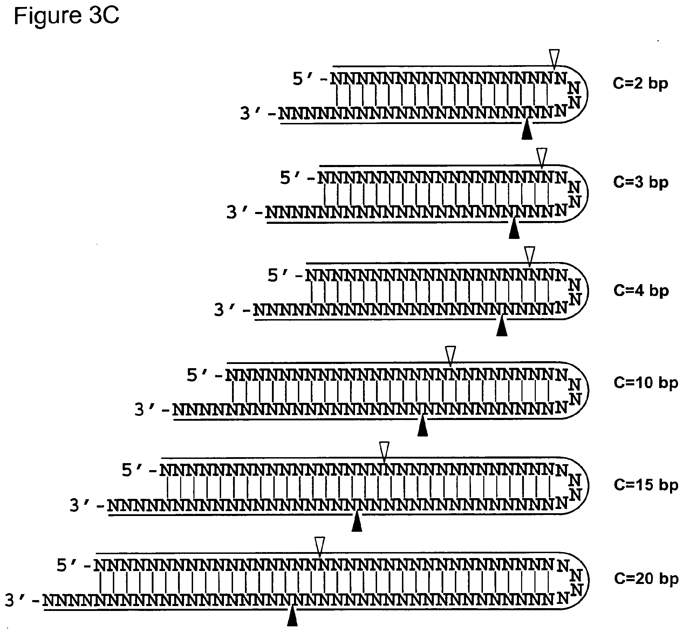

Figures 3A-3C depict examples of nicked dsRNAs of the invention where a discontinuity exists on the same side of the dsRNA molecule as the antisense strand, i.e., "nicked antisense dsRNAs". Figure 3A depicts examples of "nicked antisense" dsRNAs of the invention having a blunt end. Figure 3B depicts examples of "nicked antisense" dsRNAs of the invention having a 3' overhang of 2 nucleotides. Figure 3C depicts examples of "nicked antisense" dsRNAs of the invention having a 3' overhang of 4 nucleotides. When the dsRNA is duplexed, a discontinuity exists where Dicer cleaves the antisense strand (black arrow).

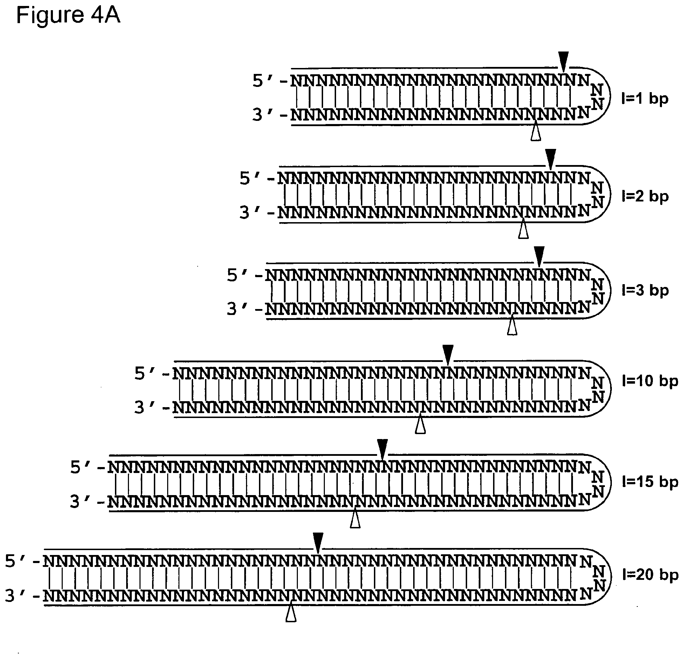

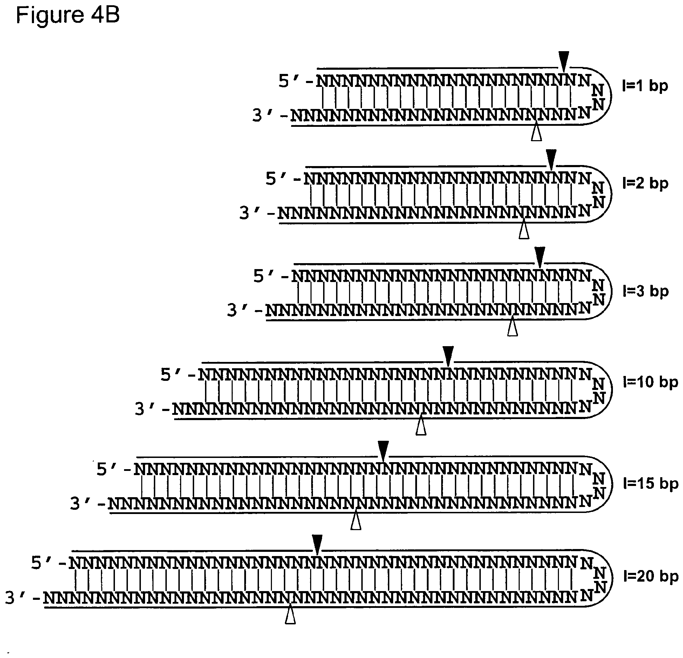

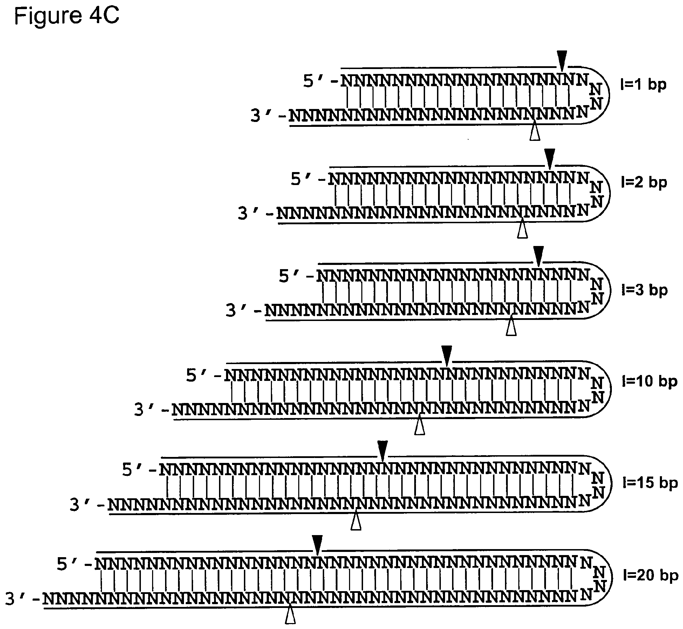

Figures 4A-4C depict examples of nicked dsRNAs where a discontinuity exists on the same side of the dsRNA molecule as the sense strand, i.e., "nicked sense dsRNAs". Figure 4A depicts examples of "nicked sense" dsRNAs having a blunt end. Figure 4B depicts examples of "nicked sense" dsRNAs having a 3' overhang of 2 nucleotides. Figure 4C depicts examples of "nicked sense" dsRNAs having a 3' overhang of 4 nucleotides. When the dsRNA is duplexed, a discontinuity exists where Dicer cleaves the sense strand (black arrow).

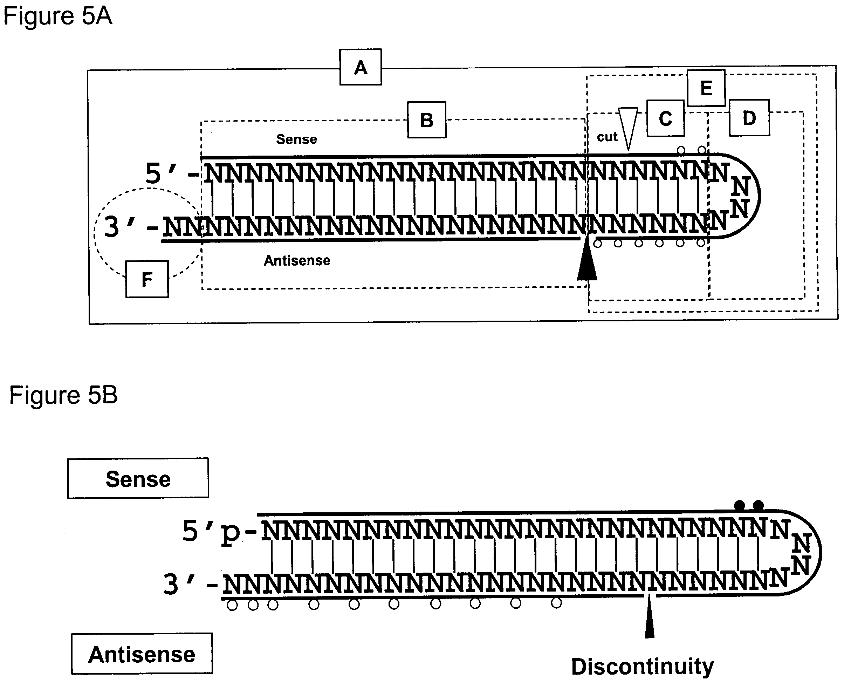

Figures 5A and 5B depict locations where modifications may be present in the nicked dsRNAs of the invention. Figure 5A depicts a dsRNA showing the positions of 2'-O-methyl modifications (shown by o) in Region C of the molecule. Figure 5B depicts a dsRNA in which the sense strand has an extended loop with a tetraloop (i.e., the discontinuity is on the same side of the dsRNA as the antisense strand; shown by black arrow). In the dsRNA depicted in Figure 5B the 5' terminus of the sense strand may be phosphorylated (shown by a "p-"); the 5' terminus of the guide strand may be dephosphorylated; the antisense strand may be modified at positions 1, 2, and 3 from the 3' terminus of the antisense strand with 2'-O-methyl (shown by ○); and the antisense strand may be modified at odd numbered positions starting at position 5 from the 3' terminus of the antisense strand with 2'-O-methyl (shown by ○). In the dsRNA depicted in Figure 5B, the sense strand may contain deoxyribonucleotides (shown by ●) at positions 11 and 12 from the 3' terminus of the antisense strand.

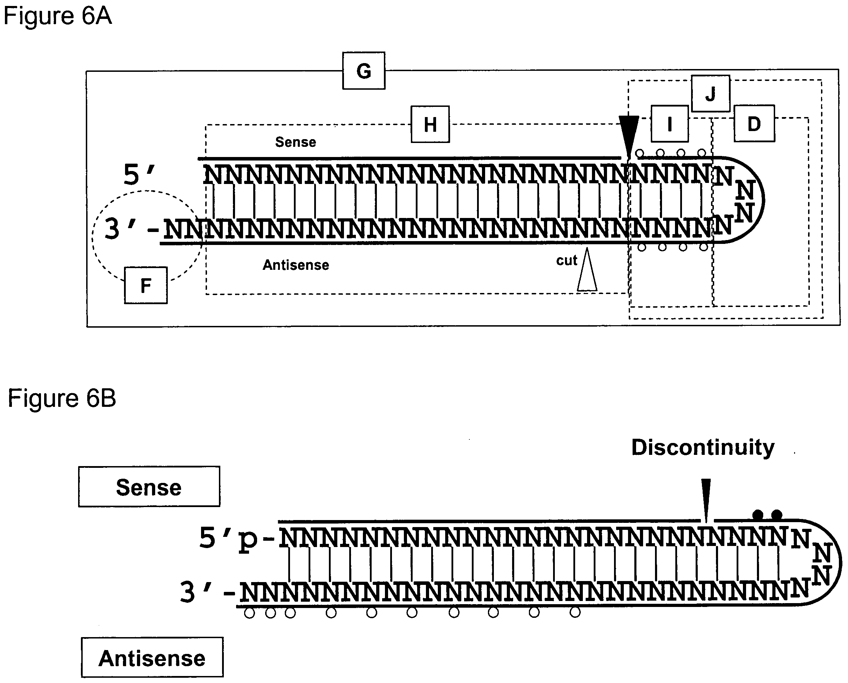

Figures 6A and 6B depict locations where modifications may be present in the nicked dsRNAs Figure 6A depicts a dsRNA showing the positions of 2'-O-methyl modifications (shown by ○) in Region C of the molecule. Figure 6B depicts a dsRNA in which the antisense strand has an extended loop with a tetraloop (i.e., the discontinuity is on the same side of the dsRNA as the sense strand; shown by black arrow). In the dsRNA depicted in Figures 4B the 5' terminus of the sense strand may be phosphorylated (shown by a "p-"); the 5' terminus of the guide strand may be dephosphorylated; the antisense strand may be modified at positions 1, 2, and 3 from the 3' terminus of the antisense strand with 2'-O-methyl (shown by ○); and the antisense strand may be modified at odd numbered positions starting at position 5 from the 3' terminus of the antisense strand with 2'-O-methyl (shown by ○). In the dsRNA depicted in Figure 6B, the antisense strand may contain deoxyribonucleotides (shown by ●) at positions 3 and 4 from the 5' terminus of the antisense strand.

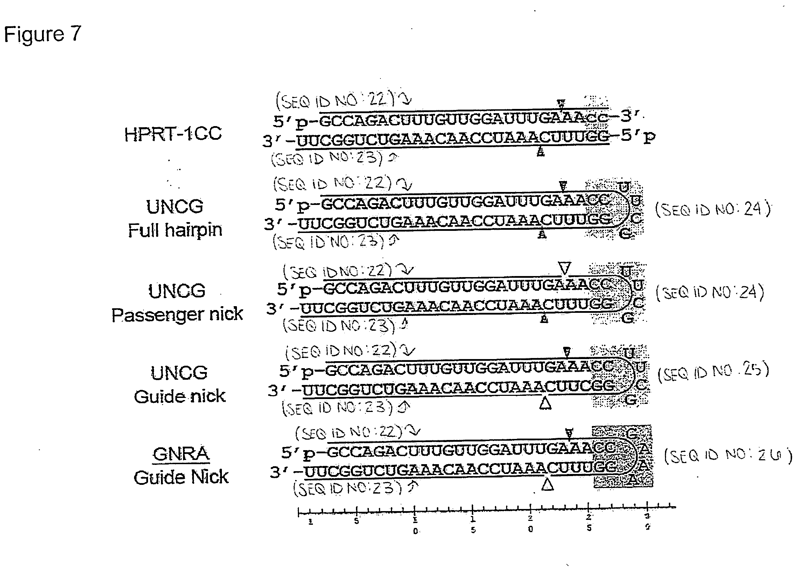

Figure 7 shows dsRNA constructs for use in experiments comparing the effect of a dsRNA with no tetraloop, the effect of a dsRNA with a tetraloop (i.e., UUCG), the effect of a dsRNA with a tetraloop (i.e., UUCG) in combination with a nick on the same side as the sense strand, the effect of a dsRNA with a tetraloop (i.e., UUCG) in combination with a nick on the same side as the antisense strand, and the effect of another tetraloop (i.e., GAAA) in combination with a nick on the same side as the antisense strand. The sequences in the dsRNAs are all based on human hypoxanthine phosphoribosyltransferase 1 (HPRT-1CC; NCBI database accession nos. NM_000194 and GI:164518913).

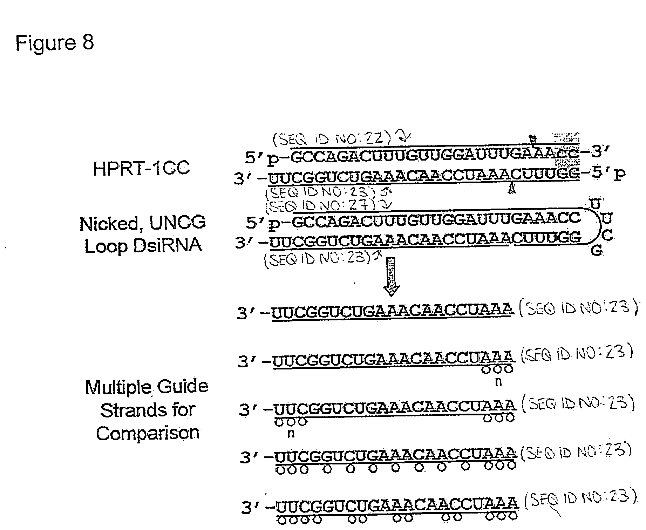

Figure 8 shows dsRNA constructs for use in experiments modifying the discontinuous antisense strand to determine their effects in the RNAi pathway at the step of Dicer processing and steps downstream of Dicer cleavage (e.g., Ago2 interaction, target recognition, Ago2 cleavage). The sequences in the dsRNAs are all based on human hypoxanthine phosphoribosyltransferase 1 (HPRT-1CC; NCBI database accession nos. NM_000194 and GI:164518913).

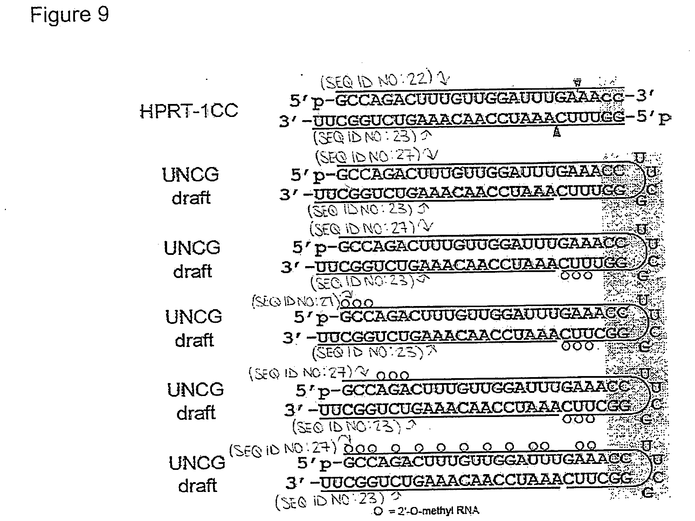

Figure 9 shows dsRNA constructs for use in experiments modifying the extended sense strand to determine their effects in the RNAi pathway at the step of Dicer processing and steps upstream of Dicer cleavage. The sequences in the dsRNAs are all based on human hypoxanthine phosphoribosyltransferase 1 (HPRT-1CC; NCBI database accession nos. NM_000194 and GI:164518913).

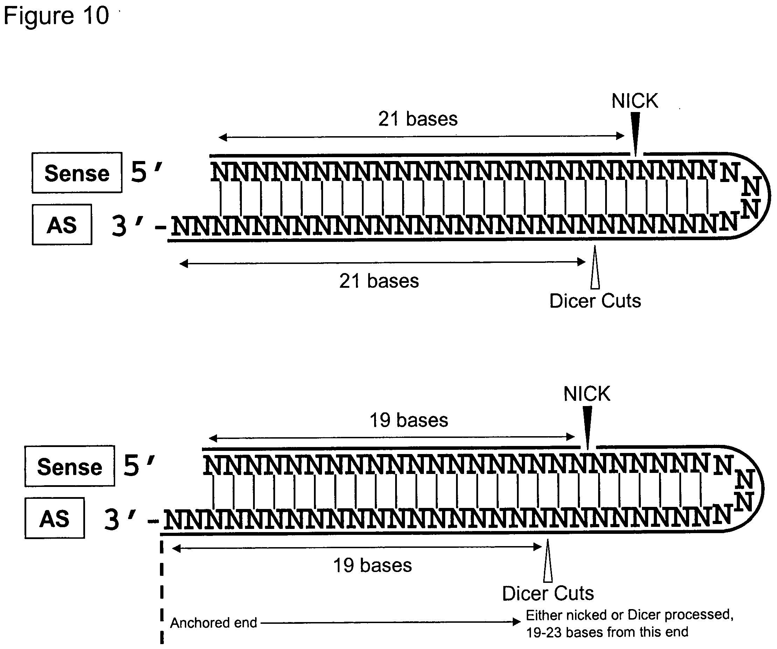

Figure 10 shows how the placement of a discontinuity defines cleavage products made by Dicer. The nicked dsRNA structure directs a unique cleavage product with the advantage that a defined 21 base antisense strand is produced and loaded into RISC. By moving the nick, production of other lengths of antisense strands are directed. Chemical modifications enforce the production of non-21 mer products.

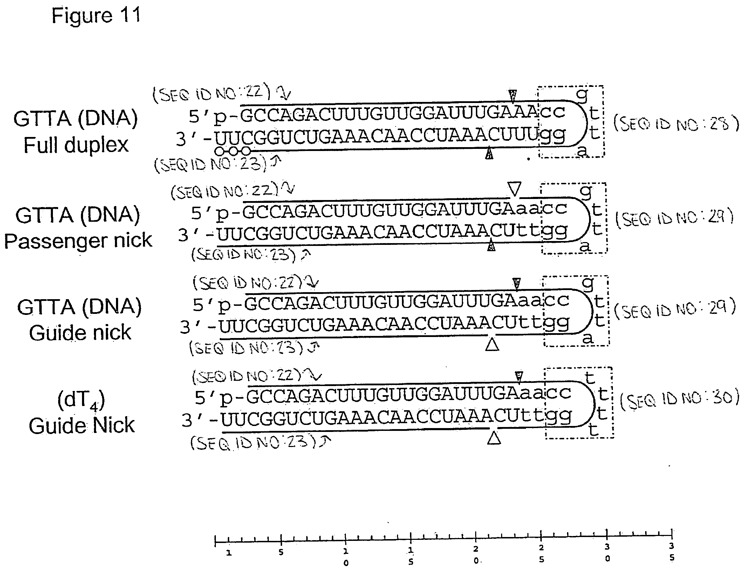

Figure 11 shows that DNA tetraloops and DNA ends control Dicer activity on the dsRNA. Double stranded RNA constructs are use in experiments comparing the effect of a dsRNA with no tetraloop, the effect of a dsRNA with a DNA tetraloop (i.e., d(GTTA)), the effect of a dsRNA with a DNA tetraloop (i.e., d(GTTA)) in combination with a nick on the same side as the sense strand, the effect of a dsRNA with a DNA tetraloop (i.e., d(GTTA)) in combination with a nick on the same side as the antisense strand, and the effect of another DNA tetraloop (i.e., d(TTTT)) in combination with a nick on the same side as the antisense strand. The sequences in the dsRNAs are all based on human hypoxanthine phosphoribosyltransferase 1 (HPRT-1CC; NCBI database accession nos. NM_000194 and GI:164518913).
Figures 12A and 12B depict the structure of a dsRNA of the invention in relation to its interaction with Dicer. Figure 12A depicts the structure of a dsRNA with a blunt end formed by the 5' terminus of the sense strand and the 3' terminus of the antisense strand. Figure 12B depictsithe structure of a dsRNA with a 3' overhang on the antisense strand. The nucleotide immediately proximal to the Dicer cleavage site must be a ribonucleotide (gray).

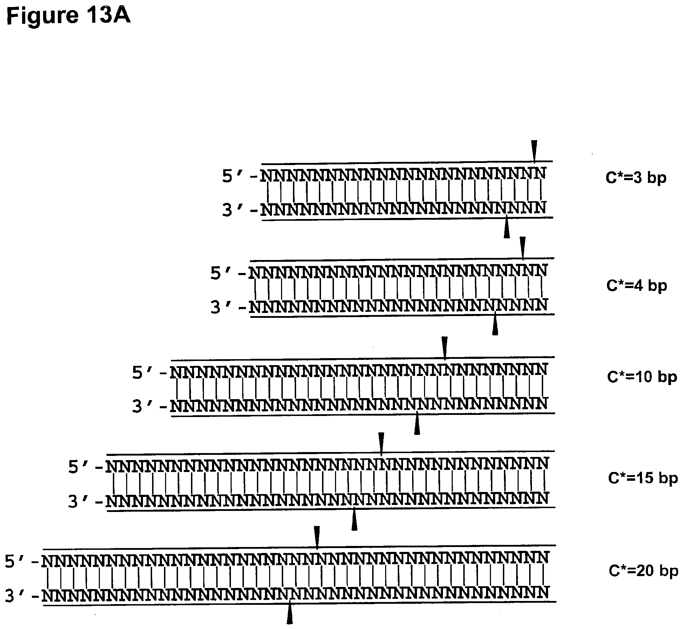

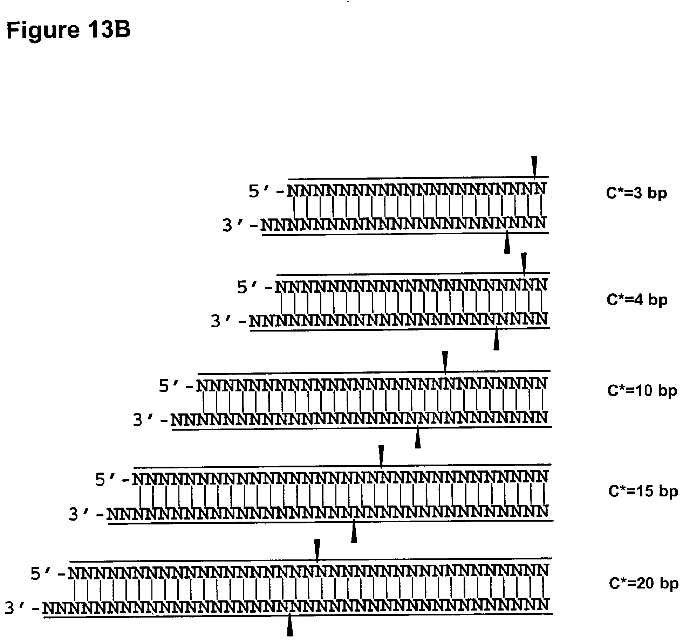

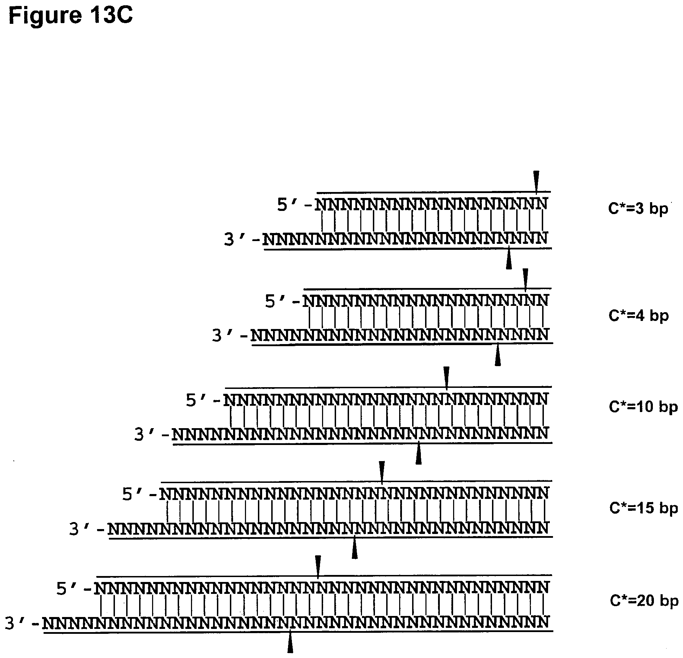

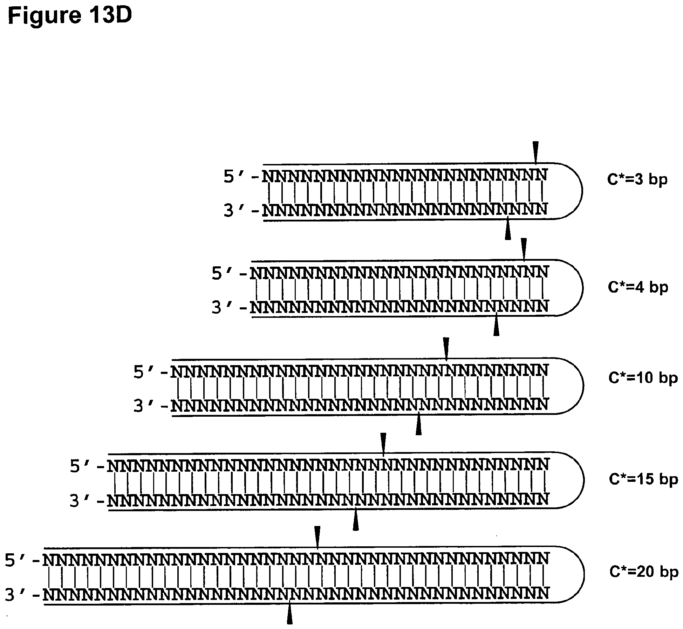

Figures 13A-13F depict examples of dsRNAs that possess modified or universal nucleotides. Figure 13A depicts examples of dsRNAs having two blunt ends. Figure 13B depicts examples of dsRNAs having a 3' overhang of 2 nucleotides on the antisense strand. Figure 13C depicts examples of dsRNAs having a 3' overhang of 4 nucleosides on the antisense strand. Figure 13D depicts examples of dsRNAs of the invention where
   the sense and antisense strands are connected by a linker and having a blunt end formed by the 5' terminus of the sense strand and the 3' terminus of the antisense strand. Figure 13E depicts examples of dsRNAs of the invention where the sense and antisense strands are connected by a linker and having a 3' overhang of 2 nucleotides on the antisense strand. Figure 13F depicts examples of dsRNAs of the invention where the sense and antisense strands are connected by a linker and having a 3' overhang of 4 nucleotides on the antisense strand. The positions occupied by nucleotides denoted by an "N" (gray) represent positions where universal nucleotides can be placed.

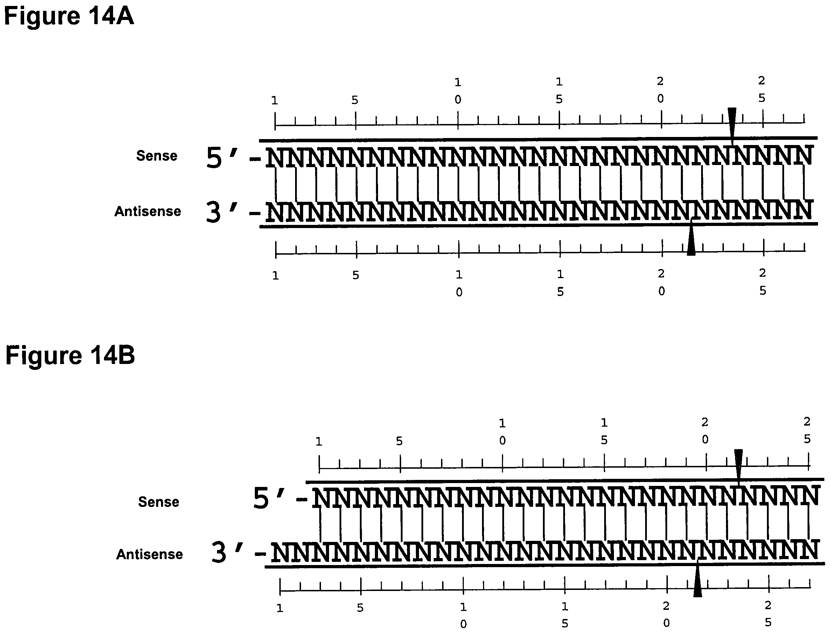

Figures 14A and 14B depict how positions of nucleotides are calculated from the 5' and 3' termini of the sense and antisense strands, respectively, of the dsRNAs that possess modified or universal nucleotides.

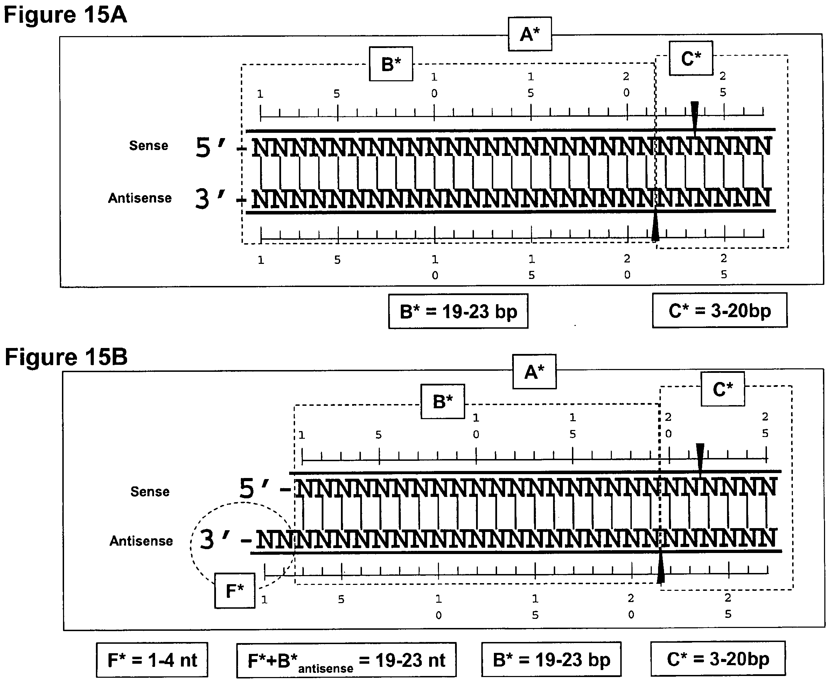

Figures 15A and 15B depict how-positions of nucleotides are calculated from the 5' and 3' termini of the sense and antisense strands, respectively, of the dsRNAs that possess modified or universal nucleotides.

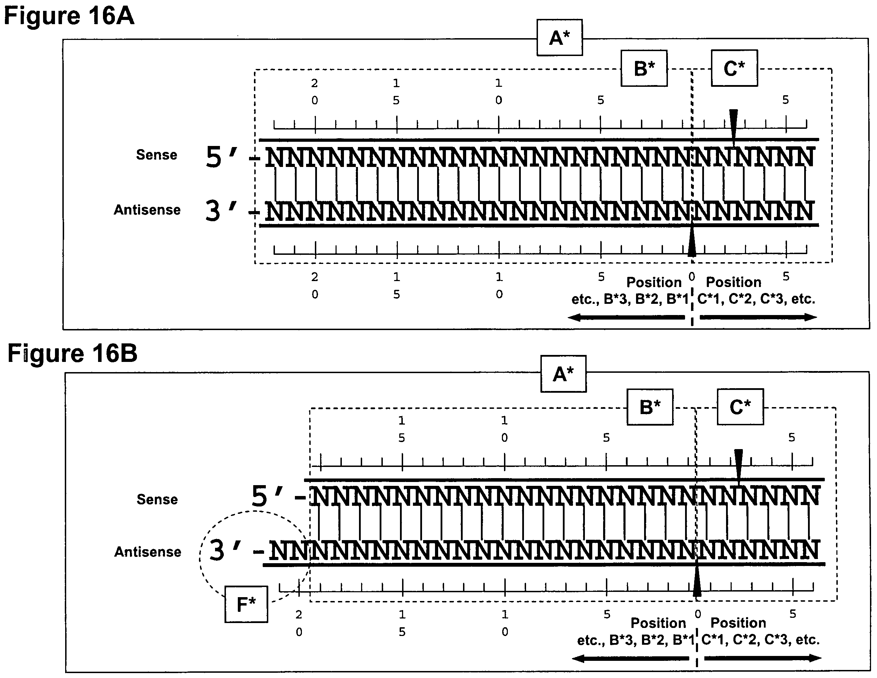

Figures 16A and 16B depict how positions of nucleotides are calculated from the 5' and 3' termini of the sense and antisense strands, respectively, of the dsRNAs that possess modified or universal nucleotides.

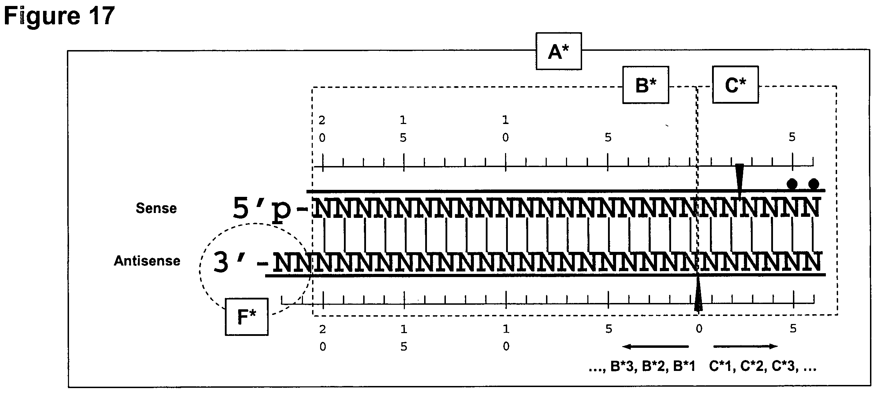

Figure 17 depicts locations where modifications may be present in the dsRNAs that possess modified or universal nucleotides. In the dsRNAs, the sense-strand contains modified nucleotides, including universal nucleotides, at positions B*1, and C*1-C*3 (shown in gray); and the antisense strand contains modified nucleotides, including universal nucleotides, at positions B*1, and C*1-C*3 (shown in gray). Additionally, in the dsRNAs the 5' terminus of the sense strand may be phosphorylated (shown by a "p-"); and the sense strand may contain deoxyribonucleotides (shown by ●) at positions 1 and 2 from the 3' terminus of the antisense strand. Dicer cleaves the dsRNA on the sense strand between positions C*2 and C*3 and on the antisense strand between positions B*1 and C*3 from the 5' terminus of the antisense strand (shown by long arrows).

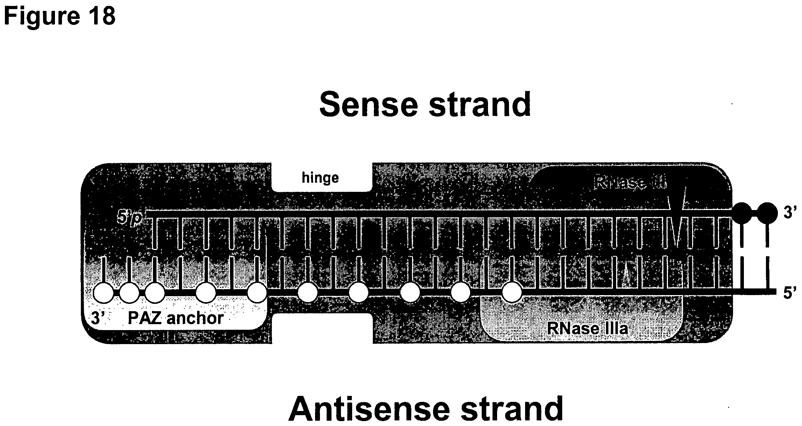

Figure 18 depicts the structure of a dsRNA in relation to its interaction with Dicer.

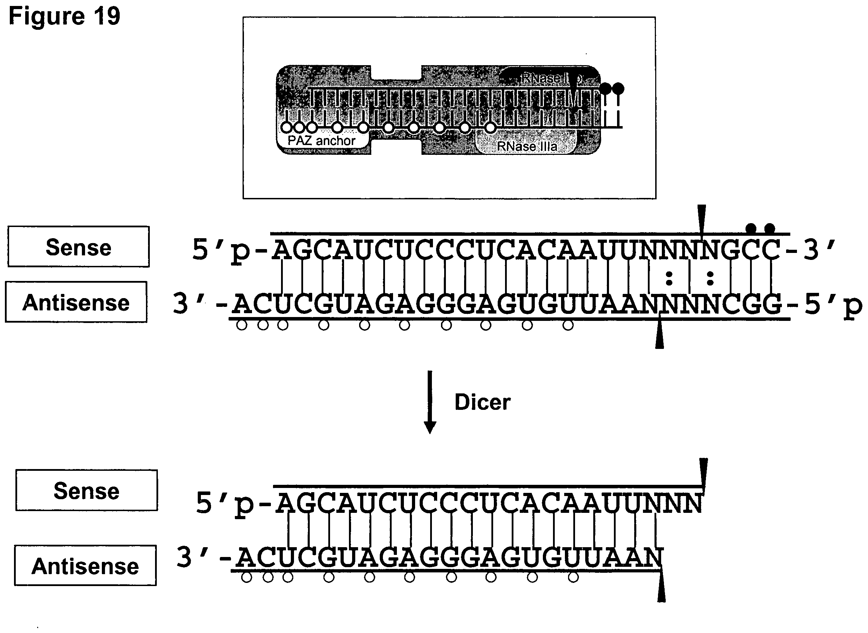

Figure 19 depicts enhanced Dicer cleavage of dsRNA substrates due to modified nucleotides, including universal nucleotides, located at positions B*1 and C*1-C*3 on the sense and antisense strands (shown by "N"); and/or due to modified nucleotides, including universal nucleotides, located on the antisense strand at positions 4-7 from the 5' terminus of the antisense strand (shown by "N"). Dicer cleaves the dsRNA between positions C*2 and C*3 on the sense strand and between positions B*1 and C*1 on the antisense strand (shown by long arrows). In the dsRNAs, the 5' terminus of the sense strand may be phosphorylated (shown by a "p-"); the antisense strand may be modified at positions 1, 2, and 3 from the 3' terminus of the antisense strand with 2'-O-methyl (shown by ○); the antisense strand may be modified at odd numbered positions starting at position 5 from the 3' terminus of the antisense strand with 2'-O-methyl (shown by ○); and the sense strand may contain deoxyribonucleotides (shown by ●) at positions 1 and 2 from the 3' terminus of the antisense strand. Dicer cleaves the dsRNA on the sense strand between positions 4 and 5 from the 3' terminus of the sense strand and on the antisense strand between positions 6 and 7 from the 5' terminus of the antisense strand (shown by long arrows). The inset depicts a dsRNA substrate in relation to its interaction with Dicer. Dicer cleavage of the dsRNA exposes the nucleotide at position C*2 on the sense strand and the nucleotide at position B*1 on the antisense strand. The sequence in the dsRNA is based on human hypoxanthine phosphoribosyltransferase 1 (HPRT-1CC; NCBI database accession nos. NM_000194 and GI:164518913). The positions occupied by nucleotides denoted by an "N" (gray) represent positions where universal nucleotides can be placed.

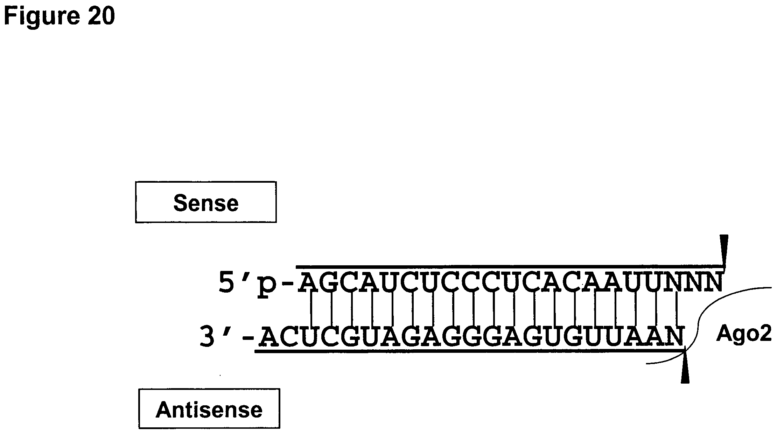

Figure 20 depicts enhanced Ago2 interaction with a dsRNA after Dicer cleavage exposes modified nucleotides in the dsRNA. After cleavage of the dsRNA, modified nucleotides, including universal bases, on the sense strand at positions B*1, C*1, and C*2 (positions 1-3 from the 3' terminus of the sense strand) or on the antisense strand at position B*1 (position 1 from the 5' terminus of the antisense strand) have increased interaction with Ago2. Dicer cleaves the dsRNA (shown by long arrows), thereby exposing internal modified nucleotides of the sense and antisense strands. The positions occupied by nucleotides denoted by an "N" (gray) represent positions where universal nucleotides can be placed.

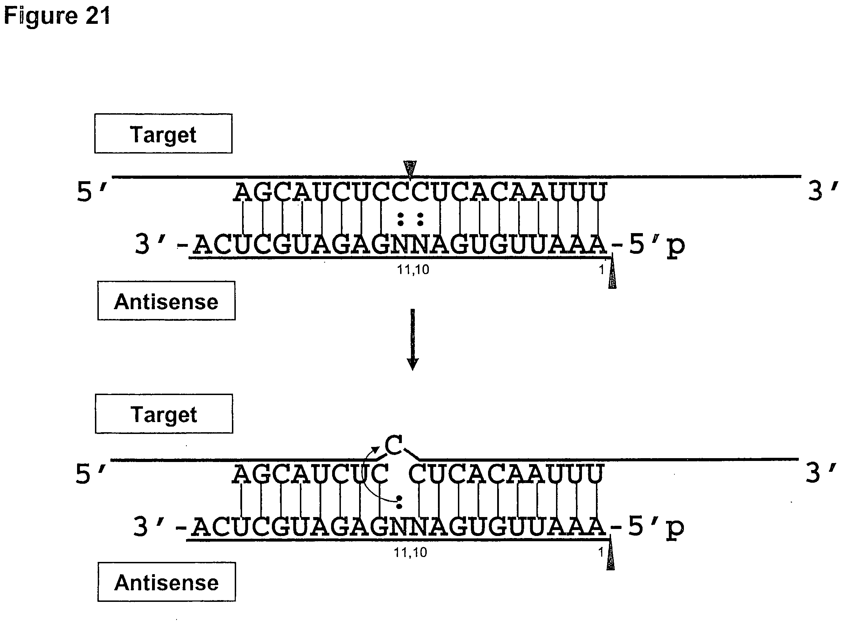

Figure 21 depicts enhanced Ago2 cleavage of target RNA by the RNase H domain when modified nucleotides, including universal bases, are present at positions 10 and 11 from the 3' terminus of the antisense strand created by Dicer cleavage of a dsRNA. Ago2 cleaves the target RNA in between the nucleotides opposite positions 10 and 11 from the 5' terminus of the antisense strand (shown by short arrow). Dicer cleaves the dsRNA (shown by long arrows), thereby exposing internal modified nucleotides of the sense and antisense strands. The positions occupied by nucleotides denoted by an "N" (gray) represent positions where universal nucleotides can be placed.

### DETAILED DESCRIPTION OF THE INVENTION

The invention provides compositions and methods for reducing expression of a target gene in a cell, involving contacting a cell with an isolated double stranded RNA (dsRNA) in an amount effective to reduce expression of a target gene in a cell. The dsRNAs of the invention possess modifications that are anticipated to alter dsRNA stability, efficacy and/or potency.

In certain aspects, the dsRNAs of the invention are referred to as "nicked dsRNAs," with such dsRNAs possessing a tetraloop and a discontinuity between the 3' terminus of the sense strand and the 5' terminus of the antisense strand at the Dicer cleavage site on either the guide strand or passenger strand. The nicked substrate permits increased Dicer cleavage of a dsRNA of the invention, as compared to a reference dsRNA. The nicked substrate also provides the ability to utilize more chemical modifications in dsRNAs (e.g., on a guide or passenger strand which has the nick).

In some aspects, the dsRNAs of the invention have modified or universal nucleotides on the sense or antisense strand at the Dicer cleavage site and modified or universal nucleotides on the antisense strand opposite to where the target strand is cleaved by Ago2/RISC. A modified or universal nucleotide at a Dicer site is expected to increase Dicer cleavage of a dsRNA of the invention, as compared to a reference dsRNA. Additionally, a modified or universal nucleotide at the Dicer site on the antisense strand exposed by Dicer processing of the dsRNA is expected to have increased interaction with Ago2, as compared to that of a reference dsRNA. A modified or universal nucleotide on the antisense strand opposite to where the target strand is cleaved by Ago2/RISC is expected to enhance cleavage of target RNA by Ago2/RISC, as compared to that of a reference dsRNA.

Examples of "nicked dsRNAs" of the invention are shown in Figures 7, 8, and 9, including control dsRNAs as a reference for comparison. Such dsRNAs of the invention possess a tetraloop and a discontinuity between the 3' terminus of the sense strand and the 5' terminus of the antisense strand at the Dicer cleavage site on either the guide strand or passenger strand. Such dsRNAs of the invention have the following structure: a sense strand and an antisense strand; the sense and antisense strands form a duplex in Region B; the sense strand contains a Region E at the 3' terminus and the Region E contains a tetraloop; the dsRNA contains a discontinuity between the 3' terminus of the sense strand and the 5' terminus of the antisense strand; and the antisense strand duplexes to a target RNA along at least 19 nucleotides of the length of the antisense strand. Alternatively, such dsRNAs have the following structure: a sense strand and an antisense strand; the sense and antisense strands form a duplex in Region H; the antisense strand contains a Region J at the 5' terminus and and the Region J contains a tetraloop; the dsRNA contains a discontinuity between the 3' terminus of the sense strand and the 5' terminus of the antisense strand; and the antisense strand duplexes to a target RNA along at least 19 nucleotides of the length of the antisense strand.

In an alternative aspect of the invention, a "nicked dsRNA" having a site of discontinuity that is displaced from the predicted site of Dicer cleavage is provided. Specifically, within the dsRNA of Figure 1A, the site of discontinuity may be shifted from its location at a predicted Dicer cleavage site to an alternative position within Region C. Optionally, the site of discontinuity within Region C remains 3' of the site at which Dicer is predicted to cleave the sense strand oligonucleotide.

A dsRNA that possesses modified or universal nucleotides is shown in Figure 19. Such dsRNAs have modified or universal nucleotides on the sense or antisense strand at the Dicer cleavage site and modified or universal nucleotides on the antisense strand opposite to where the target strand is cleaved by Ago2/RISC. Such dsRNAs have the following structure: a sense strand and an antisense strand, where the sense and antisense strands form a duplex that is 22-43 base pairs in length; and the dsRNA contains one or more modified nucleotides at any of positions B*1 on the sense strand, B*1 on the antisense strand, C*1-C*3 on the sense strand, or C*1-C*3 on the antisense strand.

### Definitions

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them below, unless specified otherwise.

As used herein, the term "nucleic acid" refers to deoxyribonucleotides, ribonucleotides, or modified nucleotides, and polymers thereof in single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

As used herein, "nucleotide" is used as recognized in the art to include those with natural bases (standard), and modified bases well known in the art. Such bases are generally located at the 1' position of a nucleotide sugar moiety. Nucleotides generally comprise a base, sugar and a phosphate group. The nucleotides can be unmodified or modified at the sugar, phosphate and/or base moiety, (also referred to interchangeably as nucleotide analogs, modified nucleotides, non-natural nucleotides, non-standard nucleotides and other; see, e.g., Usman and McSwiggen, supra; Eckstein, et al., International PCT Publication No. WO 92/07065; Usman et al, International PCT Publication No. WO 93/15187; Uhlman & Peyman, supra). There are several examples of modified nucleic acid bases known in the art as summarized by Limbach, et al, Nucleic Acids Res. 22:2183, 1994. Some of the non-limiting examples of base modifications that can be introduced into nucleic acid molecules include, hypoxanthine, purine, pyridin-4-one, pyridin-2-one, phenyl, pseudouracil, 2,4,6-trimethoxy benzene, 3-methyl uracil, dihydrouridine, naphthyl, aminophenyl, 5-alkylcytidines (e.g., 5-methylcytidine), 5-alkyluridines (e.g., ribothymidine), 5-halouridine (e.g., 5-bromouridine) or 6-azapyrimidines or 6-alkylpyrimidines (e.g. 6-methyluridine), propyne, and others (Burgin, et al., Biochemistry 35: 14090, 1996; Uhlman & Peyman, supra). By "modified bases" in this aspect is meant nucleotide bases other than adenine, guanine, cytosine and uracil at 1 ' position or their equivalents.

As used herein, a "double-stranded ribonucleic acid" or "dsRNA" is a molecule comprising two oligonucleotide strands which form a duplex. A dsRNA may contain ribonucleotides, deoxyribonucleotides, modified nucleotides, and combinations thereof. Double-stranded RNAs are substrates for proteins and protein complexes in the RNA interference pathway, e.g., Dicer and RISC. Structures of nicked dsRNAs of the invention are shown in Figures IA and IB, with such dsRNAs comprising a duplex in Region B and a duplex in Region C. The boundary between Region B and Region C is determined by the presence of the Dicer cleavage site on the antisense strand. Region C is at least 1 bp, preferably at least 2 bp or 3bp. Region E comprises Region C and Region D. Structures of nicked dsRNAs are also shown in Figures 1C and ID, which comprise a duplex in Region H and a duplex in Region I. The boundary between Region H and Region I is determined by the presence of the Dicer cleavage site on the antisense strand. Region I is at least 1 bp, preferably at least 2 bp or 3bp.

Region J comprises Region I and Region D. Optionally, a dsRNA of the invention may comprise a Region F. Structures of dsRNAs of the invention possessing modified or universal nucleotides are shown in Figures 12A and 12B, which comprise a duplex in Region B* and a duplex in Region C*. The boundary between Region B* and Region C* is determined by the presence of the Dicer cleavage site on the antisense strand. Region C* is at least 1 bp, preferably at least 2 bp or 3bp.

As used herein, "duplex" refers to a double helical structure formed by the interaction of two single stranded nucleic acids. According to the present invention, a duplex may contain first and second strands which are sense and antisense, or which are target and antisense. A duplex is typically formed by the pairwise hydrogen bonding of bases, i.e., "base pairing", between two single stranded nucleic acids which are oriented antiparallel with respect to each other. Base pairing in duplexes generally occurs by Watson-Crick base pairing, e.g., guanine (G) forms a base pair with cytosine (C) in DNA and RNA, adenine (A) forms a base pair with thymine (T) in DNA, and adenine (A) forms a base pair with uracil (U) in RNA. Conditions under which base pairs can form include physiological or biologically relevant conditions (e.g., intracellular: pH 7.2, 140 mM potassium ion; extracellular pH 7.4, 145 mM sodium ion).Furthermore, duplexes are stabilized by stacking interactions between adjacent nucletotides. As used herein, a duplex may be established or maintained by base pairing or by stacking interactions. A duplex is formed by two complementary nucleic acid strands, which may be substantially complementary or fully complementary (see below).

By "complementary" or "complementarity" is meant that a nucleic acid can form hydrogen bond(s) with another nucleic acid sequence by either traditional Watson-Crick or Hoogsteen base pairing. In reference to the nucleic molecules of the present disclosure, the binding free energy for a nucleic acid molecule with its complementary sequence is sufficient to allow the relevant function of the nucleic acid to proceed, e.g., RNAi activity. Determination of binding free energies for nucleic acid molecules is well known in the art (see, e.g., Turner, et al., CSH Symp. Quant. Biol. LII, pp. 123-133, 1987; Frier, et al., Proc. Nat. Acad. Sci. USA 83:9373-9377, 1986; Turner, et al., J. Am. Chem. Soc. 109:3783-3785, 1987). A percent complementarity indicates the percentage of contiguous residues in a nucleic acid molecule that can form hydrogen bonds (e.g., Watson-Crick base pairing) with a second nucleic acid sequence (e.g., 5, 6, 7, 8, 9, or 10 nucleotides out of a total of 10 nucleotides in the first oligonucleotide being based paired to a second nucleic acid sequence having 10 nucleotides represents 50%, 60%, 70%, 80%, 90%, and 100% complementary, respectively).To determine that a percent complementarity is of at least a certain percentage, the percentage of contiguous residues in a nucleic acid molecule that can form hydrogen bonds (e.g., Watson-Crick base pairing) with a second nucleic acid sequence is calculated and rounded to the nearest whole number (e.g., 12, 13, 14, 15, 16, or 17 nucleotides out of a total of 23 nucleotides in the first oligonucleotide being based paired to a second nucleic acid sequence having 23 nucleotides represents 52%, 57%, 61%, 65%, 70%, and 74%, respectively; and has at least 50%, 50%, 60%, 60%, 70%, and 70% complementarity, respectively). As used herein, "substantially complementary" refers to complementarity between the strands such that they are capable of hybridizing under biological conditions. Substantially complementary sequences have 60%, 70%, 80%, 90%, 95%, or even 100% complementarity. Additionally, techniques to determine if two strands are capable of hybridizing under biological conditions by examining their nucleotide sequences are well known in the art.

Single-stranded nucleic acids that base pair over a number of bases are said to "hybridize." Hybridization is typically determined under physiological or biologically relevant conditions (e.g., intracellular: pH 7.2, 140 mM potassium ion; extracellular pH 7.4, 145 mM sodium ion). Hybridization conditions generally contain a monovalent cation and biologically acceptable buffer and may or may not contain a divalent cation, complex anions, e.g. gluconate from potassium gluconate, uncharged species such as sucrose, and inert polymers to reduce the activity of water in the sample, e.g. PEG. Such conditions include conditions under which base pairs can form.

Hybridization is measured by the temperature required to dissociate single stranded nucleic acids forming a duplex, i.e., (the melting temperature; Tm). Hybridization conditions are also conditions under which base pairs can form. Various conditions of stringency can be used to determine hybridization (see, e.g., Wahl, G. M. and S. L. Berger (1987) Methods Enzymol. 152:399; Kimmel, A. R. (1987) Methods Enzymol. 152:507). Stringent temperature conditions will ordinarily include temperatures of at least about 30° C, more preferably of at least about 37° C, and most preferably of at least about 42° C. The hybridization temperature for hybrids anticipated to be less than 50 base pairs in length should be 5-10°C less than the melting temperature (Tm) of the hybrid, where Tm is determined according to the following equations. For hybrids less than 18 base pairs in length, Tm(°C)=2(# of A+T bases)+4(# of G+C bases). For hybrids between 18 and 49 base pairs in length, Tm(°C)=81.5+16.6(log 10[Na+])+0.41 (% G+C)-(600/N), where N is the number of bases in the hybrid, and [Na+] is the concentration of sodium ions in the hybridization buffer ([Na+] for 1xSSC=0.165 M). (Varying additional parameters, such as hybridization time, the concentration of detergent, e.g., sodium dodecyl sulfate (SDS), the inclusion or exclusion of carrier DNA, and wash conditions are well known to those skilled in the art.) For example, a hybridization determination buffer is shown in Table 1.

**Table 1.**

| | **final conc.** | **Vender** | **Cat#** | **Lot#** | **m.w./Stock** | **To make 50 mL solution** | |
|---|---|---|---|---|---|---|---|
| NaCl | 100 mM | Sigma | S-5150 | 41K8934 | 5M | 1 | mL |
| KCl | 80 mM | Sigma | P-9541 | 70K0002 | 74.55 | 0.298 | g |
| MgCl₂ | 8 mM | Sigma | M-1028 | 120K8933 | 1M | 0.4 | mL |
| sucrose | 2% w/v | Fisher | BP220-212 | 907105 | 342.3 | 1 | g |
| Tris-HCl | 16 mM | Fisher | BP1757-500 | 12419 | 1M | 0.8 | mL |
| NaH₂PO₄ | 1 mM | Sigma | S-3193 | 52H-029515 | 120.0 | 0.006 | g |
| EDTA | 0.02 mM | Sigma | E-7889 | 110K89271 | 0.5M | 2 | µL |
| H₂O | | Sigma | W-4502 | 51K2359 | | to 50 | mL |
| pH = 7.0 at 20°C | adjust with HCl | | | | | | |

Useful variations on hybridization conditions will be readily apparent to those skilled in the art. Hybridization techniques are well known to those skilled in the art and are described, for example, in Benton and Davis (Science 196:180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Antisense to Molecular Cloning Techniques, 1987, Academic Press, New York); and Sambrook et al., Molecular Cloning: A Laboratory Manual, Cold Spring Harbor Laboratory Press, New York.

As used herein, "oligonucleotide strand" is a single stranded nucleic acid molecule. An oligonucleotide may comprise ribonucleotides, deoxyribonucleotides, modified nucleotides (e.g., nucleotides with 2' modifications, synthetic base analogs, etc.) or combinations thereof.

As used herein, "antisense strand" refers to a single stranded nucleic acid molecule which has a sequence complementary to that of a target RNA. When the antisense strand contains modified nucleotides with base analogs, it is not necessarily complementary over its entire length, but must at least duplex with a target RNA.

As used herein, "sense strand" refers to a single stranded nucleic acid molecule which has a sequence complementary to that of an antisense strand. When the antisense strand contains modified nucleotides with base analogs, the sense strand need not be complementary over the entire length of the antisense strand, but must at least duplex with the antisense strand.

As used herein, "guide strand" refers to a single stranded nucleic acid molecule of a dsRNA, which has a sequence complementary to that of a target RNA, and results in RNA interference by binding to a target RNA. After cleavage of the dsRNA by Dicer, a fragment of the guide strand remains associated with RISC, binds a target RNA as a component of the RISC complex, and promotes cleavage of a target RNA by RISC. As used herein, the guide strand does not necessarily refer to a continuous single stranded nucleic acid and may comprise a discontinuity, preferably at a site that is cleaved by Dicer. A guide strand is an antisense strand.

As used herein, "target RNA" refers to an RNA that would be subject to modulation guided by the antisense strand, such as targeted cleavage or steric blockage. The target RNA could be, for example genomic viral RNA, mRNA, a pre-mRNA, or a non-coding RNA. The preferred target is mRNA, such as the mRNA encoding a disease associated protein, such as ApoB, Bcl2, Hif-lalpha, Survivin or a p21 ras, such as H-ras, K-ras or N-ras.

As used herein, "passenger strand" refers to an oligonucleotide strand of a dsRNA, which has a sequence that is complementary to that of the guide strand. As used herein, the passenger strand does not necessarily refer to a continuous single stranded nucleic acid and may comprise a discontinuity, preferably at a site that is cleaved by Dicer. A passenger strand is a sense strand.

As used herein, "discontinuity" or "nick" is a break in a single phosphodiester linkage of a sense strand or antisense strand. A discontinuity refers only to a break in one phosphodiester linkage of one strand of the duplex, and excludes situations where one or more nucleotides are missing (e.g., a gap). A duplex formed by a sense or antisense strand containing a discontinuity is stabilized or maintained by the interactions of surrounding nucleotides in the strand or by the complementary strand. Exemplified "nicked dsRNA" duplexes consist of one discontinuity, but "nicked dsRNA" duplexes can include two, three or four discontinuities, so long as the duplex serves as a Dicer substrate in an *in vitro* Dicer substrate assay.

As used herein, "Dicer" refers to an endoribonuclease in the RNase III family that cleaves a dsRNA, e.g., double-stranded RNA (dsRNA) or pre-microRNA (miRNA), into double-stranded nucleic acid fragments about 20-25 nucleotides long, usually with a two-base overhang on the 3' end. With respect to the dsRNAs of the invention, the duplex formed by a dsRNA is recognized by Dicer and is a Dicer substrate on at least one strand of the duplex. For certain dsRNAs of the invention (*e.g.,* dsRNAs having modified or universal nucleotides on the sense or antisense strand at the Dicer cleavage site and modified or universal nucleotides on the antisense strand opposite to where the target strand is cleaved by Ago2/RISC), Dicer cleaves the sense strand of the dsRNA between the fourth and fifth nucleotide from the 3' terminus of the sense strand and the antisense strand of such a dsRNA of the invention between the sixth and seventh nucleotide from the 5' terminus of the antisense strand. Dicer catalyzes the first step in the RNA interference pathway, which consequently results in the degradation of a target RNA. The protein sequence of human Dicer is provided at the NCBI database under accession number NP_085124, hereby incorporated by reference.

Dicer "cleavage" is determined as follows (e.g., see Collingwood et al., Oligonucleotides;18:187-200 (2008)). In a Dicer cleavage assay, RNA duplexes (100 pmol) are incubated in 20 µL of 20 mM Tris pH 8.0, 200 mM NaCl, 2.5 mM MgCl2 with or without 1 unit of recombinant human Dicer (Stratagene, La Jolla, CA) at 37°C for 18-24 hours. Samples are desalted using a Performa SR 96-well plate (Edge Biosystems, Gaithersburg, MD). Electrospray-ionization liquid chromatography mass spectroscopy (ESI-LCMS) of duplex RNAs pre- and post-treatment with Dicer is done using an Oligo HTCS system (Novatia, Princeton, NJ; Hail et al., 2004), which consists of a ThermoFinnigan TSQ7000, Xcalibur data system, ProMass data processing software and Paradigm MS4 HPLC (Michrom BioResources, Auburn, CA). In this assay, Dicer cleavage occurs where at least 5%, 10%, 20%, 30%, 40%, 50%, 60%, 70%, 80%, 90%, 95%, or even 100% of the Dicer substrate dsRNA, (i.e., 25-30 bp dsRNA, preferably 26-30 bp dsRNA) is cleaved to a shorter dsRNA (e.g., 19-23 bp dsRNA, preferably, 21-23 bp dsRNA).

As used herein, "Dicer cleavage site" refers to the sites at which Dicer cleaves a dsRNA. Dicer contains two RNase III domains which typically cleave both the sense and antisense strands of a dsRNA. The average distance between the RNase III domains and the PAZ domain determines the length of the short double-stranded nucleic acid fragments it produces and this distance can vary (Macrae I, et al. (2006). "Structural basis for double-stranded RNA processing by Dicer". Science 311 (5758): 195-8.)

As used herein, "loop" refers to a structure formed by a single strand of a nucleic acid, in which complementary regions that flank a particular single stranded nucleotide region hybridize in a way that the single stranded nucleotide region between the complementary regions is excluded from duplex formation or Watson-Crick base pairing. A loop is a single stranded nucleotide region of any length. Examples of loops include the unpaired nucleotides present in such structures as hairpins, stem loops, or extended loops.

As used herein, "extended loop" in the context of a dsRNA refers to a single stranded loop and in addition 1, 2, 3, 4, 5, 6 or up to 20 base pairs or duplexed nucleotides flanking the loop. For example, extended loops are shown in Figure 1A, Region E, and in Figure 1B, Region J. In an extended loop, nucleotides that flank the loop on the 5' side thus form a duplex with nucleotides that flank the loop on the 3' side, *e.g.,* Region C in Figure 1A, and Region in Figure 1B. An extended loop may form a hairpin or stem loop.

In the context of a dsRNA, the nucleotides that form the base pairs or duplex flanking the loop are referred to as proximal or distal according to their position in reference to the loop and the strand containing the loop. As used herein, "proximal," in the context of a sense strand having an extended loop Region E at the 3' end of a sense strand (with reference to Figures 1A-1B, and 5A and 6A), refers to when the nucleotides that form base pairs or duplex flanking the loop are in positions 5' in relation to the nucleotides that form the tetraloop (*e.g.,* the proximal nucleotides are the nucleotides at the 5' end of Region C in the sense strand). As used herein, "proximal," in the context of an antisense strand having an extended loop Region J at the 5' end of an antisense strand (with reference to Figures 1C-1D, and 5B and 6B), refers to when the nucleotides that form base pairs or duplex flanking the loop are in positions 3' in relation to the nucleotides that form the tetraloop (*e.g.,* the proximal nucleotides are the nucleotides at the 3' end of Region I in the antisense strand). As used herein, "distal," in the context of a sense strand having an extended loop Region E at the 3 ' end of a sense strand (with reference to Figures 1A-1B, and 5A and 6A), refers to when the nucleotides that form base pairs or duplex flanking the loop are in positions 3' in relation to the nucleotides that form the tetraloop (*e.g.,* the distal nucleotides are the nucleotides in Region C at the 3' end of the sense strand). As used herein, "distal," in the context of an antisense strand having an extended loop Region J at the 5' end of an antisense strand (with reference to Figures 1C-1D, and 5B and 6B), refers to when the nucleotides that form base pairs or duplex flanking the loop are in positions 5' in relation to the nucleotides that form the tetraloop (*e.g.,* the distal nucleotides are the nucleotides in Region I at the 5' end of the antisense strand).

As used herein, "tetraloop" in the context of a dsRNA refers to a loop (a single stranded region) consisting of four nucleotides that forms a stable secondary structure that contributes to the stability of an adjacent Watson-Crick hybridized nucleotides. Without being limited to theory, a tetraloop may stabilize an adjacent Watson-Crick base pair by stacking interactions. In addition, interactions among the four nucleotides in a tetraloop include but are not limited to non-Watson-Crick base pairing, stacking interactions, hydrogen bonding, and contact interactions (Cheong et al., Nature. 1990 Aug 16;346(6285):680-2; Heus and Pardi, Science. 1991 Jul 12;253(5016):191-4). A tetraloop confers an increase in the melting temperature (Tm) of an adjacent duplex that is higher than expected from a simple model loop sequence consisting of four random bases. For example, a tetraloop can confer a melting temperature of at least 55°C in 10mM NaHPO₄ to a hairpin comprising a duplex of at least 2 base pairs in length. A tetraloop may contain ribonucleotides, deoxyribonucleotides, modified nucleotides, and combinations thereof. Examples of RNA tetraloops include the UNCG family of tetraloops (e.g., UUCG), the GNRA family of tetraloops (e.g., GAAA), and the CUUG tetraloop. (Woese et al., Proc Natl Acad Sci U S A. 1990 Nov;87(21):8467-71; Antao et al., Nucleic Acids Res. 1991 Nov 11;19(21):5901-5). Examples of DNA tetraloops include the d(GNNA) family of tetraloops (e.g., d(GTTA), the d(GNRA)) family of tetraloops, the d(GNAB) family of tetraloops, the d(CNNG) family of tetraloops, the d(TNCG) family of tetraloops (e.g., d(TTCG)). (Nakano et al. Biochemistry, 41 (48), 14281 -14292, 2002.; SHINJI et al. Nippon Kagakkai Koen Yokoshu VOL.78th; NO.2; PAGE.731 (2000).)

As used herein, "overhang" refers to unpaired nucleotides, in the context of a duplex having two or four free ends at either the 5' terminus or 3' terminus of a dsRNA. In certain embodiments, the overhang is a 3' or 5' overhang on the antisense strand or sense strand.

As used herein, "target" refers to any nucleic acid sequence whose expression or activity is to be modulated. In particular embodiments, the target refers to an RNA which duplexes to a single stranded nucleic acid that is an antisense strand in a RISC complex. Hybridization of the target RNA to the antisense strand results in processing by the RISC complex. Consequently, expression of the RNA or proteins encoded by the RNA, e.g., mRNA, is reduced.

As used herein, "reference" is meant a standard or control. As is apparent to one skilled in the art, an appropriate reference is where only one element is changed in order to determine the effect of the one element.

As used herein, "modified nucleotide" refers to a nucleotide that has one or more modifications to the nucleoside, the nucleobase, pentose ring, or phosphate group. For example, modified nucleotides exclude ribonucleotides containing adenosine monophosphate, guanosine monophosphate, uridine monophosphate, and cytidine monophosphate and deoxyribonucleotides containing deoxyadenosine monophosphate, deoxyguanosine monophosphate, deoxythymidine monophosphate, and deoxycytidine monophosphate. Modifications include those naturally occuring that result from modification by enzymes that modify nucleotides, such as methyltransferases. Modified nucleotides also include synthetic or non-naturally occurring nucleotides. Synthetic or non-naturally occurring modifications in nucleotides include those with 2' modifications, e.g., 2'-methoxyethoxy, 2'-fluoro, 2'-allyl, 2'-O-[2-(methylamino)-2-oxoethyl], 4'-thio, 4'-CH₂-O-2'-bridge, 4'-(CH₂)₂-O-2'-bridge, 2'-LNA, and 2'-O-(N-methylcarbamate) or those comprising base analogs. In connection with 2'-modified nucleotides as described for the present disclosure, by "amino" is meant 2'-NH₂ or 2'-O-NH₂, which can be modified or unmodified. Such modified groups are described, e.g., in Eckstein, et al., U.S. Pat. No. 5,672,695 and Matulic-Adamic, et al., U.S. Pat. No. 6,248,878.

In reference to the nucleic molecules of the present disclosure, the modifications may exist in patterns on a strand of the dsRNA. As used herein, "alternating positions" refers to a pattern where every other nucleotide is a modified nucleotide or there is an unmodified nucleotide between every modified nucleotide over a defined length of a strand of the dsRNA (e.g., 5'-MNMNMN-3'; 3'-MNMNMN-5'; where M is a modified nucleotide and N is an unmodified nucleotide). The modification pattern starts from the first nucleotide position at either the 5' or 3' terminus according to any of the position numbering conventions described herein. The pattern of modified nucleotides at alternating positions may run the full length of the strand, but preferably includes at least 4, 6, 8, 10, 12, 14 nucleotides containing at least 2, 3, 4, 5, 6 or 7 modified nucleotides, respectively. As used herein, "alternating pairs of positions" refers to a pattern where two consecutive modified nucleotides are separated by two consecutive unmodified nucleotides over a defined length of a strand of the dsRNA (e.g., 5'-MMNNMMNNMMNN-3'; 3'-MMNNMMNNMMNN-5'; where M is a modified nucleotide and N is an unmodified nucleotide). The modification pattern starts from the first nucleotide position at either the 5' or 3' terminus according to any of the position numbering conventions described herein. The pattern of modified nucleotides at alternating positions may run the full length of the strand, but preferably includes at least 8, 12, 16, 20, 24, 28 nucleotides containing at least 4, 6, 8, 10, 12 or 14 modified nucleotides, respectively.

As used herein, "base analog" refers to a heterocyclic moiety which is located at the 1' position of a nucleotide sugar moiety in a modified nucleotide that can be incorporated into a nucleic acid duplex (or the equivalent position in a nucleotide sugar moiety substitution that can be incorporated into a nucleic acid duplex). In the dsRNAs of the invention, a base analog is generally either a purine or pyrimidine base excluding the common bases guanine (G), cytosine (C), adenine (A), thymine (T), and uracil (U). Base analogs can duplex with other bases or base analogs in dsRNAs. Base analogs include those useful in the compounds and methods of the invention., e.g., those disclosed in US Pat. Nos. 5,432,272 and 6,001,983 to Benner and US Patent Publication No. 20080213891 to Manoharan. Non-limiting examples of bases include hypoxanthine (I), xanthine (X), 3β-D-ribofuranosyl-(2,6-diaminopyrimidine; K), 3-β-D-ribofuranosyl-(1-methyl-pyrazolo[4,3-d]pyrimidine-5,7(4H,6H)-dione; P), iso-cytosine (iso-C), iso-guanine (iso- G), 1-β-D-ribofuranosyl-(5-nitroindole), 1-β-D-ribofuranosyl-(3-nitropyrrole), 5- bromouracil, 2-aminopurine, 4-thio-dT, 7-(2-thienyl)-imidazo[4,5-b]pyridine (Ds) and pyrrole-2-carbaldehyde (Pa), 2-amino-6-(2-thienyl)purine (S), 2-oxopyridine (Y), difluorotolyl, 4-fluoro-6-methylbenzimidazole, 4-methylbenzimidazole, 3-methyl isocarbostyrilyl, 5-methyl isocarbostyrilyl, and 3-methyl-7-propynyl isocarbostyrilyl, 7- azaindolyl, 6-methyl-7-azaindolyl, imidizopyridinyl, 9-methyl-imidizopyridinyl, pyrrolopyrizinyl, isocarbostyrilyl, 7-propynyl isocarbostyrilyl, propynyl-7-azaindolyl, 2,4,5-trimethylphenyl, 4-methylindolyl, 4,6-dimethylindolyl, phenyl, napthalenyl, anthracenyl, phenanthracenyl, pyrenyl, stilbenzyl, tetracenyl, pentacenyl, and structural derivatives thereof (Schweitzer et al., J. Org. Chem., 59:7238-7242 (1994); Berger et al., Nucleic Acids Research, 28(15):291 1-2914 (2000); Moran et al., J. Am. Chem. Soc, 1 19:2056-2057 (1997); Morales et al., J. Am. Chem. Soc, 121 :2323-2324 (1999); Guckian et al., J. Am. Chem. Soc, 1 18:8182-8183 (1996); Morales et al., J. Am. Chem. Soc, 122(6):1001-1007 (2000); McMinn et al., J. Am. Chem. Soc, 121 : 1 1585-1 1586 (1999); Guckian et al., J. Org. Chem., 63:9652-9656 (1998); Moran et al., Proc Natl. Acad. Sci., 94: 10506-1051 1 (1997); Das et al., J. Chem. Soc, Perkin Trans., 1 : 197-206 (2002); Shibata et al., J. Chem. Soc, Perkin Trans., 1 : 1605-161 1 (2001); Wu et al., J. Am. Chem. Soc, 122(32):7621-7632 (2000); O'Neill et al., J. Org. Chem., 67:5869-5875 (2002); Chaudhuri et al., J. Am. Chem. Soc., 117:10434-10442 (1995); and U.S. Pat. No. 6,218,108.). Base analogs may also be a universal base.

As used herein, "universal base" refers to a heterocyclic moiety located at the 1' position of a nucleotide sugar moiety in a modified nucleotide, or the equivalent position in a nucleotide sugar moiety substitution, that, when present in a nucleic acid duplex, can be positioned opposite more than one type of base without altering the double helical structure (e.g., the structure of the phosphate backbone). Additionally, the universal base does not destroy the ability of the single stranded nucleic acid in which it resides to duplex to a target nucleic acid. The ability of a single stranded nucleic acid containing a universal base to duplex a target nucleic can be assayed by methods apparent to one in the art (e.g., UV absorbance, circular dichroism, gel shift, single stranded nuclease sensitivity, etc.). Additionally, conditions under which duplex formation is observed may be varied to determine duplex stability or formation, e.g., temperature, as melting temperature (Tm) correlates with the stability of nucleic acid duplexes. Compared to a reference single stranded nucleic acid that is exactly complementary to a target nucleic acid, the single stranded nucleic acid containing a universal base forms a duplex with the target nucleic acid that has a lower Tm than a duplex formed with the complementary nucleic acid. However, compared to a reference single stranded nucleic acid in which the universal base has been replaced with a base to generate a single mismatch, the single stranded nucleic acid containing the universal base forms a duplex with the target nucleic acid that has a higher Tm than a duplex formed with the nucleic acid having the mismatched base.

Some universal bases are capable of base pairing by forming hydrogen bonds between the universal base and all of the bases guanine (G), cytosine (C), adenine (A), thymine (T), and uracil (U) under base pair forming conditions. A universal base is not a base that forms a base pair with only one single complementary base. In a duplex, a universal base may form no hydrogen bonds, one hydrogen bond, or more than one hydrogen bond with each of G, C, A, T, and U opposite to it on the opposite strand of a duplex. Preferably, a universal base does not interact with the base opposite to it on the opposite strand of a duplex. In a duplex, base pairing between a universal base occurs without altering the double helical structure of the phosphate backbone. A universal base may also interact with bases in adjacent nucleotides on the same nucleic acid strand by stacking interactions. Such stacking interactions stabilize the duplex, especially in situations where the universal base does not form any hydrogen bonds with the base positioned opposite to it on the opposite strand of the duplex. Non-limiting examples of universal-binding nucleotides include inosine, 1-β-D-ribofuranosyl-5-nitroindole, and/or 1-β-D-ribofuranosyl-3-nitropyrrole (US Pat. Appl. Publ. No. 20070254362 to Quay et al.; Van Aerschot et al., An acyclic 5-nitroindazole nucleoside analogue as ambiguous nucleoside. Nucleic Acids Res. 1995 Nov 11;23(21):4363-70; Loakes et al., 3-Nitropyrrole and 5-nitroindole as universal bases in primers for DNA sequencing and PCR. Nucleic Acids Res. 1995 Jul 11;23(13):2361-6; Loakes and Brown, 5-Nitroindole as an universal base analogue. Nucleic Acids Res. 1994 Oct 11;22(20):4039-43).

As used herein, an "enzymatically synthesized" dsRNA refers to a dsRNA with modification produced by the reaction of a nucleic acid with an enzyme, including naturally occuring enzymes, (*e.g.,* methyltransferases, nicking enzymes, kinases, phosphatases, sulfurylases, ligases, nucleases, recombinases). Correspondingly, as used herein, "enzymatic" modifications refer to those that are produced by the reaction of a nucleic acid with an enzyme, including naturally occuring enzymes.

As used herein, a "chemically synthesized" dsRNA refers to a dsRNA produced by using chemical reactions, e.g., without using enzymes. Methods of chemically synthesizing RNA molecules are known in the art, in particular, the chemical synthesis methods as described in Verma and Eckstein (1998) or as described herein. Generally, dsRNA constructs can by synthesized using solid phase oligonucleotide synthesis methods (see for example Usman et al., U.S. Pat. Nos. 5,804,683; 5,831,071; 5,998,203; 6,117,657; 6,353,098; 6,362,323; 6,437,117; 6,469,158; Scaringe et al., U.S. Pat. Nos. 6,111,086; 6,008,400; 6,111,086).

As used herein "increase" or "enhance" is meant to alter positively by at least 5% compared to a reference in an assay. An alteration may be by 5%, 10%, 25%, 30%, 50%, 75%, or even by 100% compared to a reference in an assay. By "enhance Dicer cleavage," it is meant that the processing of a quantity of a dsRNA molecule by Dicer results in more Dicer cleaved dsRNA products or that Dicer cleavage reaction occurs more quickly compared to the processing of the same quantity of a reference dsRNA in an *in vivo* or *in vitro* assay of this disclosure. In one embodiment, enhanced or increased Dicer cleavage of a dsRNA molecule is above the level of that observed with an appropriate reference dsRNA molecule. In another embodiment, enhanced or increased Dicer cleavage of a dsRNA molecule is above the level of that observed with an inactive or attenuated molecule.

By "enhance interaction with Ago2," it is meant that the association with Ago2 of a Dicer processed dsRNA is more stable or occurs more quickly compared to the association of a reference dsRNA in an *in vivo* or *in vitro* assay of this disclosure. By "enhance cleavage by Ago2/RISC," it is meant that the processing of a quantity of a target RNA molecule by Ago2/RISC bound to an antisense strand of a dsRNA results in more Ago2/RISC cleaved target RNA or that target RNA cleavage by Ago2/RISC occurs more quickly compared to that when an antisense strand from a reference dsRNA is associated with Ago2/RISC in an *in vitro* or *in vivo* assay of this disclosure.

As used herein "reduce" is meant to alter negatively by at least 5% compared to a reference in an assay. An alteration may be by 5%, 10%, 25%, 30%, 50%, 75%, or even by 100% compared to a reference in an assay. By "reduce expression," it is meant that the expression of the gene, or level of RNA molecules or equivalent RNA molecules encoding one or more proteins or protein subunits, or level or activity of one or more proteins or protein subunits encoded by a target gene, is reduced below that observed in the absence of the nucleic acid molecules (e.g., dsRNA molecule) in an in vivo or in vitro assay of this disclosure. In one embodiment, inhibition, down-regulation or reduction with a dsRNA molecule is below that level observed in the presence of an inactive or attenuated molecule. In another embodiment, inhibition, down-regulation, or reduction with dsRNA molecules is below that level observed in the presence of, e.g., an dsRNA molecules with scrambled sequence or with mismatches. In another embodiment, inhibition, down-regulation, or reduction of gene expression with a nucleic acid molecule of the instant disclosure is greater in the presence of the nucleic acid molecule than in its absence.

As used herein, "cell" is meant to include both prokaryotic (*e.g.,* bacterial) and eukaryotic (*e.g.,* mammalian or plant) cells. Cells may be of somatic or germ line origin, may be totipotent or pluripotent, and may be dividing or non-dividing. Cells can also be derived from or can comprise a gamete or an embryo, a stem cell, or a fully differentiated cell. Thus, the term "cell" is meant to retain its usual biological meaning and can be present in any organism such as, for example, a bird, a plant, and a mammal, including, for example, a human, a cow, a sheep, an ape, a monkey, a pig, a dog, and a cat. Within certain aspects, the term "cell" refers specifically to mammalian cells, such as human cells, that contain one or more isolated dsRNA molecules of the present disclosure. In particular aspects, a cell processes dsRNAs resulting in RNA intereference of target nucleic acids, and contains proteins and protein complexes required for RNAi, e.g., Dicer and RISC.

As used herein, "animal" is meant a multicellular, eukaryotic organism, including a mammal, particularly a human. The methods of the invention in general comprise administration of an effective amount of the agents herein, such as an agent of the structures of formulae herein, to a subject (e.g., animal, human) in need thereof, including a mammal, particularly a human. Such treatment will be suitably administered to subjects, particularly humans, suffering from, having, susceptible to, or at risk for a disease, or a symptom thereof.

By "pharmaceutically acceptable carrier" is meant, a composition or formulation that allows for the effective distribution of the nucleic acid molecules of the instant disclosure in the physical location most suitable for their desired activity.

The present invention is directed to compositions that contain a double stranded RNA ("dsRNA"), and methods for preparing them, that are capable of reducing the expression of target genes in eukaryotic cells.

For "nicked dsRNA" aspects of the invention, one of the strands of the dsRNA contains a region of nucleotide sequence that has a length that ranges from about 15 to about 22 nucleotides that can direct the destruction of the RNA transcribed from the target gene. Such "nicked dsRNAs" of the invention also contain an extended loop which contains a tetraloop. In some embodiments, the extended loop containing the tetraloop is at the 3' terminus of the sense strand. In other embodiments, the extended loop containing the tetraloop is at the 5' terminus of the antisense strand.

The "nicked dsRNA" aspects of the invention are based at least in part on the discovery that a nicked strand in a dsRNA is more amenable to chemical modification because it does not have to be competent for Dicer RNase III cleavage. The presence of a nick in the dsRNA can enable modifications for multiple purposes on either the antisense or sense strand. Without limitation, such advantageous purposes include silencing the sense strand, stabilizing the entire construct, enhancing delivery, pharmacokinetics, loading into complex formulations, increasing the duration of action, potency, specificity. The "nicked dsRNA" aspects of the invention are also based at least in part on the discovery that a nicked strand in a dsRNA can direct the production of substantially only one Dicer cleavage product. Placement of a nick in a dsRNA at the location of one of the two Dicer RNase III cleavage sites in a dsRNA directs Dicer toward the other site, thereby defining Dicer cleavage at the other site. However, nicked double stranded structures formed from three single stranded nucleic acids are expected to be unstable under physiological or biological conditions. Thus, certain aspects of the invention also include the feature that the dsRNA is formed from only two strands to increase the stability of the nicked dsRNA substrate.

The two stranded nicked dsRNA substrate of the invention contains an extended loop containing a tetraloop. In some embodiments of the nicked dsRNA substrate, an extended loop containing a tetraloop is placed at the 3' terminus of the sense strand. In other embodiments of the nicked dsRNA substrate, an extended loop containing a tetraloop is placed at the 5 terminus of the antisense strand. The stability of the dsRNA is particularly enhanced by the inclusion of a tetraloop, which adopts a secondary structure with thermodynamic stability even under stringent conditions (e.g., low salt conditions). Further advantages of such a dsRNA with a tetraloop are also contemplated. The "bare" ends of duplex nucleic acids have potent biological effects, including immune system stimulation. The effective elimination of one of the ends of the initial DsiRNA configuration also reduces the potential for immunostimulation, which is undesirable in some applications. Ends of dsRNA are also key points of entry for helicases and/or nucleases, thus a nicked dsRNA containing a tetraloop should be more resistant to both.

Other advantages regarding the nicked dsRNA substrate possessing a tetraloop contemplate modification of the nucleotides of the sense and antisense strands. When the discontinuity occurs on the antisense strand, antisense strand modifications are more tolerated in the RNA interference pathway. When the discontinuity occurs on the sense strand, sense strand modifications are more tolerated in the RNA interference pathway. The invention allows a greater extent of modification of sense and antisense strands. The invention also allows for more types of modifications of the antisense strand without interfering with processing by Dicer and/or Ago2. Furthermore, it is contemplated that particular modifications that are more tolerated can have additional advantages, *e.g*., enhancing Ago2 binding.

For dsRNAs of the invention which possess modified or universal nucleotides at specific site(s) (*e.g.,* on the sense or antisense strand at the Dicer cleavage site and on the antisense strand opposite to where the target strand is cleaved by Ago2/RISC), one of the strands of the dsRNA contains a region of nucleotide sequence that has a length that ranges from about 22 to about 47 nucleotides that can act as an antisense strand with respect to inhibiting the expression of the RNA transcribed from the target gene. In particular, referring to Figures 12, 15A, 15B, 16A, 16B, and 17, such a dsRNA contains more than one modified nucleotide at any of positions B*1, C*1-C*3 on the sense strand or positions B*1, C*1-C*2 on the antisense strand; more than one modified nucleotide at any of positions C*1-C*2 on the sense strand or position B*1 on the antisense strand; a modified nucleotide at position B*1 on the antisense strand; a universal nucleotide on the antisense strand at any of positions C*9-C*12 on the antisense strand; or combinations thereof.

Such aspects of the invention are based at least in part on the discovery that modifications in dsRNA can be made that enhance the interactions with or the processing of dsRNA by proteins in the RNA interference pathway. Advantages of such dsRNA substrates of the invention include enhanced cleavage of the modified dsRNA by Dicer. Specifically, in such a dsRNA of the invention, one or more modified nucleotides at positions B*1, C*1-C*3 on the sense strand or positions B*1, C*1-C*3 on the antisense strand are present. These positions encompass the Dicer cleavage sites on both sense and antisense strands of the dsRNA. In particular, modified nucleotides at positions B*1, C*1-C*3 on the sense strand or positions B*1, C*1-C*3 on the antisense strand are important for Dicer cleavage. Without being limited to any particular theory, modified nucleotides at positions B*1, C*1-C*3 on the sense strand or positions B*1, C*1-C*3 on the antisense strand promote cleavage of the dsRNA by Dicer by enhanced binding to Dicer or providing Dicer a preferred sequence.

Additional advantages of the dsRNA substrates of the invention which possess modified or universal nucleotides at specific site(s) include enhanced interaction with Ago2. In particular, referring to Figures 12, 15A, 15B, 16A, 16B, and 17, modified nucleotides at positions B*1, C*1, and C*2 on the sense strand or B*1 on the antisense strand are important for Ago2 interaction. Without being limited to any particular theory, modified nucleotides at positions B*1, C*1, and C*2 on the sense strand or position B*1 on the antisense strand, which become exposed after cleavage of the dsRNA by Dicer, enhance binding to Ago2 of the Dicer processed dsRNA. Specifically, a modified nucleotide at position B*1 on the antisense strand has enhanced interaction with Ago2 after Dicer has processed the dsRNA. Without being limited to any particular theory, a modified nucleotide at position B*1 on the antisense strand when exposed has enhanced interaction with a binding pocket in Ago2.

Further advantages of those dsRNA substrates of the invention which possess modified or universal nucleotides at specific site(s) include enhanced cleavage of target RNA when the antisense strand or a portion thereof is bound to RISC. The dsRNA can contain a universal nucleotide on the antisense strand at any of positions C*9-C*12 on the antisense strand. In a dsRNA of the invention, these positions also correspond to positions 9-12 from a Dicer cleavage site on the antisense strand. Without being limited to any particular theory, a modified nucleotide at positions C*9-C*12 on the antisense strand enhances cleavage of the target RNA by lowering the energy required for catalysis by Ago2/RISC. Consequently, such a modified dsRNA of the invention reduces target gene expression in comparison to a reference dsRNA.

### Compositions

In a first aspect, the present invention provides novel compositions for RNA interference (RNAi). The compositions comprise either a double stranded ribonucleic acid (dsRNA) which is a precursor molecule, *i.e.,* the dsRNA of the present invention is processed in vivo to produce an active small interfering nucleic acid (siRNA). The dsRNA is processed by Dicer to an active siRNA which is incorporated into the RISC complex. The precursor molecule is also termed a precursor RNAi molecule herein. As used herein, the term active siRNA refers to a dsRNA in which each strand comprises RNA, RNA analog(s), or RNA and DNA. The siRNA comprises between 19 and 23 nucleotides or comprises 21 nucleotides. The active siRNA has 2 bp overhangs on the 3' ends of each strand such that the duplex region in the siRNA comprises 17-21 nucleotides, or 19 nucleotides. Typically, the antisense strand of the siRNA is substantially complementary with the target sequence of the target gene.

The duplex region refers to the region in two complementary or substantially complementary oligonucleotides that form base pairs with one another, either by Watson-Crick base pairing or any other manner that allows for a duplex between oligonucleotide strands that are complementary or substantially complementary. For example, an oligonucleotide strand having 21 nucleotide units can base pair with another oligonucleotide of 21 nucleotide units, yet only 19 bases on each strand are complementary or substantially complementary, such that the "duplex region" consists of 19 base pairs. The remaining base pairs may, for example, exist as 5' and 3' overhangs. Further, within the duplex region, 100% complementarity is not required; substantial complementarity is allowable within a duplex region. Substantial complementarity refers to complementarity between the strands such that they are capable of annealing under biological conditions. Techniques to determine if two strands are capable of annealing under biological conditions are well know in the art. Alternatively, two strands can be synthesized and added together under biological conditions to determine if they anneal to one another.

As used herein, a siRNA having a sequence substantially complementary to a target mRNA sequence means that the siRNA has sequence complementarity or duplexes to trigger the destruction of the target mRNA by the RNAi machinery (*e.g.,* the RISC complex) or process. The siRNA molecule can be designed such that every residue of the antisense strand is complementary to a residue in the target molecule. In cases where nucleotides with universal bases are used, the siRNA molecule duplexes with the target mRNA. Alternatively, substitutions can be made within the molecule to increase stability and/or enhance processing activity of said molecule. Substitutions can be made within the strand or can be made to residues at the ends of the strand.

In certain aspects of the present invention, the dsRNA, *i.e.,* the precursor RNAi molecule, has a length sufficient such that it is processed by Dicer to produce an siRNA. In some embodiments, a suitable "nicked dsRNA" of the invention contains a sense oligonucleotide sequence that contains an extended loop and is at least 19 nucleotides in length and no longer than about 80 nucleotides in length. This sense oligonucleotide that contains an extended loop can be between about 40, 50, 60, or 70 nucleotides in length. This sense oligonucleotide that contains an extended loop can be about 35 or 37 nucleotides in length or 35 nucleotides in length.

In other embodiments, a suitable dsRNA of the invention which possesses a modified or universal nucleotide at specific site(s) contains a sense oligonucleotide sequence that is at least 22 nucleotides in length and no longer than about 43 nucleotides in length. This sense oligonucleotide can be between about 20, 30, 40, or 50 nucleotides in length. In certain embodiments, this sense oligonucleotide is between about 25 and 30 nucleotides in length.

The antisense oligonucleotide of a dsRNA of the invention can have any sequence that anneals to the sense oligonucleotide to form a duplex under biological conditions, such as within the cytoplasm of a eukaryotic cell, or under the conditions of an acceptable pharmaceutical formulation. Generally, the duplex between the sense and antisense strands is at least 19 base pairs in length and no longer than about 26 base pairs. Generally, the antisense oligonucleotide will have at least 19 complementary base pairs with the sense oligonucleotide, more typically the antisense oligonucleotide will have about 21 or more complementary base pairs, or about 25 or more complementary base pairs with the sense oligonucleotide sequence. In certain embodiments, the sense oligonucleotide contains an extended loop which includes a tetraloop. In other embodiments, the 3' terminus of the antisense strand of the dsRNA has an overhang. In a particular embodiment, the 3' overhang is 2 nucleotides. The sense strand may also have a 5' phosphate.

In another embodiment, a "nicked dsRNA" of the invention (*i.e.,* the precursor RNAi molecule) has a length sufficient such that it is processed by Dicer to produce an siRNA. According to this embodiment, a suitable "nicked dsRNA" contains an antisense oligonucleotide sequence that contains an extended loop and is at least 27 nucleotides in length and no longer than about 70 nucleotides in length. This antisense oligonucleotide that contains an extended loop can be between about 40, 50, 60, or 70 nucleotides in length. This antisense oligonucleotide that contains an extended loop can be about 35 or 37 nucleotides in length or 35 nucleotides in length. The sense oligonucleotide of the dsRNA can have any sequence that anneals to the antisense oligonucleotide to form a duplex under biological conditions, such as within the cytoplasm of a eukaryotic cell. Generally, the sense oligonucleotide will have at least 19 complementary base pairs with the antisense oligonucleotide, more typically the sense oligonucleotide will have about 21 or more complementary base pairs, or about 25 or more complementary base pairs with the antisense oligonucleotide sequence. In certain embodiments, the antisense oligonucleotide contains an extended loop which includes a tetraloop.

In certain aspects, the sense and antisense oligonucleotide sequences of the dsRNA exist on two separate oligonucleotide strands that can be and typically are chemically synthesized. In some embodiments, both strands are between 26 and 30 nucleotides in length. In other embodiments, both strands are between 25 and 30 nucleotides in length. In one embodiment, one or both oligonucleotide strands are capable of serving as a substrate for Dicer. In other embodiments, at least one modification is present that promotes Dicer to bind to the dsRNA structure in an orientation that maximizes the dsRNA structure's effectiveness in inhibiting gene expression. The dsRNA can contain one or more ribonucleic acid (RNA) or deoxyribonucleic acid (DNA) base substitutions.

The sense and antisense oligonucleotides are not required to be completely complementary. In fact, in one embodiment, the 3' terminus of the sense strand contains one or more mismatches or modified nucleotides with base analogs . In one aspect, about two mismatches or modified nucleotides with base analogs are incorporated at the 3' terminus of the sense strand. The use of mismatches or decreased thermodynamic stability (specifically at the 3'-sense/5'-antisense position) has been proposed to facilitate or favor entry of the antisense strand into RISC (Schwarz et al., 2003; Khvorova et al., 2003), presumably by affecting some rate-limiting unwinding steps that occur with entry of the siRNA into RISC. Thus, terminal base composition has been included in design algorithms for selecting active 21mer siRNA duplexes (Ui-Tei et al., 2004; Reynolds et al., 2004). With Dicer cleavage of the dsRNA of this embodiment, the small end-terminal sequence which contains the mismatches or modified nucleotides with base analogs will either be left unpaired with the antisense strand (become part of a 3'-overhang) or be cleaved entirely off the final 21-mer siRNA. These mismatches or modified nucleotides with base analogs, therefore, do not persist in the final RNA component of RISC.

It has been found that the long dsRNA species having duplexes of 25 to about 30 nucleotides give unexpectedly effective results in terms of potency and duration of action. Without wishing to be bound by the underlying theory of the invention, it is thought that the longer dsRNA species serve as a substrate for the enzyme Dicer in the cytoplasm of a cell. In addition to cleaving the dsRNA of the invention into shorter segments, Dicer is thought to facilitate the incorporation of a single-stranded cleavage product derived from the cleaved dsRNA into the RISC complex that is responsible for the destruction of the cytoplasmic RNA derived from the target gene. Studies have shown that the cleavability of a dsRNA species by Dicer corresponds with increased potency and duration of action of the dsRNA species (Collingwood et al., 2008).

A dsRNA containing an extended loop with a tetraloop is produced upon annealing of the two oligonucleotides making up the dsRNA composition. The extended loop containing the tetraloop or the tetraloop structure will not block Dicer activity on the dsRNA and will not interfere with the directed destruction of the RNA transcribed from the target gene. Suitable dsRNA compositions may also contain sense or antisense strand formed from two separate oligonucleotides chemically linked outside their annealing region by chemical linking groups. Many suitable chemical linking groups are known in the art and can be used. Suitable groups will not block Dicer activity on the dsRNA and will not interfere with the directed destruction of the RNA transcribed from the target gene.

In certain embodiments, the dsRNA, *i.e.,* the precursor RNAi molecule, has several properties which enhance its processing by Dicer. According to such embodiments, the dsRNA has a length sufficient such that it is processed by Dicer to produce an active siRNA and at least one of the following properties: (i) the dsRNA is asymmetric, e.g., has a 3' overhang on the antisense strand and (ii) the dsRNA has a modified 3' end on the sense strand to direct orientation of Dicer binding and processing of the dsRNA to an active siRNA. According to such embodiments, where applied to "nicked dsRNAs," the longest strand in the dsRNA comprises 24-30 nucleotides. In one embodiment, the dsRNA is asymmetric such that the sense strand comprises 22-28 nucleotides and the antisense strand comprises 24-30 nucleotides. Thus, the resulting dsRNA has an overhang on the 3' end of the antisense strand. The overhang is 1-3 nucleotides, for example 2 nucleotides. The sense strand may also have a 5' phosphate.

In another embodiment of "nicked dsRNAs" of the invention, the sense strand is modified for Dicer processing by suitable modifiers located at positions 11 and 12 from the 3' terminus of the sense strand with an extended loop or at positions 2 and 4 from the 5' terminus of the sense strand with an extended loop, *i.e.,* the dsRNA is designed to direct orientation of Dicer binding and processing.

For any aspect of the invention, suitable modifiers include nucleotides such as deoxyribonucleotides, acyclonucleotides and the like and sterically hindered molecules, such as fluorescent molecules and the like. Acyclonucleotides substitute a 2-hydroxyethoxymethyl group for the 2'-deoxyribofuranosyl sugar normally present in dNMPs. In one embodiment, deoxynucleotides are used as the modifiers. When sterically hindered molecules are utilized, they are attached to the ribonucleotide at the 3' end of the antisense strand. Thus, the length of the strand does not change with the incorporation of the modifiers. In another embodiment, the invention contemplates substituting two DNA bases in the dsRNA to direct the orientation of Dicer processing of the antisense strand. In a further embodiment of the present invention, two terminal DNA bases are substituted for two ribonucleotides on the 3'-end of the sense strand forming a blunt end of the duplex on the 3' end of the sense strand and the 5' end of the antisense strand, and a two-nucleotide RNA overhang is located on the 3'-end of the antisense strand. This is an asymmetric composition with DNA on the blunt end and RNA bases on the overhanging end.

For dsRNAs of the invention which possess modified or universal nucleotides at specific site(s) (*e.g.,* on the sense or antisense strand at the Dicer cleavage site and on the antisense strand opposite to where the target strand is cleaved by Ago2/RISC), in further embodiments, the dsRNA has a length sufficient such that it is processed by Dicer to produce an active siRNA and at least one of the following properties: (i) the dsRNA is asymmetric, e.g., has a 3' overhang on the antisense strand and (ii) the dsRNA has a modified 3' end on the sense strand to direct orientation of Dicer binding and processing of the dsRNA to an active siRNA. According to such embodiments, the longest strand in the dsRNA comprises 19-43 nucleotides. In one embodiment, the dsRNA is asymmetric such that the sense strand comprises 22-43 nucleotides and the antisense strand comprises 26-47 nucleotides. Thus, the resulting dsRNA has an overhang on the 3' end of the antisense strand. The overhang may be 1-4 nucleotides, for example 2 nucleotides. The sense strand may also have a 5' phosphate.

In additional embodiments, for dsRNAs that possess modified or universal nucleotides at specific site(s), referring to Figures 12, 15A, 15B, 16A, 16B, and 17, the sense strand is modified for Dicer processing by suitable modifiers located at any of positions B*1, C*1-C*3 on the sense strand or positions B*1, C*1-C*3 on the antisense strand. In other various embodiments of aspects of the invention, a dsRNA contains more than one modifier at any of positions B*1, C*1, and C*3 on the sense strand or position B*1 on the antisense strand. In a particular embodiment of aspects of the invention, a dsRNA contains a modifier at position B*1 on the antisense strand. In still other various embodiments, a dsRNA contains a universal nucleotide on the antisense strand at any of positions B*9-B*11 on the antisense strand. In further embodiments, a dsRNA contains modifiers at any combination of positions described herein.

Advantages of the dsRNA substrates of the invention possessing modified or universal nucleotides at specific site(s) include enhanced cleavage of the modified dsRNA by Dicer, and enhanced control over the final Dicer product length. Specifically, in such a dsRNA of the invention, one or more modified nucleotides at positions B*1, C*1-C*3 on the sense strand or positions B*1, C*1-C*3 on the antisense strand are present, which encompass the Dicer cleavage sites on both sense and antisense strands of the dsRNA. Without being limited to any particular theory, modified nucleotides at positions B*1, C*1-C*3 on the sense strand or positions B*1, C*1-C*3 on the antisense strand promote cleavage of the dsRNA by Dicer by enhancing binding to Dicer or providing Dicer a preferred sequence for cleavage. In various embodiments of aspects of the invention, the dsRNA enhances cleavage by Dicer on either the sense or antisense strand in comparison to a reference dsRNA. In further emodiments of aspects of the invention, the dsRNA more accurately defines the position of Dicer cleavages.

Additional advantages regarding the dsRNA substrates of the invention possessing modified or universal nucleotides at specific site(s) include enhanced interaction with Ago2 after cleavage of the dsRNA by Dicer. Modified nucleotides at any of the positions described herein that enhance interaction with Ago2 do not interfere with Dicer processing, which occurs before the dsRNA can be further processed by Ago2. Without being limited to any particular theory, modified nucleotides at positions B*1, C*1, and C*2 on the sense strand or position B*1 on the antisense strand, which become exposed after cleavage of the dsRNA by Dicer, enhance binding to Ago2 of the Dicer processed dsRNA. Additionally, without being limited to theory, modified nucleotides at positions B*1, C*1, and C*2 on the sense strand or position B*1 on the antisense strand, which become exposed after cleavage of the dsRNA by Dicer, enhance unwinding of the duplex of the Dicer processed dsRNA. Without being limited to any particular theory, a modified nucleotide at position B*1 on the antisense strand when exposed has enhanced interaction with a binding pocket in Ago2. Without being bound to theory, the enhanced interaction of an exposed nucleotide at this position to Ago2 may occur through stacking interaction with a tyrosine residue in the binding pocket. In various embodiments of aspects of the invention, the dsRNA has enhanced interaction with Ago2 compared to a reference dsRNA.

Further advantages regarding the dsRNA substrates of the invention possessing modified or universal nucleotides at specific site(s) include enhanced cleavage of target RNA when the antisense strand or a portion thereof is bound to RISC. Consequently, the dsRNA reduces target gene expression in comparison to a reference dsRNA. Without being limited to any particular theory, a modified nucleotide at positions B*9-B*11 from the 5' terminus of the antisense strand enhances cleavage of the target RNA by lowering the energy for catalysis by Ago2/RISC. In such a dsRNA of the invention, these positions also correspond to positions 9-12 from a Dicer cleavage site on the antisense strand. Without being bound to theory, during cleavage of target RNA by Ago2/RISC, one of the bases adjacent to the cleavage site is pulled out of the duplex to distort the RNA target backbone. The distortion of the phosphate backbone reduces the energy needed to perform the hydrolytic cleavage of the phosphate bond. In further embodiments of aspects of the invention, the dsRNA contains a universal nucleotide at position B*10 or B*11 on the antisense strand. In specific embodiments, the dsRNA contains a universal nucleotide at position 10 or 11 from a Dicer cleavage site on the antisense strand. In other embodiments of aspects of the invention, the dsRNA contains universal nucleotides at positions B*10 and B*11 on the antisense strand. In specific embodiments, the dsRNA contains a universal nucleotides at positions 10 and 11 from a Dicer cleavage site on the antisense strand. In still further embodiments, the dsRNA contains universal nucleotides on the antisense strand at positions B*10 and B*11on the antisense strand and a universal nucleotide at any one of position B*9, position B*12, or positions B*9 and B*12 on the antisense strand. In specific embodiments, the dsRNA contains universal nucleotides at positions 10 and 11 from a Dicer cleavage site on the antisense strand and a universal nucleotide at any one of position 9, position 12, or positions 9 and 12 from a Dicer cleavage site on the antisense strand. In various embodiments of aspects of the invention, the antisense strand enhances cleavage of a target RNA by Ago2/RISC in comparison to an antisense strand of a reference dsRNA.

Suitable modifiers of such dsRNAs of the invention include nucleotides such as deoxyribonucleotides, dideoxyribonucleotides, acyclonucleotides and the like and sterically hindered molecules, such as fluorescent molecules and the like.

The sense and antisense strands anneal under biological conditions, such as the conditions found in the cytoplasm of a cell. In addition, a region of one of the sequences, particularly of the antisense strand, of the dsRNA has a sequence length of at least 19 nucleotides, wherein these nucleotides are in the 21-nucleotide region adjacent to the 3' end of the antisense strand and are substantially complementary or duplexes to a nucleotide sequence of the RNA produced from the target gene.

Further in accordance with this embodiment, the dsRNA, i.e., the precursor RNAi molecule, may also have one or more of the following additional properties: (a) the antisense strand has a right shift from the typical 21mer, (b) the strands may not be completely complementary, i.e., the strands may contain simple mismatch pairings and (c) base modifications such as locked nucleic acid(s) may be included in the 5' end of the sense strand. A "typical" 21mer siRNA is designed using conventional techniques. In one technique, a variety of sites are commonly tested in parallel or pools containing several distinct siRNA duplexes specific to the same target with the hope that one of the reagents will be effective (Ji et al., 2003). Other techniques use design rules and algorithms to increase the likelihood of obtaining active RNAi effector molecules (Schwarz et al., 2003; Khvorova et al., 2003; Ui-Tei et al., 2004; Reynolds et al., 2004; Krol et al., 2004; Yuan et al., 2004; Boese et al., 2005). High throughput selection of siRNA has also been developed (U.S. published patent application No. 2005/0042641 A1). Potential target sites can also be analyzed by secondary structure predictions (Heale et al., 2005). This 21mer is then used to design a right shift to include 3-9 additional nucleotides on the 5' end of the 21mer. The sequence of these additional nucleotides may have any sequence. In one embodiment, the added ribonucleotides are based on the sequence of the target gene. Even in this embodiment, full complementarity between the target sequence and the antisense siRNA is not required.

The sense and antisense oligonucleotides are not required to be completely complementary. They only need to duplex or to be substantially complementary to anneal under biological conditions and to provide a substrate for Dicer that produces a siRNA sufficiently complementary to the target sequence. Locked nucleic acids, or LNA's, are well known to a skilled artisan (Elman et al., 2005; Kurreck et al., 2002; Crinelli et al., 2002; Braasch and Corey, 2001 ; Bondensgaard et al., 2000; Wahlestedt et al., 2000). In one embodiment, an LNA is incorporated at the 5' terminus of the sense strand. In another embodiment, an LNA is incorporated at the 5' terminus of the sense strand in duplexes designed to include a 3' overhang on the antisense strand.

In one embodiment, the dsRNA has an asymmetric structure, with the sense strand having a length of 35 nucleotides, and the antisense strand having a length of 21 nucleotides with a 2 nucleotide 3'-overhang. In another embodiment, the dsRNA has an asymmetric structure, with the sense strand having a length of 37 nucleotides, and the antisense strand having a length of 21 nucleotides with a 2 nucleotide 3'-overhang. In yet another embodiment, the dsRNA has an asymmetric structure, with the antisense strand having a length of 35 nucleotides with a 2 nucleotide 3'-overhang, and the sense strand having a length of 21 nucleotides. In still another embodiment, the dsRNA has an asymmetric structure, with the sense strand having a length of 37 nucleotides with a 2 nucleotide 3'-overhang, and the antisense strand having a length of 21 nucleotides. In various embodiments, this dsRNA having an asymmetric structure further contains 2 deoxyribonucleotides at the 3' end of the antisense strand.

In another embodiment of an aspect of the present invention, the dsRNA, i.e., the precursor RNAi molecule, has several properties which enhances its processing by Dicer. According to this embodiment, the dsRNA has a length sufficient such that it is processed by Dicer to produce an siRNA and at least one of the following properties: (i) the dsRNA is asymmetric, e.g., has a 3' overhang on the sense strand and (ii) the dsRNA has a modified 3' end on the antisense strand to direct orientation of Dicer binding and processing of the dsRNA to an active siRNA. According to this embodiment, the longest strand in the dsRNA comprises 35-40 nucleotides. In one embodiment, the sense strand comprises 35-40 nucleotides and the antisense strand comprises 21-24 nucleotides. Thus, in some embodiments the resulting dsRNA has an overhang on the 3' end of the sense strand. The overhang may be 1-4 nucleotides. In another embodiment, the antisense strand comprises 35-40 nucleotides and the sense strand comprises 21-24 nucleotides. Thus, in some embodiments, the resulting dsRNA has an overhang on the 3' end of the sense strand. The overhang may be 1-4 nucleotides. The antisense strand may also have a 5' phosphate. In another embodiment, this dsRNA having an asymmetric structure further contains 2 deoxynucleotides at the 3' end of the antisense strand.

Further in accordance with this embodiment, the dsRNA, i.e., the precursor RNAi molecule, may also have one or more of the following additional properties: (a) the antisense strand has a right shift from the typical 21mer and (b) the strands may not be completely complementary, i.e., the strands may contain simple mismatch pairings. A "typical" 21mer siRNA is designed using conventional techniques, such as described above. This 21mer is then used to design a right shift to include 1-7 additional nucleotides on the 5' end of the 21 mer. The sequence of these additional nucleotides may have any sequence. Although the added ribonucleotides may be complementary to the target gene sequence, full complementarity between the target sequence and the antisense siRNA is not required. That is, the resultant antisense siRNA is sufficiently complementary with the target sequence. The first and second oligonucleotides are not required to be completely complementary. They only need to be substantially complementary to anneal under biological conditions and to provide a substrate for Dicer that produces a siRNA sufficiently complementary to the target sequence.

In other embodiments, the dsRNA compositions of the invention possessing modified or universal nucleotides at specific site(s) contain two separate oligonucleotides that can be chemically linked outside their annealing region by chemical linking groups. The "bare" ends of duplex nucleic acids have potent biological effects, including immune system stimulation. The effective elimination of one of the ends of the initial DsiRNA configuration also reduce the potential for immunostimulation, which is undesirable in some applications. Ends of dsRNA are also key points of entry for helicases and/or nucleases, thus dsRNA containing a linker should be more resistant to both.

Many suitable chemical linking groups are known in the art and can be used. Suitable groups will not block Dicer activity on the dsNA and will not interfere with the directed destruction of the RNA transcribed from the target gene. Alternatively, the two separate oligonucleotides can be linked by a third oligonucleotide such that a hairpin structure is produced upon annealing of the two oligonucleotides making up the dsNA composition. The hairpin structure will not block Dicer activity on the dsNA and will not interfere with the directed destruction of the RNA transcribed from the target gene. In particular embodiments, the hairpin structure comprises a tetraloop. The stability of the dsRNA is particularly enhanced by the inclusion of a tetraloop, which adopts a secondary structure with thermodynamic stability even under stringent conditions (e.g., 10mM NaHPO₄). Further advantages of such a dsRNA with a tetraloop are also contemplated.

One feature of the dsRNA compositions of the present invention is that they can serve as a substrate for Dicer. Typically, the dsRNA compositions of this invention will not have been treated with Dicer, other RNases, or extracts that contain them. In the current invention this type of pretreatment can prevent Dicer interaction. Several methods are known and can be used for determining whether a dsRNA composition serves as a substrate for Dicer. For example, Dicer activity can be measured in vitro using the Recombinant Dicer Enzyme Kit (Genlantis, San Diego, Calif.) according to the manufacturer's instructions. Dicer activity can be measured in vivo by treating cells with dsRNA and maintaining them for 24 h before harvesting them and isolating their RNA. RNA can be isolated using standard methods, such as with the RNeasy® Kit (Qiagen) according to the manufacturer's instructions. The isolated RNA can be separated on a 10% PAGE gel which is used to prepare a standard RNA blot that can be probed with a suitable labeled deoxyoligonucleotide, such as an oligonucleotide labeled with the Starfire® Oligo Labeling System (Integrated DNA Technologies, Inc., Coralville, Iowa).

The effect that a dsRNA has on a cell can depend upon the cell itself. In some circumstances a dsRNA could induce apoptosis or gene silencing in one cell type and not another. Thus, it is possible that a dsRNA could be suitable for use in one cell and not another. To be considered "suitable" a dsRNA composition need not be suitable under all possible circumstances in which it might be used, rather it need only be suitable under a particular set of circumstances.

### Substitutions and Modifications

Modifications can be included in the dsRNAs of the invention as described herein. Preferably, modifications are made such that the modification does not prevent the dsRNA composition from serving as a substrate for Dicer.

The introduction of substituted and modified nucleotides into Dicer substrate RNA molecules provides a way to overcome potential limitations of in vivo stability and bioavailability inherent to native RNA molecules (i.e., having standard nucleotides) that are exogenously delivered. For example, the use of modified nucleotides in Dicer substrate RNA molecules may enable a lower dose of a particular nucleic acid molecule for a given therapeutic effect, which is advantageuos if a modified nucleotides has an effect of a longer half-life in serum. Furthermore, certain substitutions and modifications can improve the bioavailability of Dicer substrate RNA by targeting particular cells or tissues or improving cellular uptake of the Dicer substrate RNA molecules. Therefore, even if the activity of a modified dsRNA as described herein is reduced as compared to a native RNA molecule, the overall activity of the substituted or modified Dicer substrate RNA molecule can be greater than that of the native RNA molecule due to improved stability or delivery of the molecule. Unlike native unmodified Dicer substrate RNA, substituted and modified Dicer substrate RNA can also reduce the possibility of activating the interferon response, or other immunomodulatory effects, in, e.g., humans.

In one embodiment, one or more modifications are made that enhance Dicer processing of the dsRNA. In a second embodiment, one or more modifications are made that result in more effective RNAi generation. In a third embodiment, one or more modifications are made that support a greater RNAi effect. In a fourth embodiment, one or more modifications are made that result in greater potency per each dsRNA molecule to be delivered to the cell. Modifications can be incorporated in the 3'-terminal region, the 5'-terminal region, in both the 3'-terminal and 5'-terminal region or in some instances in various positions within the sequence. With the restrictions noted above in mind any number and combination of modifications can be incorporated into the dsRNA. Where multiple modifications are present, they may be the same or different. Modifications to bases, sugar moieties, the phosphate backbone, and their combinations are contemplated. The 5' terminus of the sense strand can be phosphorylated.

Examples of modifications contemplated for the phosphate backbone include phosphonates, including methylphosphonate, phosphorothioate, and phosphotriester modifications such as alkylphosphotriesters, and the like. Examples of modifications contemplated for the sugar moiety include 2'-alkyl pyrimidine, such as 2'-O-methyl, T-fluoro, amino, and deoxy modifications and the like (see, e.g., Amarzguioui et al., 2003). Examples of modifications contemplated for the base groups include abasic sugars, 2-O-alkyl modified pyrimidines, 4-thiouracil, 5-bromouracil, 5-iodouracil, and 5-(3-aminoallyl)-uracil and the like. Locked nucleic acids, or LNA's, could also be incorporated. Many other modifications are known and can be used so long as the above criteria are satisfied. Examples of modifications are also disclosed in U.S. Pat. Nos. 5,684,143, 5,858,988 and 6,291,438 and in U.S. published patent application No. 2004/0203145 A1. Other modifications are disclosed in Herdewijn (2000), Eckstein (2000), Rusckowski et al. (2000), Stein et al. (2001); Vorobjev et al. (2001).

One or more modifications contemplated can be incorporated into either strand. The placement of the modifications in the DsiRNA can greatly affect the characteristics of the DsiRNA, including conferring greater potency and stability, reducing toxicity, enhancing Dicer processing, and minimizing an immune response.

In further embodiments, a double stranded nucleotide as described herein that decreases expression of a target gene by RNAi according to the instant disclosure further comprises one or more natural or synthetic non-standard nucleoside. In related embodiments, the non-standard nucleoside is one or more deoxyuridine, locked nucleic acid (LNA) molecule (e.g., a 5 -methyl uridine, LNA), or a universal-binding nucleotide. In certain embodiments, the universal-binding nucleotide can be C-phenyl, C-naphthyl, inosine, azole carboxamide, 1-β-D-ribofuranosyl-4-nitroindole, 1-β-D-ribofuranosyl-5-nitroindole, 1-β-D-ribofuranosyl-6-nitroindole, or 1-β-D-ribofuranosyl-3-nitropyrrole.

Substituted or modified nucleotides present in the double stranded nucleotide as described herein , preferably in the antisense strand, but also optionally in the sense or both strands, comprise modified or substituted nucleotides according to this disclosure having properties or characteristics similar to natural or standard ribonucleotides. For example, a double stranded nucleotide as described herein that may include nucleotides having a northern conformation (e.g., northern pseudorotation cycle, see, e.g., Saenger, Principles of Nucleic Acid Structure, Springer-Verlag Ed., 1984). As such, chemically modified nucleotides present in a double stranded nucleotide as described herein, preferably in the antisense strand, but also optionally in the passsenger or both strands, are resistant to nuclease degradation while at the same time maintaining the capacity to mediate RNAi. Exemplary nucleotides having a northern configuration include locked nucleic acid (LNA) nucleotides (e.g., 2'-O,4'-C-methylene-(D-ribofuranosyl) nucleotides); 2'-methoxyethyl (MOE) nucleotides; 2'-methyl-thio-ethyl, 2'-deoxy-2'-fluoro nucleotides. 2'-deoxy-2'-chloro nucleotides, 2'-azido nucleotides, 5-methyluridines, or 2'-O-methyl nucleotides. In certain embodiments, the LNA is a 5-methyluridine LNA.

As described herein, the first and second strands of a double stranded nucleotide as described herein or analog thereof provided by this disclosure can anneal or hybridize together (i.e., due to complementarity between the strands) to form at least one double-stranded region having a length of about 25 to about 30 base pairs. In other embodiments, the a double stranded nucleotide has at least one double-stranded region ranging in length from about 26 to about 40 base pairs or about 27 to about 30 base pairs or about 30 to about 35 base pairs. In other embodiments, the two or more strands of a double stranded nucleotide as described herein may optionally be covalently linked together by nucleotide or non-nucleotide linker molecules.

In certain embodiments, the a double stranded nucleotide as described herein or analog thereof comprises an overhang of one to four nucleotides on one or both 3'-ends, such as an overhang comprising a deoxyribonucleotide or two deoxyribonucleotides (e.g., thymidine, adenine). In some embodiments, a double stranded nucleotide or analogs thereof have a blunt end at one end of the Dicer substrate nucleic acid. In certain embodiments, the 5'-end of the first or second strand is phosphorylated. In any of the embodiments of a double stranded nucleotide as described herein, the 3'-terminal nucleotide overhangs can comprise ribonucleotides or deoxyribonucleotides that are chemically-modified at a nucleic acid sugar, base, or backbone. In any of the embodiments of a double stranded nucleotide as described herein , the 3'-terminal nucleotide overhangs can comprise one or more universal ribonucleotides. In any of the embodiments of a double stranded nucleotide as described herein , the 3'-terminal nucleotide overhangs can comprise one or more acyclic nucleotides. In any of the embodiments of a double stranded nucleotide as described herein, the a double stranded nucleotides can further comprise a terminal phosphate group, such as a 5'-phosphate (see Martinez et al., Cell. 110:563-574, 2002; and Schwarz et al., Molec. Cell 10:537-568, 2002) or a 5',3'-diphosphate.

As set forth herein, the terminal structure of a double stranded nucleotide as described herein that decrease expression of a target gene by, e.g., RNAi, may either have a blunt end or an overhang. In certain embodiments, the overhang may be at the 3' terminus of the antisense strand or the 5' terminus of the sense strand. Furthermore, since the overhanging sequence may have low specificity to a target gene, it is not necessarily complementary (antisense) or identical (sense) to a target gene sequence. In further embodiments, a double stranded nucleotide as described herein that decreases expression of a target gene by RNAi may further comprise a low molecular weight structure (e.g., a natural nucleic acid molecule such as a tRNA, rRNA or viral nucleic acid, or an artificial nucleic acid molecule) at, e.g., one or more overhanging portion of the Dicer substrate nucleic acid.

In further embodiments, a a double stranded nucleotide as described herein that decreases expression of a target gene by RNAi according to the instant disclosure further comprises a 2'-sugar substitution, such as 2'-deoxy, 2'-O-methyl, 2'-O-methoxyethyl, 2'-O-2-methoxyethyl, halogen, 2'-fluoro, 2'-O-allyl, or the like, or any combination thereof. In still further embodiments, a double stranded nucleotide as described herein that decreases expression of a target gene by RNAi according to the instant disclosure further comprises a terminal cap substituent on one or both ends of the first strand or second strand, such as an alkyl, abasic, deoxy abasic, glyceryl, dinucleotide, acyclic nucleotide, inverted deoxynucleotide moiety, or any combination thereof. In certain embodiments, at least one or two 5'-terminal ribonucleotides of the sense strand within the double-stranded region have a 2'-sugar substitution. In certain other embodiments, at least one or two 5'-terminal ribonucleotides of the antisense strand within the double-stranded region have a 2'-sugar substitution. In certain embodiments, at least one or two 5'-terminal ribonucleotides of the sense strand and the antisense strand within the double-stranded region have a 2'-sugar substitution.

In yet other embodiments, a double stranded nucleotide as described herein that decreases expression of a target gene (including an mRNA splice variant thereof) by RNAi according to the instant disclosure further comprises at least one modified internucleoside linkage, such as independently a phosphorothioate, chiral phosphorothioate, phosphorodithioate, phosphotriester, aminoalkylphosphotriester, methyl phosphonate, alkyl phosphonate, 3'-alkylene phosphonate, 5'-alkylene phosphonate, chiral phosphonate, phosphonoacetate, thiophosphonoacetate, phosphinate, phosphoramidate, 3'-amino phosphoramidate, aminoalkylphosphoramidate, thionophosphoramidate, thionoalkylphosphonate, thionoalkylphosphotriester, selenophosphate, boranophosphate linkage, or any combination thereof.

A modified internucleotide linkage, as described herein, can be present in one or more strands of a a double stranded nucleotide as described herein , e.g., in the antisense strand, the sense strand, both strands, or a plurality of strands. The a double stranded nucleotide as described herein of this disclosure can comprise one or more modified internucleotide linkages at the 3'-end, the 5'-end, or both of the 3'- and 5'-ends of the antisense strand or the sense strand or both strands. In one embodiment, a a double stranded nucleotide capable of decreasing expression of a target gene (including a specific or selected mRNA splice variant thereof) by RNAi has one modified internucleotide linkage at the 3'-end, such as a phosphorothioate linkage. For example, this disclosure provides a a double stranded nucleotide capable of decreasing expression of a target gene by RNAi having about 1 to about 8 or more phosphorothioate internucleotide linkages in one Dicer substrate nucleic acid strand. In yet another embodiment, this disclosure provides a a double stranded nucleotide capable of decreasing expression of a target gene by RNAi having about 1 to about 8 or more phosphorothioate internucleotide linkages in both Dicer substrate nucleic acid strands. In other embodiments, an exemplary a double stranded nucleotide of this disclosure can comprise from about 1 to about 5 or more consecutive phosphorothioate internucleotide linkages at the 5'-end of the sense strand, the antisense strand, both strands, or a plurality of strands. In another example, an exemplary a double stranded nucleotide of this disclosure can comprise one or more pyrimidine phosphorothioate internucleotide linkages in the sense strand, the antisense strand, both strands, or a plurality of strands. In yet another example, an exemplary dsRNA molecule of this disclosure can comprise one or more purine phosphorothioate internucleotide linkages in the sense strand, the antisense strand, both strands or a plurality of strands.

In another aspect of the instant disclosure, there is provided a double stranded nucleotide that decreases expression of a target gene, comprising a first strand that is complementary to a target mRNA and a second strand that is complementary to the first strand, wherein the first and second strands form a double-stranded region of about 25 to about 30 base pairs or about 25 to about 40 base pairs; wherein at least one base of the dsRNA is substituted with a base analog.

Base analogs include those disclosed in US Pat. Nos. 5,432,272 and 6,001,983 to Benner and US Patent Publication No. 20080213891 to Manoharan. Non-limiting examples of bases include hypoxanthine (I), xanthine (X), 3β-D-ribofuranosyl-(2,6-diaminopyrimidine; K), 3-β-D-ribofuranosyl-(1-methyl-pyrazolo[4,3-d]pyrimidine-5,7(4H,6H)-dione; P), iso-cytosine (iso-C), iso- guanine (iso-G), 1-β-D-ribofuranosyl-(5-nitroindole), 1-β-D-ribofuranosyl-(3-nitropyrrole), 5-bromouracil, 2-aminopurine, 4-thio-dT, 7-(2-thienyl)-imidazo[4,5- bjpyridine (Ds) and pyrrole-2-carbaldehyde (Pa), 2-amino-6-(2-thienyl)purine (S), 2- oxopyridine (Y), difluorotolyl, 4-fluoro-6-methylbenzimidazole, 4-methylbenzimidazole, 3-methyl isocarbostyrilyl, 5-methyl isocarbostyrilyl, and 3-methyl-7-propynyl isocarbostyrilyl, 7-azaindolyl, 6-methyl-7-azaindolyl, imidizopyridinyl, 9-methyl-imidizopyridinyl, pyrrolopyrizinyl, isocarbostyrilyl, 7- propynyl isocarbostyrilyl, propynyl-7-azaindolyl, 2,4,5-trimethylphenyl, 4- methylindolyl, 4,6-dimethylindolyl, phenyl, napthalenyl, anthracenyl, phenanthracenyl, pyrenyl, stilbenzyl, tetracenyl, pentacenyl, and structural derivatives thereof (Schweitzer et al., J. Org. Chem., 59:7238-7242 (1994); Berger et al., Nucleic Acids Research, 28(15):2911-2914 (2000); Moran et al., J. Am. Chem. Soc, 1 19:2056-2057 (1997); Morales et al., J. Am. Chem. Soc, 121 :2323-2324 (1999); Guckian et al., J. Am. Chem. Soc, 118:8182-8183 (1996); Morales et al., J. Am. Chem. Soc, 122(6): 1001 -1007 (2000); McMinn et al., J. Am. Chem. Soc, 121 :1 1585-11586 (1999); Guckian et al., J. Org. Chem., 63:9652-9656 (1998); Moran et al., Proc. Natl. Acad. Sci., 94:10506-1051 1 (1997); Das et al., J. Chem. Soc, Perkin Trans., 1 :197-206 (2002); Shibata et al., J. Chem. Soc, Perkin Trans., 1 : 1605-161 1 (2001); Wu et al., J. Am. Chem. Soc, 122(32):7621-7632 (2000); O'Neill et al., J. Org. Chem., 67:5869- 5875 (2002); Chaudhuri et al., J. Am. Chem. Soc, 1 17:10434-10442 (1995); and U.S. Pat. No. 6,218,108.). Base analogs may also be a universal base.

In certain embodiments, the first and second strands of double stranded nucleotide as described herein, which decreases expression of a target gene and has at least one base analog, that can anneal, duplex, or hybridize together (i.e., due to complementarity between the strands) to form at least one double-stranded region having a length or a combined length of about 25 to about 30 base pairs or about 25 to about 40 base pairs. In some embodiments, double stranded nucleotide has at least one double-stranded region ranging in length from about 25 base pairs to about 30 base pairs. In other embodiments, the Dicer substrate nucleic acid has at least one double-stranded region ranging in length from about 21 to about 40 base pairs or about 21 to about 30 base pairs or about 25 to about 30 base pairs. In certain embodiments, the double stranded nucleotide or analog thereof has an overhang of one to four nucleotides on one or both 3'-ends, such as an overhang comprising a deoxyribonucleotide or two deoxyribonucleotides (e.g., thymidine). In some embodiments, Dicer substrate nucleic acid molecule or analog thereof has a blunt end at one or both ends of the double stranded nucleotide as described herein . In certain embodiments, the 5'-end of the first or second strand is phosphorylated.

In further embodiments, at least one pyrimidine nucleoside of the double stranded nucleotide as described herein is a locked nucleic acid (LNA) in the form of a bicyclic sugar, wherein R² is oxygen, and the 2'-O and 4'-C form an oxymethylene bridge on the same ribose ring. In a related embodiment, the LNA is having a base substitution, such as a 5-methyluridine LNA. In other embodiments, at least one, at least three, or all uridines of the first strand of the Dicer substrate nucleic acid are replaced with 5-methyluridine or 5-methyluridine LNA, or at least one, at least three, or all uridines of the second strand of the Dicer substrate nucleic acid are replaced with 5-methyluridine, 5-methyluridine LNA, or any combination thereof (e.g., such changes are made on both strands, or some substitutions include 5-methyluridine only, 5-methyluridine LNA only, or one or more 5-methyluridine with one or more 5-methyluridine LNA).

In still further embodiments, a double stranded nucleotide or analog thereof according to the instant disclosure further comprises a terminal cap substituent on one or both ends of the first strand or second strand, such as an alkyl, abasic, deoxy abasic, glyceryl, dinucleotide, acyclic nucleotide, inverted deoxynucleotide moiety, or any combination thereof. In further embodiments, one or more internucleoside linkage can be optionally modified. For example, a double stranded nucleotide as described herein or one containing an analog thereof of a modified nucleotide according to the instant disclosure wherein at least one internucleoside linkage is modified to a phosphorothioate, chiral phosphorothioate, phosphorodithioate, phosphotriester, aminoalkylphosphotriester, methyl phosphonate, alkyl phosphonate, 3'-alkylene phosphonate, 5'-alkylene phosphonate, chiral phosphonate, phosphonoacetate, thiophosphonoacetate, phosphinate, phosphoramidate, 3'-amino phosphoramidate, aminoalkylphosphoramidate, thionophosphoramidate, thionoalkylphosphonate, thionoalkylphosphotriester, selenophosphate, boranophosphate linkage, or any combination thereof.

In still another embodiment, a double stranded nucleotide as described herein that decreases expression of a target gene by RNAi, comprising a first strand that is complementary to a target mRNA and a second strand that is complementary to the first strand, wherein the first and second strands form a non-overlapping double-stranded region of about 25 to about 30 base pairs or about 25 to about 40 base pairs. Any of the substitutions or modifications described herein are contemplated within this embodiment as well.

In another exemplary of this disclosure, the double stranded nucleotide as described herein comprise at least two or more modified nucleosides can each be independently selected from a base analog that comprises any chemical modification or substitution as contemplated herein, e.g., an alkyl (e.g., methyl), halogen, hydroxy, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, carbonyl, alkanoylamino, carbamoyl, carbonylamino, alkylsulfonylamino, or heterocyclo group. When two or more modified ribonucleotides are present, each modified ribonucleotide can be independently modified to have the same, or different, modification or substitution in the base analog and the C2 position on the furanose ring.

In other detailed embodiments, one or more modified nucleosides, including those with a base analog described in this disclosure can be located at any ribonucleotide position, or any combination of ribonucleotide positions, on either or both of the antisense and sense strands of a double stranded nucleotide of this disclosure, including at one or more multiple terminal positions as noted above, or at any one or combination of multiple non-terminal ("internal") positions. In this regard, each of the sense and antisense strands can incorporate about 1 to about 6 or more of the substituted nucleosides.

In certain embodiments, when two or more modified nucleosides are incorporated within a double stranded nucleotide as described herein, at least one of the modified nucleosides will be at a 3'- or 5'-end of one or both strands, and in certain embodiments at least one of the substituted pyrimidine nucleosides will be at a 5'-end of one or both strands. In other embodiments, the modified nucleosides are located at a position corresponding to a position of a pyrimidine in an unmodified double stranded nucleotide as described herein that is constructed as a homologous sequence for targeting a cognate mRNA, as described herein.

Substituting modified nucleosides into a double stranded nucleotide as described herein will often increase resistance to enzymatic degradation, such as exonucleolytic degradation, including 5'-exonucleolytic or 3'-exonucleolytic degradation. As such, the double stranded nucleotide as described herein will exhibit significant resistance to enzymatic degradation compared to a corresponding double stranded nucleotide having standard nucleotides, and will thereby possess greater stability, increased half-life, and greater bioavailability in physiological environments (e.g., when introduced into a eukaryotic target cell). In addition to increasing resistance of the substituted or modified Dicer substrate RNAs to exonucleolytic degradation, the incorporation of one or more modified nucleosides having a base analog described in this disclosure will render double stranded nucleotide as described herein more resistant to other enzymatic or chemical degradation processes, and thus more stable and bioavailable than otherwise identical Dicer substrate RNAs that do not include the substitutions or modifications. In related aspects of this disclosure, double stranded nucleotide substitutions or modifications described herein will often improve stability of a modified double stranded nucleotide for use within research, diagnostic and treatment methods wherein the modified Dicer substrate nucleic acid is contacted with a biological sample, e.g., a mammalian cell, intracellular compartment, serum or other extra cellular fluid, tissue, or other in vitro or in vivo physiological compartment or environment. In one embodiment, diagnosis is performed on an isolated biological sample. In another embodiment, the diagnostic method is performed in vitro. In a further embodiment, the diagnostic method is not performed (directly) on a human or animal body.

In addition to increasing stability of substituted or modified double stranded nucleotide as described herein, incorporation of one or more modified nucleosides with base analogs described in this disclosure in a Dicer substrate nucleic acid designed for gene silencing will yield additional desired functional results, including increasing a melting point of a substituted or modified Dicer substrate nucleic acid compared to a corresponding, unmodified double stranded nucleotide. By thus increasing a double stranded nucleotide melting point, the subject substitutions or modifications will often block or reduce the occurrence or extent of partial dehybridization of the substituted or modified double stranded nucleotide (that would ordinarily occur and render the unmodified double stranded nucleotide more vulnerable to degradation by certain exonucleases), thereby increasing the stability of the substituted or modified double stranded nucleotide.

In another aspect of this disclosure, substitutions or modifications of double stranded nucleotide as described herein will reduce "off-target effects" of the substituted or modified double stranded nucleotide when they are contacted with a biological sample (e.g., when introduced into a target eukaryotic cell having specific, and non-specific mRNA species present as potential specific and non-specific targets). In related embodiments, substituted or modified double stranded nucleotide according to this disclosure are employed in methods of gene silencing, wherein the substituted or modified double stranded nucleotide as described herein exhibit reduced or eliminated off target effects compared to a corresponding, unmodified double stranded nucleotide, e.g., as determined by non-specific activation of genes in addition to a target (i.e., homologous or cognate) gene in a cell or other biological sample to which the modified double stranded nucleotide is exposed under conditions that allow for gene silencing activity to be detected.

In further embodiments, double stranded nucleotide of this disclosure can comprise one or more sense (second) strand that is homologous or corresponds to a sequence of a target gene and an antisense (first) strand that is complementary to the sense strand and a sequence of the target gene. In exemplary embodiments, at least one strand of the double stranded nucleotide as described herein incorporates one or more base analogs described in this disclosure (e.g., wherein a pyrimidine is replaced by more than one 5-methyluridine or the ribose is modified to incorporate a 2'-O-methyl substitution or any combination thereof). These and other multiple substitutions or modifications described in this disclosure can be introduced into one or more pyrimidines, or into any combination and up to all pyrimidines present in one or both strands of a double stranded nucleotide as described herein.

Within certain aspects, the present disclosure provides double stranded nucleotide that decreases expression of a target gene by RNAi, and compositions comprising one or more double stranded nucleotide as described herein, wherein at least one double stranded nucleotide comprises one or more universal-binding nucleotide(s) in the first, second or third position in the anti-codon of the antisense strand of the double stranded nucleotide duplex and wherein the double stranded nucleotide as described herein is capable of specifically binding to a target sequence, such as an nucleic acid expressed by a target cell. In cases wherein the sequence of a target nucleic acid includes one or more single nucleotide substitutions, double stranded nucleotide comprising a universal-binding nucleotide retains its capacity to specifically bind a target nucleic acid, thereby mediating gene silencing and, as a consequence, overcoming escape of the target from dsRNA-mediated gene silencing. Non-limiting examples of universal-binding nucleotides that may be suitably employed in the compositions and methods disclosed herein include inosine, 1-β-D-ribofuranosyl-5-nitroindole, and 1-β-D-ribofuranosyl-3-nitropyrrole. For the purpose of the present disclosure, a universal-binding nucleotide is a nucleotide that can form a hydrogen bonded nucleotide pair with more than one nucleotide type.

Non-limiting examples for the above compositions includes modifying the anti-codons for tyrosine (AUA) or phenylalanine (AAA or GAA), cysteine (ACA or GCA), histidine (AUG or GUG), asparagine (AUU or GUU), isoleucine (UAU) and aspartate (AUC or GUC) within the anti-codon of the antisense strand of the dsRNA molecule.

For example, within certain embodiments, the isoleucine anti-codon UAU, for which AUA is the cognate codon, may be modified such that the third-position uridine (U) nucleotide is substituted with the universal-binding nucleotide inosine (I) to create the anti-codon UAI. Inosine is an exemplary universal-binding nucleotide that can nucleotide-pair with an adenosine (A), uridine (U), and cytidine (C) nucleotide, but not guanosine (G). This modified anti-codon UAI increases the specific-binding capacity of the Dicer substrate nucleic acid molecule and thus permits the Dicer substrate nucleic acid to pair with mRNAs having any one of AUA, UUA, and CUA in the corresponding position of the coding strand thereby expanding the number of available nucleic acid degradation targets to which the Dicer substrate nucleic acid may specifically bind.

Alternatively, the anti-codon AUA may also or alternatively be modified by substituting a universal-binding nucleotide in the third or second position of the anti-codon such that the anti-codon(s) represented by UAI (third position substitution) or UIU (second position substitution) to generate Dicer substrate nucleic acid that are capable of specifically binding to AUA, CUA and UUA and AAA, ACA and AUA.

In certain aspects, double stranded nucleotide disclosed herein can include from about 1 universal-binding nucleotide and about 10 universal-binding nucleotides. Within certain aspects, the presently disclosed double stranded nucleic acid may comprise a sense strand that is homologous to a sequence of a target gene and an antisense strand that is complementary to the sense strand, with the proviso that at least one nucleotide of the antisense strand of the otherwise complementary Dicer substrate nucleic acid duplex is replaced by one or more universal-binding nucleotide.

By way of background, within the silencing complex, the double stranded nucleotide as described herein is positioned so that a target nucleic acid can interact with it. The RISC will encounter thousands of different RNAs that are in a typical cell at any given moment. But, the double stranded nucleotide as described herein, which is a Dicer substrate nucleic acid, loaded in RISC will adhere well to a target nucleic acid that has close complementarity with the antisense of the Dicer substrate nucleic acid molecule. So, unlike an interferon response to a viral infection, the silencing complex is highly selective in choosing a target nucleic acid. RISC cleaves the captured target nucleic acid strand in two and releases the two pieces of the nucleic acid (now rendered incapable of directing protein synthesis) and moves on. RISC itself stays intact and is capable of finding and cleaving additional target nucleic acid molecules.

It will be understood that, regardless of the position at which the one or more universal-binding nucleotide is substituted, the double stranded nucleotide as described herein is capable of binding to a target gene and one or more variant(s) thereof thereby facilitating the degradation of the target gene or variant thereof via a RISC complex. Thus, the double stranded nucleotides of the present disclosure are suitable for introduction into cells to mediate targeted post-transcriptional gene silencing of a target gene or variants thereof.

In one embodiment, the antisense strand or the sense strand or both strands have one or more 2'-O-methyl modified nucleotides. In another embodiment, the antisense strand contains 2'-O-methyl modified nucleotides. In another embodiment, the antisense stand contains a 3' overhang that is comprised of 2'-O-methyl modified nucleotides. The antisense strand could also include additional 2'-O-methyl modified nucleotides.

Additionally, the dsRNA structure can be optimized to ensure that the oligonucleotide segment generated from Dicer's cleavage will be the portion of the oligonucleotide that is most effective in inhibiting gene expression. For example, in one embodiment of the invention a 27-bp oligonucleotide of the dsRNA structure is synthesized wherein the anticipated 21 to 22-bp segment that will inhibit gene expression is located on the 3'-end of the antisense strand. The remaining bases located on the 5'-end of the antisense strand will be cleaved by Dicer and will be discarded. This cleaved portion can be homologous (i.e., based on the sequence of the target sequence) or non-homologous and added to extend the nucleic acid strand.

### Preparation of Double-Stranded RNA Oligonucleotides

### Oligonucleotide Synthesis and Purification

DsiRNA molecules can be designed to interact with various sites in the RNA message, for example, target sequences within the RNA sequences described herein. The sequence of one strand of the DsiRNA molecule(s) is complementary to the target site sequences described above. The DsiRNA molecules can be chemically synthesized using methods described herein. Inactive DsiRNA molecules that are used as control sequences can be synthesized by scrambling the sequence of the DsiRNA molecules such that it is not complementary to the target sequence.

RNA may be produced enzymatically or by partial/total organic synthesis, and modified ribonucleotides can be introduced by in vitro enzymatic or organic synthesis. In one embodiment, each strand is prepared chemically. Methods of synthesizing RNA molecules are known in the art, in particular, the chemical synthesis methods as described in Verma and Eckstein (1998) or as described herein. Generally, DsiRNA constructs can by synthesized using solid phase oligonucleotide synthesis methods as described for 19-23mer siRNAs (see for example Usman et al., U.S. Pat. Nos. 5,804,683; 5,831,071; 5,998,203; 6,117,657; 6,353,098; 6,362,323; 6,437,117; 6,469,158; Scaringe et al., U.S. Pat. Nos. 6,111,086; 6,008,400; 6,111,086).

In a non-limiting example, RNA oligonucleotides are synthesized using solid phase phosphoramidite chemistry, deprotected and desalted on NAP-5 columns (Amersham Pharmacia Biotech, Piscataway, N.J.) using standard techniques (Damha and Olgivie, 1993; Wincott et al., 1995). The oligomers are purified using ionexchange high performance liquid chromatography (IE-HPLC) on an Amersham Source 15Q column (1.0 cm.times.25 cm; Amersham Pharmacia Biotech, Piscataway, N.J.) using a 15 min step-linear gradient. The gradient varies from 90:10 Buffers A:B to 52:48 Buffers A:B, where Buffer A is 100 mM Tris pH 8.5 and Buffer B is 100 mM Tris pH 8.5, 1 M NaCl. Samples are monitored at 260 nm and peaks corresponding to the full-length oligonucleotide species are collected, pooled, desalted on NAP-5 columns, and lyophilized.

The purity of each oligomer is determined by capillary electrophoresis (CE) on a Beckman PACE 5000 (Beckman Coulter, Inc., Fullerton, Calif.). The CE capillaries has a 100 um inner diameter and contains ssDNA 100R Gel (Beckman-Coulter). Typically, about 0.6 nmole of oligonucleotide is injected into a capillary, run in an electric field of 444 V/cm and detected by UV absorbance at 260 nm. Denaturing Tris-Borate-7 M-urea running buffer is purchased from Beckman-Coulter. Oligoribonucleotides are obtained that are at least 90% pure as assessed by CE for use in experiments described below. Compound identity is verified by matrix-assisted laser desorption ionization time-of-flight (MALDI-TOF) mass spectroscopy on a Voyager DE.TM. Biospectometry Work Station (Applied Biosystems, Foster City, Calif.) following the manufacturer's recommended protocol. Relative molecular masses of all oligomers can be obtained, often within 0.2% of expected molecular mass.

### Preparation of Duplexes

For example, single-stranded RNA (ssRNA) oligomers are resuspended at 100 uM concentration in duplex buffer consisting of 100 mM potassium acetate, 30 mM HEPES, pH 7.5. Complementary sense and antisense strands are mixed in equal molar amounts to yield a final solution of 50 uM duplex. Samples are heated to 95°C for 5' and allowed to cool to room temperature before use. Double-stranded RNA (dsRNA) oligomers are stored at -20°C. Single-stranded RNA oligomers are stored lyophilized or in nuclease-free water at -80°C.

### Double stranded RNAs in RNA Interference

Methods of RNA interference may also be used in the practice of the dsRNAs of the invention. See, e.g., Scherer and Rossi, Nature Biotechnology 2 1:1457-65 (2003) for a review on sequence-specific mRNA knockdown of using antisense oligonucleotides, ribozymes, DNAzymes. See also, International Patent Application PCT/US2003/030901 (Publication No. WO 2004-029219 A2), filed September 29, 2003 and entitled "Cell-based RNA Interference and Related Methods and Compositions." The controllable inhibition of the expression of a target gene may be effected by controlling the synthesis of the product of the target gene in the target cell (e.g., the hepatocytes in the liver cancer model). WO2008021393

For example, in evaluating the effect of a nicked dsRNA with a tetraloop, appropriate references would include a dsRNA having the same nucleotide sequences with a nick and no tetraloop, a dsRNA having the same nucleotide sequences with no nick and a tetraloop, and a dsRNA having the same nucleotide sequences with no nick and no tetraloop to determine respectively the effect of the nick, the effect of the tetraloop, or the combined effect of the nick and the tetraloop, *e.g.,* a synergistic effect. Any structural features, *e.g.,* nicks, or modifications should be present in corresponding positions in both dsRNAs being compared.

Similarly, in evaluating the effect of a dsRNA with modified nucleotide(s), appropriate references would include a dsRNA having the nucleotide sequences with common RNA or DNA nucleotides (A, C, G, T, U) at the position(s) corresponding to those of the modified nucleotide(s) to determine respectively the effect of a modified nucleotide, or the combined effect of the modified nucleotides, e.g., a synergistic effect. In placing common nucleotides (A, C, G, T, U) in a reference dsRNA at the position(s) corresponding to those of the modified nucleotide(s), the common nucleotides presereve the double helical structure of the phosphate backbone of the duplex. As above, any structural features, *e.g.,* overhangs, nicks, or loops, should be present in corresponding positions in both dsRNAs being compared.

In assays involving the dsRNAs of the invention, *e.g.,* cell culture assays of RNA interference, in vitro assays, and in vivo assays, an appropriate reference is also a negative control, which represents an experimental condition in which no effect is observed or expected to be observed. For example, in evaluating the in vitro or in vivo effects of a dsRNA of the invention, it is appropriate to use as a negative control treatment with buffer alone or another dsRNA with the same nucleotide composition, but in which the nucleotide sequence is scrambled.

### RNAi In Vitro Assay to Assess DsiRNA Activity

An *in vitro* assay that recapitulates RNAi in a cell-free system is used to evaluate DsiRNA constructs. For example, such an assay comprises a system described by Tuschl et al., 1999, Genes and Development, 13, 3191-3197 and Zamore et al., 2000, Cell, 101, 25-33 adapted for use with DsiRNA agents directed against target RNA. A Drosophila extract derived from syncytial blastoderm is used to reconstitute RNAi activity in vitro. Target RNA is generated via in vitro transcription from an appropriate plasmid using T7 RNA polymerase or via chemical synthesis. Sense and antisense DsiRNA strands (for example 20 uM each) are annealed by incubation in buffer (such as 100 mM potassium acetate, 30 mM HEPES-KOH, pH 7.4, 2 mM magnesium acetate) for 1 minute at 90°C followed by 1 hour at 37°C, then diluted in lysis buffer (for example 100 mM potassium acetate, 30 mM HEPES-KOH at pH 7.4, 2 mM magnesium acetate). Annealing can be monitored by gel electrophoresis on an agarose gel in TBE buffer and stained with ethidium bromide. The Drosophila lysate is prepared using zero to two-hour-old embryos from Oregon R flies collected on yeasted molasses agar that are dechorionated and lysed. The lysate is centrifuged and the supernatant isolated. The assay comprises a reaction mixture containing 50% lysate [vol/vol], RNA (10-50 pM final concentration), and 10% [vol/vol] lysis buffer containing DsiRNA (10 nM final concentration). The reaction mixture also contains 10 mM creatine phosphate, 10 ug/ml creatine phosphokinase, 100 um GTP, 100 uM UTP, 100 uM CTP, 500 uM ATP, 5 mM DTT, 0.1 U/uL RNasin (Promega), and 100 uM of each amino acid. The final concentration of potassium acetate is adjusted to 100 mM. The reactions are pre-assembled on ice and preincubated at 25°C for 10 minutes before adding RNA, then incubated at 25°C for an additional 60 minutes. Reactions are quenched with 4 volumes of 1.25xPassive Lysis Buffer (Promega). Target RNA cleavage is assayed by RT-PCR analysis or other methods known in the art and are compared to control reactions in which DsiRNA is omitted from the reaction.

Alternately, internally-labeled target RNA for the assay is prepared by in vitro transcription in the presence of [alpha-32P] CTP, passed over a G50 Sephadex column by spin chromatography and used as target RNA without further purification. Optionally, target RNA is 5'-32P-end labeled using T4 polynucleotide kinase enzyme. Assays are performed as described above and target RNA and the specific RNA cleavage products generated by RNAi are visualized on an autoradiograph of a gel. The percentage of cleavage is determined by PHOSPHOR IMAGER® (autoradiography) quantitation of bands representing intact control RNA or RNA from control reactions without DsiRNA and the cleavage products generated by the assay.

### Nucleic Acid Inhibition of Target RNA

DsiRNA molecules targeted to the genomic RNA are designed and synthesized as described above. These nucleic acid molecules can be tested for cleavage activity in vivo, for example, using the following procedure. Two formats are used to test the efficacy of DsiRNAs. First, the reagents are tested in cell culture using, for example, human hepatoma (Huh7) cells, to determine the extent of RNA and protein inhibition. DsiRNA reagents are selected against the target as described herein. RNA inhibition is measured after delivery of these reagents by a suitable transfection agent to, for example, cultured epidermal keratinocytes. Relative amounts of target RNA are measured versus actin using real-time PCR monitoring of amplification (eg., ABI 7700 TAQMAN). A comparison is made to a mixture of oligonucleotide sequences made to unrelated targets or to a randomized DsiRNA control with the same overall length and chemistry, but randomly substituted at each position. Primary and secondary lead reagents are chosen for the target and optimization performed. After an optimal transfection agent concentration is chosen, a RNA time-course of inhibition is performed with the lead DsiRNA molecule. In addition, a cell-plating format can be used to determine RNA inhibition.
In addition, a cell-plating format can also be used to determine RNA inhibition.

### Delivery of DsiRNA to Cells

Cells stably transfected with the DsiRNA are seeded, for example, at 8.5x103 cells per well of a 96-well platein DMEM(Gibco) the day before transfection. DsiRNA (final concentration, for example, 200pM, InM, 10nM or 25 nM) and cationic lipid Lipofectamine2000 (e.g., final concentration 0.5 ul/well) are complexed in Optimem (Gibco) at 37°C for 20 minutes inpolypropelyne microtubes. Following vortexing, the complexed DsiRNA is added to each well and incubated for 24-72 hours.

### TAQMAN® (Real-Time PCR Monitoring of Amplification) and Lightcycler Quantification of mRNA

Total RNA is prepared from cells following DsiRNA delivery, for example, using Ambion Rnaqueous 4-PCR purification kit for large scale extractions, or Ambion Rnaqueous-96 purification kit for 96-well assays. For Taqman analysis, dual-labeled probes are synthesized with, for example, the reporter dyes FAM or VIC covalently linked at the 5'-end and the quencher dye TAMARA conjugated to the 3'-end. One-step RT-PCR amplifications are performed on, for example, an ABI PRISM 7700 Sequence detector using 50 uL reactions consisting of 10 uL total RNA, 100 nM forward primer, 100 mM reverse primer, 100 nM probe, 1xTaqMan PCR reaction buffer (PE-Applied Biosystems), 5.5 mM MgCl2, 100 uM each dATP, dCTP, dGTP and dTTP, 0.2U RNase Inhibitor (Promega), 0.025U AmpliTaq Gold (PE-Applied Biosystems) and 0.2U M-MLV Reverse Transcriptase (Promega). The thermal cycling conditions can consist of 30 minutes at 48°C, 10 minutes at 95°C, followed by 40 cycles of 15 seconds at 95°C and 1 minute at 60°C. Quantitation of target mRNA level is determined relative to standards generated from serially diluted total cellular RNA (300, 100, 30, 10 ng/rxn) and normalizing to, for example, 36B4 mRNA in either parallel or same tube TaqMan reactions. For RNA quantitation, appropriate PCR primers and probe(s) specific for control genes are used.

### Western Blotting

Nuclear extracts can be prepared using a standard micro preparation technique (see for example Andrews and Faller, 1991, Nucleic Acids Research, 19, 2499). Protein extracts from supernatants are prepared, for example using TCA precipitation. An equal volume of 20% TCA is added to the cell supernatant, incubated on ice for 1 hour and pelleted by centrifugation for 5 minutes. Pellets are washed in acetone, dried and resuspended in water. Cellular protein extracts are run on a 10% Bis-Tris NuPage (nuclear extracts) or 4-12% Tris-Glycine (supernatant extracts) polyacrylamide gel and transferred onto nitro-cellulose membranes. Non-specific binding can be blocked by incubation, for example, with 5% non-fat milk for 1 hour followed by primary antibody for 16 hour at 4°C. Following washes, the secondary antibody is applied, for example (1:10,000 dilution) for 1 hour at room temperature and the signal detected with SuperSignal reagent (Pierce).

### RNAi Mediated Inhibition of Target Expression

DsiRNA constructs are tested for efficacy in reducing target RNA expression, for example using the following protocol. Cells are plated approximately 24 hours before transfection in 96-well plates at 5,000-7,500 cells/well, 100 ul/well, such that at the time of transfection cells are 70-90% confluent. For transfection, annealed DsiRNAs are mixed with the transfection reagent (Lipofectamine 2000, Invitrogen) in a volume of 50 ul/well and incubated for 20 minutes at room temperature. The DsiRNA transfection mixtures are added to cells to give a final DsiRNA concentration of 50 pM, 200 pM, or 1 nM in a volume of 150 ul. Each DsiRNA transfection mixture is added to 3 wells for triplicate DsiRNA treatments. Cells are incubated at 37°C for 24 hours in the continued presence of the DsiRNA transfection mixture. At 24 hours, RNA is prepared from each well of treated cells. The supernatants with the transfection mixtures are first removed and discarded, then the cells are lysed and RNA prepared from each well. Target RNA level or expression following treatment is evaluated by RT-PCR for the target gene and for a control gene (36B4, an RNA polymerase subunit) for normalization. Triplicate data is averaged and the standard deviations determined for each treatment. Normalized data are graphed and the percent reduction of target mRNA by active DsiRNAs in comparison to their respective control DsiRNAs (e.g., inverted control DsiRNAs) is determined.

### Serum Stability for DsiRNAs

Serum stability of DsiRNA agents is assessed via incubation of DsiRNA agents in 50% fetal bovine serum for various periods of time (up to 24 h) at 37°C. Serum is extracted and the nucleic acids are separated on a 20% non-denaturing PAGE and visualized with Gelstar stain. Relative levels of protection from nuclease degradation are assessed for DsiRNAs (optionally with and without modifications).

### RNA Interference Based Therapy

As is known, RNAi methods are applicable to a wide variety of genes in a wide variety of organisms and the disclosed compositions and methods can be utilized in each of these contexts. Examples of genes which can be targeted by the disclosed compositions and methods include endogenous genes which are genes that are native to the cell or to genes that are not normally native to the cell. Without limitation these genes include oncogenes, cytokine genes, idiotype (Id) protein genes, prion genes, genes that expresses molecules that induce angiogenesis, genes for adhesion molecules, cell surface receptors, proteins involved in metastasis, proteases, apoptosis genes, cell cycle control genes, genes that express EGF and the EGF receptor, multi-drug resistance genes, such as the MDR1 gene.

More specifically, the target mRNA of the invention specifies the amino acid sequence of a cellular protein (e.g., a nuclear, cytoplasmic, transmembrane, or membrane-associated protein). In another embodiment, the target mRNA of the invention specifies the amino acid sequence of an extracellular protein (e.g., an extracellular matrix protein or secreted protein). As used herein, the phrase "specifies the amino acid sequence" of a protein means that the mRNA sequence is translated into the amino acid sequence according to the rules of the genetic code. The following classes of proteins are listed for illustrative purposes: developmental proteins (e.g., adhesion molecules, cyclin kinase inhibitors, Wnt family members, Pax family members, Winged helix family members, Hox family members, cytokines/lymphokines and their receptors, growth/differentiation factors and their receptors, neurotransmitters and their receptors); oncogene-encoded proteins (e.g., ABLI, BCLI, BCL2, BCL6, CBFA2, CBL, CSFIR, ERBA, ERBB, EBRB2, ETSI, ETSI, ETV6, FGR, FOS, FYN, HCR, HRAS, JUN, KRAS, LCK, LYN, MDM2, MLL, MYB, MYC, MYCLI, MYCN, NRAS, PIM I, PML, RET, SRC, TALI, TCL3, and YES); tumor suppressor proteins (e.g., APC, BRCA1, BRCA2, MADH4, MCC, NF I, NF2, RB I, TP53, and WTI); and enzymes (e.g., ACC synthases and oxidases, ACP desaturases and hydroxylases, ADP-glucose pyrophorylases, ATPases, alcohol dehydrogenases, amylases, amyloglucosidases, catalases, cellulases, chalcone synthases, chitinases, cyclooxygenases, decarboxylases, dextriinases, DNA and RNA polymerases, galactosidases, glucanases, glucose oxidases, granule-bound starch synthases, GTPases, helicases, hernicellulases, integrases, inulinases, invertases, isomerases, kinases, lactases, lipases, lipoxygenases, lysozymes, nopaline synthases, octopine synthases, pectinesterases, peroxidases, phosphatases, phospholipases, phosphorylases, phytases, plant growth regulator synthases, polygalacturonases, proteinases and peptidases, pullanases, recombinases, reverse transcriptases, RUBISCOs, topoisomerases, and xylanases).

In one aspect, the target mRNA molecule of the invention specifies the amino acid sequence of a protein associated with a pathological condition. For example, the protein may be a pathogen-associated protein (e.g., a viral protein involved in immunosuppression of the host, replication of the pathogen, transmission of the pathogen, or maintenance of the infection), or a host protein which facilitates entry of the pathogen into the host, drug metabolism by the pathogen or host, replication or integration of the pathogen's genome, establishment or spread of infection in the host, or assembly of the next generation of pathogen. Pathogens include RNA viruses such as flaviviruses, picornaviruses, rhabdoviruses, filoviruses, retroviruses, including lentiviruses, or DNA viruses such as adenoviruses, poxviruses, herpes viruses, cytomegaloviruses, hepadnaviruses or others. Additional pathogens include bacteria, fungi, helminths, schistosomes and trypanosomes. Other kinds of pathogens can include mammalian transposable elements. Alternatively, the protein may be a tumor-associated protein or an autoimmune disease-associated protein.

The target gene may be derived from or contained in any organism. The organism may be a plant, animal, protozoa, bacterium, virus or fungus. See e.g., U.S. Pat. No. 6,506,559.

### Pharmaceutical Compositions

### Formulation and Mode of Administration

In another aspect, the present invention provides for a pharmaceutical composition comprising the dsRNA of the present invention. The dsRNA sample can be suitably formulated and introduced into the environment of the cell by any means that allows for a sufficient portion of the sample to enter the cell to induce gene silencing, if it is to occur. Many formulations for dsRNA are known in the art and can be used so long as dsRNA gains entry to the target cells so that it can act. See, e.g., U.S. published patent application Nos. 2004/0203145 A1 and 2005/0054598 A1. For example, dsRNA can be formulated in buffer solutions such as phosphate buffered saline solutions, liposomes, micellar structures, and capsids. Formulations of dsRNA with cationic lipids can be used to facilitate transfection of the dsRNA into cells. For example, cationic lipids, such as lipofectin (U.S. Pat. No. 5,705,188, incorporated herein by reference), cationic glycerol derivatives, and polycationic molecules, such as polylysine (published PCT International Application WO 97/30731), can be used. Suitable lipids include Oligofectamine, Lipofectamine (Life Technologies), NC388 (Ribozyme Pharmaceuticals, Inc., Boulder, Colo.), or FuGene 6 (Roche) all of which can be used according to the manufacturer's instructions.

It can be appreciated that the method of introducing dsRNA into the environment of the cell will depend on the type of cell and the make up of its environment. For example, when the cells are found within a liquid, one preferable formulation is with a lipid formulation such as in lipofectamine and the dsRNA can be added directly to the liquid environment of the cells. In several cell culture systems, cationic lipids have been shown to enhance the bioavailability of oligonucleotides to cells in culture (Bennet, et al., 1992, Mol. Pharmacology, 41, 1023-1033). Lipid formulations can also be administered to animals such as by intravenous, intramuscular, or intraperitoneal injection, or orally or by inhalation or other methods as are known in the art. When the formulation is suitable for administration into animals such as mammals and more specifically humans, the formulation is also pharmaceutically acceptable. Pharmaceutically acceptable formulations for administering oligonucleotides are known and can be used. In some instances, it may be preferable to formulate dsRNA in a buffer or saline solution and directly inject the formulated dsRNA into cells, as in studies with oocytes. The direct injection of dsRNA duplexes may also be done. For suitable methods of introducing dsRNA see U.S. published patent application No. 2004/0203145 A1.

Suitable amounts of dsRNA must be introduced and these amounts can be determined using standard methods. Typically, effective concentrations of individual dsRNA species in the environment of a cell will be about 50 nanomolar or less 10 nanomolar or less, or compositions in which concentrations of about 1 nanomolar or less can be used. In other embodiment, methods utilize a concentration of about 200 picomolar or less and even a concentration of about 50 picomolar or less can be used in many circumstances.

The method can be carried out by addition of the dsRNA compositions to any extracellular matrix in which cells can live provided that the dsRNA composition is formulated so that a sufficient amount of the dsRNA can enter the cell to exert its effect. For example, the method is amenable for use with cells present in a liquid such as a liquid culture or cell growth media, in tissue explants, or in whole organisms, including animals, such as mammals and especially humans.

Expression of a target gene can be determined by any suitable method now known in the art or that is later developed. It can be appreciated that the method used to measure the expression of a target gene will depend upon the nature of the target gene. For example, when the target gene encodes a protein the term "expression" can refer to a protein or transcript derived from the gene. In such instances the expression of a target gene can be determined by measuring the amount of mRN A corresponding to the target gene or by measuring the amount of that protein. Protein can be measured in protein assays such as by staining or immunoblotting or, if the protein catalyzes a reaction that can be measured, by measuring reaction rates. All such methods are known in the art and can be used. Where the gene product is an RNA species expression can be measured by determining the amount of RNA corresponding to the gene product. Several specific methods for detecting gene expression are described in Example 1. The measurements can be made on cells, cell extracts, tissues, tissue extracts or any other suitable source material.

The determination of whether the expression of a target gene has been reduced can be by any suitable method that can reliably detect changes in gene expression. Typically, the determination is made by introducing into the environment of a cell undigested dsRNA such that at least a portion of that dsRNA enters the cytoplasm and then measuring the expression of the target gene. The same measurement is made on identical untreated cells and the results obtained from each measurement are compared.

The dsRNA can be formulated as a pharmaceutical composition which comprises a pharmacologically effective amount of a dsRNA and pharmaceutically acceptable carrier. A pharmacologically or therapeutically effective amount refers to that amount of a dsRNA effective to produce the intended pharmacological, therapeutic or preventive result. The phrases "pharmacologically effective amount" and "therapeutically effective amount" or simply "effective amount" refer to that amount of an RNA effective to produce the intended pharmacological, therapeutic or preventive result. For example, if a given clinical treatment is considered effective when there is at least a 20% reduction in a measurable parameter associated with a disease or disorder, a therapeutically effective amount of a drug for the treatment of that disease or disorder is the amount necessary to effect at least a 20% reduction in that parameter.

The phrase "pharmaceutically acceptable carrier" refers to a carrier for the administration of a therapeutic agent. Exemplary carriers include saline, buffered saline, dextrose, water, glycerol, ethanol, and combinations thereof. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract. The pharmaceutically acceptable carrier of the disclosed dsRNA composition may be micellar structures, such as a liposomes, capsids, capsoids, polymeric nanocapsules, or polymeric microcapsules.

Polymeric nanocapsules or microcapsules facilitate transport and release of the encapsulated or bound dsRNA into the cell. They include polymeric and monomeric materials, especially including polybutylcyanoacrylate. A summary of materials and fabrication methods has been published (see Kreuter, 1991). The polymeric materials which are formed from monomeric and/or oligomeric precursors in the polymerization/nanoparticle generation step, are per se known from the prior art, as are the molecular weights and molecular weight distribution of the polymeric material which a person skilled in the field of manufacturing nanoparticles may suitably select in accordance with the usual skill.

Suitably formulated pharmaceutical compositions of this invention can be administered by any means known in the art such as by parenteral routes, including intravenous, intramuscular, intraperitoneal, subcutaneous, transdermal, airway (aerosol), rectal, vaginal and topical (including buccal and sublingual) administration. In some embodiments, the pharmaceutical compositions are administered by intravenous or intraparenteral infusion or injection.

### Dosage

In general a suitable dosage unit of dsRNA will be in the range of 0.001 to 0.25 milligrams per kilogram body weight of the recipient per day, or in the range of 0.01 to 20 micrograms per kilogram body weight per day, or in the range of 0.01 to 10 micrograms per kilogram body weight per day, or in the range of 0.10 to 5 micrograms per kilogram body weight per day, or in the range of 0.1 to 2.5 micrograms per kilogram body weight per day. Pharmaceutical composition comprising the dsRNA can be administered once daily. However, the therapeutic agent may also be dosed in dosage units containing two, three, four, five, six or more sub-doses administered at appropriate intervals throughout the day. In that case, the dsRNA contained in each sub-dose must be correspondingly smaller in order to achieve the total daily dosage unit. The dosage unit can also be compounded for a single dose over several days, e.g., using a conventional sustained release formulation which provides sustained and consistent release of the dsRNA over a several day period. Sustained release formulations are well known in the art. In this embodiment, the dosage unit contains a corresponding multiple of the daily dose. Regardless of the formulation, the pharmaceutical composition must contain dsRNA in a quantity sufficient to inhibit expression of the target gene in the animal or human being treated. The composition can be compounded in such a way that the sum of the multiple units of dsRNA together contain a sufficient dose.

Data can be obtained from cell culture assays and animal studies to formulate a suitable dosage range for humans. The dosage of compositions of the invention lies within a range of circulating concentrations that include the ED₅₀ (as determined by known methods) with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range of the compound that includes the IC₅₀ (i.e., the concentration of the test compound which achieves a half-maximal inhibition of symptoms) as determined in cell culture. Such information can be used to more accurately determine useful doses in humans. Levels of dsRNA in plasma may be measured by standard methods, for example, by high performance liquid chromatography.

It is known that synthetic nucleic acids, such as dsRNAs, can stimulate the innate immune system and trigger a Type I Interferon response (Marques and Williams, 2005; Schlee et al., 2006). In vivo, all cell types (and receptors) are present, so there is a considerable risk of triggering the immune system, especially if the dsRNA is administered using a lipid-based delivery tool. Lipid-based delivery approaches maximizes exposure of the cargo to the endosomal compartment where Toll-like Receptors (TLRs) 3, 7, and 8 reside (Heil et al., 2004; Hornung et al., 2005; Sioud, 2005), which appear to be the primary molecules responsible for immune recognition of siRNAs. In vitro, the risk of triggering an immune response is highly dependent on the specific cell line employed, and many tissue culture lines lack the immune receptors necessary to respond to siRNAs. However, certain cell types express receptors that recognize and respond to the presence of longer dsRNAs, such as the DsiRNAs employed here, that do not respond to 21-mer siRNAs (Reynolds et al., 2006). For example, the T98G neuroblastoma cell line has been shown to respond to 27-mer but not to 21-mer dsRNAs, and it is thought that this respon¬siveness relates to recognition of blunt ends on longer RNAs by the cytoplasmic receptor RIG-I (Marques et al., 2006).

Chemical modification of an RNA duplex can block the immune response to a sequence that is normally immunostimulatory, even *in vivo* (Morrissey et al., 2005b). 2'OMe U and G bases seem to be potent in preventing immune recognition, and it is thought that this occurs via direct competitive inhibition of TLR binding unmodified RNAs by 2'OMe-containing RNAs (Judge et al., 2006; Robbins et al., 2007).

### Disease Treatment using RNAi based therapy

In a further aspect, the present invention relates to a method for treating a subject having a disease or at risk of developing a disease caused by the expression of a target gene. In this embodiment, the dsRNA can act as novel therapeutic agents for controlling one or more of cellular proliferative and/or differentiative disorders, disorders associated with bone metabolism, immune disorders, hematopoietic disorders, cardiovascular disorders, liver disorders, viral diseases, or metabolic disorders. The method comprises administering a pharmaceutical composition of the invention to the patient (e.g., human), such that expression of the target gene is silenced. Because of their high specificity, the dsRNAs of the present invention specifically target mRNAs of target genes of diseased cells and tissues.

In the prevention of disease, the target gene may be one which is required for initiation or maintenance of the disease, or which has been identified as being associated with a higher risk of contracting the disease. In the treatment of disease, the dsRNA can be brought into contact with the cells or tissue exhibiting the disease. For example, dsRNA substantially identical to all or part of a mutated gene associated with cancer, or one expressed at high levels in tumor cells, e.g. aurora kinase, may be brought into contact with or introduced into a cancerous cell or tumor gene.

Examples of cellular proliferative and/or differentiative disorders include cancer, e.g., carcinoma, sarcoma, metastatic disorders or hematopoietic neoplastic disorders, e.g., leukemias. A metastatic tumor can arise from a multitude of primary tumor types, including but not limited to those of prostate, colon, lung, breast and liver origin. As used herein, the terms "cancer," "hyperproliferative," and "neoplastic" refer to cells having the capacity for autonomous growth, i.e., an abnormal state of condition characterized by rapidly proliferating cell growth. These terms are meant to include all types of cancerous growths or oncogenic processes, metastatic tissues or malignantly transformed cells, tissues, or organs, irrespective of histopathologic type or stage of invasiveness. Proliferative disorders also include hematopoietic neoplastic disorders, including diseases involving hyperplastic/neoplatic cells of hematopoietic origin, e.g., arising from myeloid, lymphoid or erythroid lineages, or precursor cells thereof.

The present invention can also be used to treat a variety of immune disorders, in particular those associated with overexpression of a gene or expression of a mutant gene. Examples of hematopoietic disorders or diseases include, without limitation, autoimmune diseases (including, for example, diabetes mellitus, arthritis (including rheumatoid arthritis, juvenile rheumatoid arthritis, osteoarthritis, psoriatic arthritis), multiple sclerosis, encephalomyelitis, myasthenia gravis, systemic lupus erythematosis, automimmune thyroiditis, dermatitis (including atopic dermatitis and eczematous dermatitis), psoriasis, Sjogren's Syndrome, Crohn's disease, aphthous ulcer, iritis, conjunctivitis, keratoconjunctivitis, ulcerative colitis, asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyclitis, acute necrotizing hemorrhagic encephalopathy, idiopathic bilateral progressive sensorineural hearing, loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, and interstitial lung fibrosis), graft-versus-host disease, cases of transplantation, and allergy.

In another embodiment, the invention relates to a method for treating viral diseases, including but not limited to human papilloma virus, hepatitis C, hepatitis B, herpes simplex virus (HSV), HIV-AIDS, poliovirus, and smallpox virus. dsRNAs of the invention are prepared as described herein to target expressed sequences of a virus, thus ameliorating viral activity and replication. The molecules can be used in the treatment and/or diagnosis of viral infected tissue, both animal and plant. Also, such molecules can be used in the treatment of virus-associated carcinoma, such as hepatocellular cancer.

The dsRNA of the present invention can also be used to inhibit the expression of the multi-drug resistance 1 gene ("MDR1 "). "Multi-drug resistance" (MDR) broadly refers to a pattern of resistance to a variety of chemotherapeutic drugs with unrelated chemical structures and different mechanisms of action. Although the etiology of MDR is multifactorial, the overexpression of P-glycoprotein (Pgp), a membrane protein that mediates the transport of MDR drugs, remains the most common alteration underlying MDR in laboratory models (Childs and Ling, 1994). Moreover, expression of Pgp has been linked to the development of MDR in human cancer, particularly in the leukemias, lymphomas, multiple myeloma, neuroblastoma, and soft tissue sarcoma (Fan et al.). Recent studies showed that tumor cells expressing MDR-associated protein (MRP; Cole et al., 1992), lung resistance protein (LRP; Scheffer et al., 1995) and mutation of DNA topoisomerase II (Beck, 1989) also may render MDR.

### Cell culture models

In several cell culture systems, cationic lipids have been shown to enhance the bioavailability of oligonucleotides to cells in culture (Bennet, et al., 1992, Mol. Pharmacology, 41, 1023-1033). In one embodiment, the dsRNA molecules of the invention are complexed with cationic lipids for cell culture experiments. The dsRNA molecules and cationic lipid mixtures are prepared in serum-free DMEM immediately prior to addition to the cells. DMEM plus additives are warmed to room temperature (about 20-25°C) and cationic lipid is added to the final desired concentration and the solution is vortexed briefly. DsiRNA molecules are added to the final desired concentration and the solution is again vortexed briefly and incubated for 10 minutes at room temperature. In dose response experiments, the RNA/lipid complex is serially diluted into DMEM following the 10 minute incubation. The level of inhibition of gene expression by the dsRNA is 5%, 10%, 20%, 30%, 40%, 50%, 60%, 70%, 80%, 90%, 95%, or even 100% when compared to a reference control. The level of inhibition can be measured by examining the levels of protein, assaying protein activity, or assaying a phenotype. The decrease in the levels of target RNA by the dsRNA is 5%, 10%, 20%, 30%, 40%, 50%, 60%, 70%, 80%, 90%, 95%, or even 100% when compared to a reference control.

### Animal model

### DsiRNA Efficacy in a Mouse Model of Disease

Mouse models of various diseases have been described (e.g., cancer, inflammatory diseases, atherosclerosis, obesity). Accordingly, a mouse disease model is are administered a DsiRNA agent of the present invention via hydrodynamic tail vein injection. 3-4 mice per group (divided based upon specific DsiRNA agent tested) are injected with 50 ug or 200 ug of DsiRNA. Art-recognized methods that vary according to the model used are used to evaluate the DsiRNA. The level of inhibition of gene expression by the dsRNA is 5%, 10%, 20%, 30%, 40%, 50%, 60%, 70%, 80%, 90%, 95%, or even 100% when compared to a reference control. The level of inhibition can be measured by examining the levels of protein, assaying protein activity, or assaying a phenotype. The decrease in the levels of target RNA by the dsRNA is 5%, 10%, 20%, 30%, 40%, 50%, 60%, 70%, 80%, 90%, 95%, or even 100% when compared to a reference control.

The practice of the present invention employs, unless otherwise indicated, conventional techniques of chemistry, molecular biology, microbiology, recombinant DNA, genetics, immunology, cell biology, cell culture and transgenic biology, which are within the skill of the art. See, e.g., Maniatis et al., 1982, Molecular Cloning (Cold Spring Harbor Laboratory Press, Cold Spring Harbor, N.Y.); Sambrook et al., 1989, Molecular Cloning, 2nd Ed. (Cold Spring Harbor Laboratory Press, Cold Spring Harbor, N.Y.); Sambrook and Russell, 2001. Molecular Cloning, 3rd Ed. (Cold Spring Harbor Laboratory Press, Cold Spring Harbor, N.Y.); Ausubel et al., 1992), Current Protocols in Molecular Biology (John Wiley & Sons, including periodic updates); Glover, 1985, DNA Cloning (IRL Press, Oxford); Anand, 1992; Guthrie and Fink, 1991; Harlow and Lane, 1988, Antibodies, (Cold Spring Harbor Laboratory Press, Cold Spring Harbor, N.Y.); Jakoby and Pastan, 1979; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Antisense To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Riott, Essential Immunology, 6th Edition, Blackwell Scientific Publications, Oxford, 1988; Hogan et al., Manipulating the Mouse Embryo, (Cold Spring Harbor Laboratory Press, Cold Spring Harbor, N.Y., 1986); Westerfield, M., The zebrafish book. A guide for the laboratory use of zebrafish (Danio rerio), (4th Ed., Univ. of Oregon Press, Eugene, 2000).

### Other Embodiments

From the foregoing description, it will be apparent that variations and modifications may be made to the invention described herein to adopt it to various usages and conditions. Such embodiments are also within the scope of the following claims.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

In case of conflict, the present specification, including definitions, will control. In addition, the materials, methods, and examples are illustrative only and not intended to be limiting.

Reference is made to the following publications: Amarzguioui, M. and Prydz (2004). An algorithm for selection of functional siRNA sequences. Biochem Biophys Res Commun 316:1050-1058; Amarzguioui, M. et al. (2003). Tolerance for Mutation and Chemical Modifications in a siRNA. Nucleic Acids Research 31 :589-595; Bartlett, D. W. and Davis, M. E. (2006) Effect of siRNA nuclease stability on the in vitro and in vivo kinetics of siRNA-mediated gene silencing. Biotechnol Bioeng; Beale, S. E. et al. (2005). siRNA target site secondary structure predictions using local stable substructures. Nucl Acids Res 33:e3O (ppl-10); Beck, W. T. (1989). Unknotting the complexities of multidrug resistance: the involvement of DNA topoisomerases in drug action and resistance. J Natl Cancer Inst 81 :1683-1685; Boese, Q. et al. (2005). Mechanistic insights aid computational short intervening RNA design. Methods Enzymol 392:73-96;
Bondensgaard, K. et al. (2000). Structural studies of LNA:RNA duplexes by NMR: conformations and implications for RNase H activity. Chemistry 6:2687-2695; Braasch, D. A. and Corey, D. R. (2001). Locked nucleic acid (LNA): fine-tuning the recognition of DNA and RNA. Chem Biol 8:1-7; Bohula, E. A. et al. (2003). The efficacy of small interfering RNAs targeted to the type 1 insulin-like growth factor receptor (IGF1R) is influenced by secondary structure in the IGF1R transcript. J Biol Chem 278: 15991- 15997; Bridge et al. (2003). Induction of an Interferon Response by RNAi Vectors in Mammalian Cells. Nature Genetics 34:263-264; Chang et al. (1985). Gene Expression from Both Intronless and Intron-Containing Rous Sarcoma Virus Clones is Specifically Inhibited by Anti-Sense RNA. Molecular and Cellular Biology 5:2341-2348; Check (2003). RNA to the Rescue? Nature 425: 10-12; Childs, S, and V. Ling (1994). The MDR superfamily of genes and its biological implications. Important Adv Oncol, pp. 21-36; Chiu et al. (2003). siRNA Function in RNAi: A Chemical Modification Analysis. RNA 9: 1034-1048; Cole, S. P. et al. (1992). Overexpression of a transporter gene in a multidrug-resistant human lung cancer cell line. Science 258:1650-1654; Collingwood, M.A. et al. (2008). Chemical Modification Patterns Compatible with High Potency Dicer-Substrate Small Interfering RNAs. Oligonucleotides. 2008 Summer; 18(2):187-200; Crinelli, R. et al. (2002). Design and characterization of decoy oligonucleotides containing locked nucleic acids. Nucleic Acids Res 30:2435-2443; Damha, M. J., and Ogilvie, K. K. (1993). Oligoribonucleotide synthesis. The silyl-phosphoramidite method. Methods Mol Biol 20:81-114; Eckstein, F. (2000). Phosphorothioate oligodeoxynucleotides: what is their origin and what is unique about them? Antisense Nucleic Acid Drug Dev 10:117-21; Elman, J. et al. (2005). Locked nucleic acid (LNA) mediated improvements in siRNA stability and functionality. Nucleic Acids Res 33:439-447; Fan., D. et al., (1994). Reversal of multidrug resistance In Reversal of Multidrug Resistance in Cancer, Kellen, J. A., ed., CRC Press, Boca Raton, Fla., pp. 93-125; Fire, A. et al. (1998). Potent and specific genetic interference by double-stranded RNA in Caenorhabditis elegans. Nature 391:806-811; Graessmann, M. et al. (1991). Inhibition of SV40 gene expression by microinjected small antisense RNA and DNA molecules. Nucleic Acids Res 19:53-59; Gunnery, S., and Mathews, M. B. (1998) RNA binding and modulation of PKR activity. Methods 15:189-98; Hamada et al. (2002). Effects on RNA Interference in Gene Expression (RNAi) in Cultured Mammalian Cells of Mismatches and the Introduction of Chemical Modifications at the 3'-Ends of siRNAs. Antisense and Nucleic Acid Drug Development 12:301-309; Hannon (2002). RNA Interference. Nature 418:244-251; Haupenthal, J. et al. (2006). Inhibition of RNAse A family enzymes prevents degradation and loss of silencing activity of siRNAs in serum. Biochem Pharmacol, 71, 702-710; Haupenthal, J. et al. (2007). RNAse A-like enzymes in serum inhibit the anti-neoplastic activity of siRNA targeting polo-like kinase 1. Int J. Cancer*;* Herdewijn, P. (2000). Heterocyclic modifications of oligonucleotides and antisense technology. Antisense Nucleic Acid Drug Dev 10:297-310; Hohjoh, J. (2002) RNA interference (RNAi) induction with various types of synthetic oligonucleotide duplexes in cultured human cells. FEBS Lett 521:195-199; Ji, J. et al. (2003). Enhanced gene silencing by the application of multiple specific small interfering RNAs. FEBS Lett 552:247-252; Judge, A. D. et al. (2006). Design of noninflammatory synthetic siRNA mediating potent gene silencing in vivo. Mol Ther, 13, 494-505; Khvorova, A. et al. (2003). Functional siRNAs and miRNAs exhibit strand bias. Cell 115:209-216; Kim, D. H., and J. J. Rossi (2003). Coupling of RNAi-mediated target downregulation with gene replacement. Antisense Nucleic Acid Drug Dev 13:151-155; Kim, D. H. et al. (2004). Interferon induction by siRNAs and ssRNAs synthesized by phage polymerase. Nat Biotechnol 22:321-325; Kretshmer-Kazemi Far et al. (2003). The Activity of siRNA in Mammalian Cells is Related to Structural Target Accessibility: A Comparison with Antisense Oligonucleotides. Nucleic Acids Research 31: 4417-4424; Krol, J. et al. (2004). Structural features of microRNA (miRNA) precursors and their relevance to miRNA biogenesis and small interfering RNA/short hairpin RNA design. J Biol Chem 279:42230-42239; Kreuter, J. (1991) Nanoparticles-preparation and applications. In: Microcapsules and nanoparticles in medicine and pharmacy, Donbrow M., ed, CRC Press, Boca Raton, Fla., pp. 125-14; Kurreck, J. et al. (2002). Design of antisense oligonucleotides stabilized by locked nucleic acids. Nucleic Acids Res 30:1911-1918; Kurreck, J. (2003) Antisense technologies. Improvement through novel chemical modifications. Eur J Biochem, 270, 1628-1644; Levin, A. A. (1999) A review of the issues in the pharmacokinetics and toxicology of phosphorothioate antisense oligonucleotides. Biochim Biophys Acta, 1489, 69-84; Liu, et al. (2003). R2D2, a Bridge Between the Initiator and Effector Steps of the Drosophila RNAi Pathway. Science 301:1921-1925; Manoharan, M. (2002) Oligonucleotide conjugates as potential antisense drugs with improved uptake, biodistribution, targeted delivery, and mechanism of action. Antisense Nucleic Acid Drug Dev, 12, 103-128; Markovtsov, V. et al. (2000). Cooperative assembly of an hnRNP complex induced by a tissue specific homolog of polypyrimidine tract binding protein. Mol Cell Biol 20:7463-79; Marques, J. T. and Williams, B. R. (2005) Activation of the mammalian immune system by siRNAs. Nat Biotechnol, 23, 1399-1405; Marques, J. T., et al. (2006). A structural basis of discriminating between self and nonself double-stranded RNAs in mammalian cells. Nature Biotechnology, 24:559-565; Martinez, J. et al. (2002). Single-stranded antisense siRNAs guide target RNA cleavage in RNAi. Cell 110:563-574; Matteucci, M. (1997) Oligonucleotide analogues: an overview. Ciba Found Symp, 209, 5-14; discussion 14-18; McManus et al. (2002). Gene Silencing in Mammals by Small Interfering RNAs. Nature Reviews Genetics 3:737-747; Melton, D. A. (1985). Injected anti-sense RNAs specifically block messenger RNA translation in vivo. Proc Natl Acad Sci USA 82:144-148; Minks, M. A. et al. (1979). Structural requirements of double-stranded RNA for the activation of the 2'-5'-oligo(A) polymerase and protein kinase of interferon-treated HeLa cells. J Biol Chem 254:10180-10183; Morrissey, D. V. et al. (2005). Potent and persistent in vivo anti-HBV activity of chemically modified siRNAs. Nat Biotechnol, 23, 1002-1007; Napoli, C. et al. (1990). Introduction of a chimeric chalcone synthase gene into petunia results in reversible co-suppression of homologous genes in trans. Plant Cell 2:279-289; Nawrot, B. and Sipa, K. (2006) Chemical and structural diversity of siRNA molecules. Curr Top Med Chem, 6, 913-925; Ngo et al. (1998). Double-Stranded RNA Induces mRNA Degradation in Trypanosoma brucei. Proc Natl Acad Sci USA 95:14687-14692; Pellino et al. (2003). R2D2 Leads the Silencing Trigger to mRNA's Death Star. Cell 115:132-133; Persengiev, S. P. et al. (2004). Nonspecific, concentration-dependent stimulation and repression of mammalian gene expression by small interfering RNAs (siRNAs). RNA 10:12-18; Raemdonck, K. et al. (2006). In situ analysis of single-stranded and duplex siRNA integrity in living cells. Biochemistry, 45, 10614-10623; Rana, T. M. (2007) Illuminating the silence: understanding the structure and function of small RNAs. Nat Rev Mol Cell Biol, 8, 23-36; Reynolds, A. et al. (2004). Rational siRNA design for RNA interference. Nat Biotechnol 22:326-330; Robbins, M. A. et al. (2006). Stable expression of shRNAs in human CD34(+) progenitor cells can avoid induction of interferon responses to siRNAs in vitro. Nat Biotechnol, 24, 566-571; Romano, N. and G. Macino (1992). Quelling: transient inactivation of gene expression in Neurospora crassa by transformation with homologous sequences. Mol Microbiol 6:3343-53; Rusckowski, M. et al. (2000). Biodistribution and metabolism of a mixed backbone oligonucleotide (GEM 231) following single and multiple dose administration in mice. Antisense Nucleic Acid Drug Dev 10:333-345; Scheffer, G. L. et al. (1995). The drug resistance-related protein LRP is the human major vault protein. Nat Med 1:578-582; Scherer, L. and J. J. Rossi (2004). RNAi applications in mammalian cells. Biotechniques 36:557-561; Scherer et al. (2003). Approaches for the Sequence-Specific Knockdown of mRNA, Nature Biotechnology 21:1457-1465; Schlee, M. et al. (2006). siRNA and is RNA: two edges of one sword. Mol Ther, 14, 463-470; Schwarz, D. S. et al. (2003). Asymmetry in the assembly of the RNAi enzyme complex. Cell 115:199-208; Shen, L. et al. (2003). Evaluation of C-5 propynyl pyrimidine-containing oligonucleotides in vitro and in vivo. Antisense Nucleic Acid Drug Dev, 13, 129-142; Siolas, D. et al. (2005). Synthetic shRNAs as potent RNAi triggers. Nat Biotechnol 23:227-231; Sioud, M. (2005) Induction of inflammatory cytokines and interferon responses by double-stranded and single-stranded siRNAs is sequence-dependent and requires endosomal localization. J Mol Biol, 348, 1079-1090; Skipper, (2003). Elegant Tour de Force. Nature Reviews Genetics 4: 79-80; Sledz et al. (2003). Activation of the Interferon System by Short-Interfering RNAs. Nature Cell Biology 5:834-839; Stein, D. A. et al. (2001). Inhibition of Vesivirus infections in mammalian tissue culture with antisense morpholino oligomers. Antisense Nucleic Acid Drug Dev 11:317-25; Swayze, E. E. et al. (2007). Antisense oligonucleotides containing locked nucleic acid improve potency but cause significant hepatotoxicity in animals. Nucleic Acids Res, 35, 687-700; Ui-Tei, K. et al. (2004). Guidelines for the selection of highly effective siRNA sequences for mammalian and chick RNA interference. Nucleic Acids Res 32:936-948; Verma, S, and F. Eckstein (1998). Modified oligonucleotides: synthesis and strategy for users. Annu Rev Biochem 67:99-134; Vorobjev, P. E. et al. (2001). Nuclease resistance and RNase H sensitivity of oligonucleotides bridged by oligomethylenediol and oligoethylene glycol linkers. Antisense Nucleic Acid Drug Dev 11:77-85; Wahlestedt, C. et al. (2000). Potent and nontoxic antisense oligonucleotides containing locked nucleic acids. Proc Natl Acad Sci USA 97:5633-5638; Waterhouse et al. (2003). Exploring Plant Genomes by RNA-Induced Gene Silencing. Nature Reviews Genetics 4: 29-38; Wincott, F. et al. (1995). Synthesis, deprotection, analysis and purification of RNA and ribozymes. Nucleic Acids Res 23:2677-84; Wolin, S. L. and T. Cedervall (2002). The La protein. Annu Rev Biochem 71:375-403; Xu et al. (2003). Effective Small Interfering RNAs and Phosphorothioate Antisense DNAs Have Different Preferences for Target Sites in the Luciferase mRNAs. Biochemical and Biophysical Research Communications 306:712-717; Yuan, B. et al. (2004). siRNA Selection Server: an automated siRNA oligonucleotide prediction server. Nucl Acids Res 32(Webserver issue):W130-134; Zhang, H. Y. et al. (2006). RNA Interference with chemically modified siRNA. Curr Top Med Chem, 6, 893-900.

### EXAMPLES

The present invention is described by reference to the following Examples, which are offered by way of illustration and are not intended to limit the invention in any manner. Standard techniques well known in the art or the techniques specifically described below were utilized.

### Example 1. In vitro cell culture assay to Assess Nucleic Acid Inhibition of Target RNA

The dsRNAs of the invention are administered to human hepatoma (Huh7) cells and subsequently levels of targeted mRNAs are measured in the human hepatoma (Huh7) cells,to assess in vitro efficacy of the dsRNAs of the invention against the targeted transcripts.

Double stranded RNAs specific for the human target gene Hypoxanthine-Guanine Phosphoribosyl Transferase (HPRT1; GenBank Accession No. NM_000194 and GI:164518913) are tested for efficacy in human hepatoma (Huh7) cells. The preceding target gene is selected from among art-recognized "housekeeping" genes. Housekeeping genes are selected as target genes for the double purposes of assuring that target genes possessed strong and homogenous expression in human liver cells and of minimizing inter-animal expression level variability.

Exemplary nicked dsRNAs are shown in Figures 7, 8, 9 and 11, with appropriate control dsRNAs to be used as a reference for comparison. Specific nicked dsRNAs for targeting HPRT1 are shown, for example, in Figures 7, 8, 9, and 11. Specific sequences of nicked dsRNAs targeting GAPDH, LMNA, HNRPA1 and ATP1B3 may be similarly constructed for targeting their respective transcript in human liver cells.

For dsRNAs possessing modified or universal nucleotides at specific site(s), exemplary structures of dsRNAs useful for targeting HPRT1 are illustrated in Figures 19, and 20. Specific sequences of dsRNAs possessing such modified or universal nucleotides and targeting HPRT1, GAPDH, LMNA, HNRPA1 and ATP1B3 may be similarly constructed for targeting their respective transcript in human liver cells. Specific sequences of such DsiRNAs and targeting HPRT1 are, for example:

### HPRT1 DsiRNA agents:

**A.** 
5'-   AGCAUCUCCCUCACAAUUXXXXGCC-3'
3'-ACUCGUAGAGGGAGUGUUAAXXXXCGG-5'
**B.** 
5'-   AGCAUCUCCCUCACAAUUUCCGACC-3'
3'-ACUCGUAGAGXXAGUGUUAAAGGCUGG-5'
**C.**

5'- AGCAUCUCCCUCACAAUUXXXXGCC-3'

3'-ACUCGUAGAGXXAGUGUUAAXXXXCGG-5'

Legend: **Upper Case = RNA residues; X = xanthosine**

DsiRNA molecules targeted to the genomic RNA are designed and synthesized as described herein. These nucleic acid molecules can be tested *in vivo* for the ability to reduce gene expression and for cleavage activity, for example, using the following procedure. Two formats are used to test the efficacy of DsiRNAs. The reagents are tested in cell culture using, for example, human hepatoma (Huh7) cells, to determine the extent of RNA and protein inhibition. DsiRNA reagents are selected against the target as described herein. RNA inhibition is measured after delivery of these reagents by a suitable transfection agent to, for example, cultured epidermal keratinocytes. Relative amounts of target RNA are measured versus actin using real-time PCR monitoring of amplification (eg., ABI 7700 TAQMAN). A comparison is made to a mixture of oligonucleotide sequences made to unrelated targets or to a randomized DsiRNA control with the same overall length and chemistry, but randomly substituted at each position. Primary and secondary lead reagents are chosen for the target and optimization performed. After an optimal transfection agent concentration is chosen, a RNA time-course of inhibition is performed with the lead DsiRNA molecule. In addition, a cell-plating format can be used to determine RNA inhibition.

DsiRNA constructs are tested for efficacy in reducing target RNA expression, for example using the following protocol. Cells are plated approximately 24 hours before transfection in 96-well plates at 5,000-7,500 cells/well, 100 ul/well, such that at the time of transfection cells are 70-90% confluent. For transfection, annealed DsiRNAs are mixed with the transfection reagent (Lipofectamine 2000, Invitrogen) in a volume of 50 (µl/well and incubated for 20 minutes at room temperature. The DsiRNA transfection mixtures are added to cells to give a final DsiRNA concentration of 50 pM, 200 pM, or 1 nM in a volume of 150 ul. Each DsiRNA transfection mixture is added to 3 wells for triplicate DsiRNA treatments. Cells are incubated at 37°C for 24 hours in the continued presence of the DsiRNA transfection mixture. At 24 hours, RNA is prepared from each well of treated cells. The supernatants with the transfection mixtures are first removed and discarded, then the cells are lysed and RNA prepared from each well. Target RNA level or expression following treatment is evaluated by a quantitative method (*e.g.,* RT-PCR, Northern blot) for the target gene and for a control gene (*e.g.,* actin or 36B4, an RNA polymerase subunit) for normalization. Alternatively, the cells are lysed and total protein is prepared from each well. Target protein level or expression following treatement is evaluated by Western blot and the signal is quantified. Triplicate data is averaged and the standard deviations determined for each treatment. Normalized data are graphed and the percent reduction of target mRNA by dsRNAs of the invention in comparison to appropriate control dsRNAs (e.g., inverted control dsRNAs) is determined.

Thus, it can be shown that the nicked dsRNAs of the invention reduce gene expression of specific target in cells, especially in comparison to a reference dsRNA. It is expected that the nicked dsRNAs possessing a tetraloop exhibit enhanced cleavage by Dicer. Without being bound to a particular theory, enhanced cleavage by Dicer of a dsRNA of the invention results in increased levels of siRNA, compared to that of a control dsRNA. It is also expected that a nick in the dsRNA allows more chemical modifications to be utilized upon the same strand as the nick by relieving the need to have that strand function as a Dicer substrate. Thus, the nicked dsRNA reduces expression of a target gene and enhances cleavage by Dicer in comparison to a reference dsRNA.

It can also be shown that the dsRNAs of the invention containing modified or universal nucleotides reduce gene expression of specific target in cells, especially in comparison to a reference dsRNA. It is expected that such dsRNAs of the invention exhibit enhanced cleavage by Dicer, enhanced association with Ago2, and/or enhanced cleavage of target RNAs by Ago2/RISC. Without being bound by theory, enhanced cleavage by Dicer of a dsRNA of the invention results in increased levels of siRNA, compared to that of a control dsRNA. Also without being bound by theory, enhanced association with Ago2 of an siRNA of a dsRNA of the invention or enhanced cleavage by Ago2/RISC results in increased reduction of target gene expression, compared to that of a control dsRNA. Without being bound by a particular theory, enhanced cleavage by Ago2/RISC results in increased reduction of target gene expression, compared to that of a control dsRNA. Thus, the dsRNAs containing modified or universal nucleotides reduce expression of a target gene, enhance cleavage by Dicer, enhance Ago2 interaction, and/or enhance cleavage by Ago2/RISC in comparison to a reference dsRNA.

Therefore, the instant example shows that double-stranded RNAs possessing structures encompassed by the dsRNAs of the invention are robustly effective sequence-specific inhibitors *in vitro* of expression of target genes in human hepatoma (Huh7) cells.

### Example 2. In vitro assay to Assess Serum Stability

Serum stability of DsiRNA agents is assessed *via* incubation of DsiRNA agents in 50% fetal bovine serum for various periods of time (up to 24 h) at 37°C. Serum is extracted and the nucleic acids are separated on a 20% non-denaturing PAGE and visualized with Gelstar stain. Relative levels of protection from nuclease degradation are assessed for DsiRNAs (optionally with and without modifications).

Thus, it can be shown that the nicked dsRNAs of the invention reduce gene expression of a specific target, especially in comparison to a reference dsRNA. It is expected that nicked dsRNAs of the invention possessing a tetraloop have enhanced cleavage by Dicer.

### Example 3. In vivo assay of nicked dsRNA Possessing a Tetraloop

The invention provides compositions for reducing expression of a target gene in a cell, involving contacting a cell with nicked dsRNA having a tetraloop in an amount effective to reduce expression of a target gene in a subject in need thereof. The nicked dsRNAs of the invention are systemically administered to mice and subsequently levels of targeted mRNAs are measured in liver samples of treated mice. The study assesses *in vivo* efficacy of the dsRNAs of the invention against the targeted transcripts.

Nicked double stranded RNA agents specific for the following mouse target genes are tested for efficacy in mouse liver: Hypoxanthine-Guanine Phosphoribosyl Transferase (HPRT1; GenBank Accession No. NM_013556); Glyceraldehyde 3-Phosphate Dehydrogenase (GAPDH; GenBank Accession No. NM_008084); Lamin A (LMNA; GenBank Accession No. NM_019390); Heterogeneous Nuclear Ribonucleoprotein A1 (HNRPA1; GenBank Accession No. NM_010447) and ATPase, Na+/K+ Transporting, Beta 3 Polypeptide (ATP1B3; GenBank Accession No. NM_007502; two distinct locations are targeted within the ATP1B3 mRNA). The preceding target genes are selected from among art-recognized "housekeeping" genes. Housekeeping genes are selected as target genes for the double purposes of assuring that target genes possess strong and homogenous expression in mouse liver tissues and of minimizing inter-animal expression level variability.

Specific sequences of nicked dsRNAs targeting HPRT1, GAPDH, LMNA, HNRPA1 and ATP1B3 can be constructed according to the structure of the nicked dsRNAs of the invention, with such nicked dsRNAs containing any one of the following sequences on the antisense strand for targeting their respective transcripts:
**HPRT1 antisense sequence:**
   3'-UUCGGUCUGAAACAACCUAAACUUUAA-5'
**GAPDH antisense sequence:**
   3'-ACUCGUAGAGGGAGUGUUAAAGGUAGG-5'
**LMNA antisense sequence:**
   3'-CUCGAACUGAAGGUCUUCUUGUAAAUG-5'
**HNRPA1 antisense sequence:**
   3'-GUCCUGACAUAAACACUGAUUAACAUA-5'
**ATP1B3 antisense sequence:**
   3'-AUCCCUAUGUUACCAUGGAACGGUUGU-5'
**ATP1B3 antisense sequence:**
   3'-GGUCUGCCUAUAGGUGUUUAUAGCACA-5'

Legend: **Upper Case = RNA residues**

Mice (CD-1 females) weighing approximately 25 grams are purchased, housed, treated and sacrificed.

An initial dose-ranging and timepoint selection study may be performed to establish *in vivo* efficacy of the nicked dsRNAs against targeted transcripts, while also establishing the optimal nicked dsRNA dose and sample collection time for the two independent, active sequences initially targeted (HPRT1). Different doses (50 and 200 µg) of the nicked dsRNAs to be tested are dissolved in phosphate-buffered saline (PBS; 2.5 mL total volume per dose) and administered to mice as single hydrodynamic injections through the tail vein. Liver samples are collected from dosed mice at the following timepoints: 24, 48 and 72 hours, and 7 days after administration. A total of four animals per group are treated with the dsRNAs in order to assure that at least 3 animals can be evaluated at each dosage/timepoint.

The study may also be performed using the following conditions. A dose (200 µg) of the nicked dsRNA to be tested is dissolved in phosphate-buffered saline (PBS; 2.5 mL total volume per dose) and administered to mice as single hydrodynamic injections through the tail vein. Liver samples are collected from dosed mice at 24 hours after administration. A total of seven animals per group are treated with each nicked dsRNA agent.

Target mRNA levels are assessed using quantitative reverse transcriptase-polymerase chain reaction ("qRT-PCR"). cDNAs are synthesized using a mix of oligo-dT and random hexamer priming. qPCR reactions are run in triplicate. Absolute quantification is performed by extrapolation against a standard curve run against a cloned linearized amplicon target. Data are normalized using the control as 100%. Data are normalized setting the control gene expression level to be the measured target mRNA expression values for all mice not administered target mRNA-specific DsiRNA agents, which are averaged to obtain a 100% control value (*e.g.,* for mice injected with GAPDH DsiRNAs, the set of HPRT1, LMNA, HNRPA1, ATP1B3-1 and ATP1B3-3 mice are all used as negative controls to yield normalized, basal GAPDH levels. Thus, there are seven study mice and 35 control mice for each arm of the study). In evaluating the significance of the results, P values are calculated using a one-tailed, unpaired T-Test. Values below 0.05 are deemed to be statistically significant.

Thus, reduced levels of targeted mRNAs may be observed in liver samples of treated mice due to treatment with the nicked dsRNAs of the invention. Results of the studies are expected to show that nicked dsRNA agents directed against HPRT1 target sequences are effective at reducing target mRNA levels *in vivo* when administered at 50 microgram and 200 microgram concentrations, with reductions in target gene transcript expression levels of at least 10, 20, 30, 40, 50, 60, 70, 80, 90, or 100% observed at 24 hours post-administration when compared to a control that is not expected to reduce gene transcript levels.

### Example 4. In vivo assay of dsRNA containing modified or universal nucleotides

The invention provides compositions for reducing expression of a target gene in a cell, involving contacting a cell with dsRNA containing modified or universal nucleotides in an amount effective to reduce expression of a target gene in a subject in need thereof. Such dsRNAs of the invention are systemically administered to mice and, subsequently, levels of targeted mRNAs are measured in liver samples of treated mice. The study assesses *in vivo* efficacy of dsRNAs of the invention possessing modified or universal nucleotides against the targeted transcripts.

Double stranded RNA agents specific for the following mouse target genes are tested for efficacy in mouse liver: Hypoxanthine-Guanine Phosphoribosyl Transferase (HPRT1; GenBank Accession No. NM_013556); Glyceraldehyde 3-Phosphate Dehydrogenase (GAPDH; GenBank Accession No. NM_008084); Lamin A (LMNA; GenBank Accession No. NM_019390); Heterogeneous Nuclear Ribonucleoprotein A1 (HNRPA1; GenBank Accession No. NM_010447) and ATPase, Na+/K+ Transporting, Beta 3 Polypeptide (ATP1B3; GenBank Accession No. NM_007502; two distinct locations are targeted within the ATP1B3 mRNA). As in Example 3 above, the preceding target genes are selected from among art-recognized "housekeeping" genes.

Double stranded RNAs targeting HPRT1, GAPDH, LMNA, HNRPA1 and ATP1B3 may be constructed according to the structure of the dsRNAs of the invention, based on the respective sequences described above. Specific sequences of DsiRNAs containing modified or universal nucleotides according to the structure of the invention and targeting HPRT1 and GAPDH are, for example:

### HPRT1 DsiRNA agents:

**A.** 
5'-AAGCCAGACUUUGUUGGAUUXXXXAUU-3'
3'-UUCGGUCUGAAACAACCUAAXXXXUAA-5'
**B.** 
5'-AAGCCAGACUUUGUUGGAUUUGAAAUU-3'
3'-UUCGGUCUGAXXCAACCUAAACUUUAA-5'
**C.**

5'-AAGCCAGACUUUGUUGGAUUXXXXAUU-3'
3'-UUCGGUCUGAXXCAACCUAAXXXXUAA-5'

### GAPDH DsiRNA agents:

**A.** 
5'-UGAGCAUCUCCCUCACAAUUXXXXUCC-3'
3'-ACUCGUAGAGGGAGUGUUAAXXXXAGG-5'
**B.** 
5'-UGAGCAUCUCCCUCACAAUUUCCAUCC-3'
3'-ACUCGUAGAGXXAGUGUUAAAGGUAGG-5'
**C.**

5'-UGAGCAUCUCCCUCACAAUUXXXXUCC-3'
3'-ACUCGUAGAGXXAGUGUUAAXXXXAGG-5'

Legend: **Upper Case = RNA residues; X = xanthosine**

Mice (CD-1 females) weighing approximately 25 grams are purchased, housed, treated and sacrificed.

An initial dose-ranging and timepoint selection study may be performed to establish *in vivo* efficacy of the dsRNAs containing modified or universal nucleotides against targeted transcripts, while also establishing the optimal dsRNA dose and sample collection time for the two independent, active sequences initially targeted (HPRT1). Different doses (50 and 200 µg) of the dsRNAs to be tested are dissolved in phosphate-buffered saline (PBS; 2.5 mL total volume per dose) and administered to mice as single hydrodynamic injections through the tail vein. Liver samples are collected from dosed mice at the following timepoints: 24, 48 and 72 hours, and 7 days after administration. A total of four animals per group are treated with the dsRNAs in order to assure that at least 3 animals can be evaluated at each dosage/timepoint.

The study may also be performed using the following conditions. A dose (200 µg) of the dsRNA to be tested is dissolved in phosphate-buffered saline (PBS; 2.5 mL total volume per dose) and administered to mice as single hydrodynamic injections through the tail vein. Liver samples are collected from dosed mice at 24 hours after administration. A total of seven animals per group are treated with each dsRNA agent.

Target mRNA levels are assessed using quantitative reverse transcriptase-polymerase chain reaction ("qRT-PCR"). cDNAs are synthesized using a mix of oligo-dT and random hexamer priming. qPCR reactions are run in triplicate. Absolute quantification is performed by extrapolation against a standard curve run against a cloned linearized amplicon target. Data are normalized using the control as 100%. Data are normalized setting the control gene expression level to be the measured target mRNA expression values for all mice not administered target mRNA-specific DsiRNA agents, which were averaged to obtain a 100% control value (e.g., for mice injected with GAPDH DsiRNAs, the set of HPRT1, LMNA, HNRPA1, ATP1B3-1 and ATP1B3-3 mice are all used as negative controls to yield normalized, basal GAPDH levels. Thus, there are seven study mice and 35 control mice for each arm of the study). In evaluating the significance of the results, P values are calculated using a one-tailed, unpaired T-Test. Values below 0.05 are deemed to be statistically significant.

Thus, reduced levels of targeted mRNAs may be observed in liver samples of treated mice due to treatment with the dsRNAs of the invention containing modified or universal nucleotides. Results of the studies are expected to show that dsRNAs of the invention directed against HPRT1 target sequences are effective at reducing target mRNA levels in vivo when administered at 50 microgram and 200 microgram concentrations, with reductions in target gene transcript expression levels of at least 10, 20, 30, 40, 50, 60, 70, 80, 90, or 100% observed at 24 hours post-administration when compared to a control that is not expected to reduce gene transcript levels.

All patents and publications mentioned in the specification are indicative of the levels of skill of those skilled in the art to which the invention pertains. The methods and compositions described herein as presently representative of preferred embodiments are exemplary and are not intended as limitations on the scope of the invention. The present invention teaches one skilled in the art to test various combinations and/or substitutions of chemical modifications described herein toward generating nucleic acid constructs with improved activity for mediating RNAi activity. Such improved activity can comprise improved stability, improved bioavailability, and/or improved activation of cellular responses mediating RNAi. Therefore, the specific embodiments described herein are not limiting and one skilled in the art can readily appreciate that specific combinations of the modifications described herein can be tested without undue experimentation toward identifying DsiRNA molecules with improved RNAi activity.

The invention illustratively described herein suitably can be practiced in the absence of any element or elements, limitation or limitations that are not specifically disclosed herein.

In addition, where features or aspects of the invention are described in terms of Markush groups or other grouping of alternatives, those skilled in the art will recognize that the invention is also thereby described in terms of any individual member or subgroup of members of the Markush group or other group.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Embodiments of this invention are described herein, including the best mode known to the inventors for carrying out the invention.

## Claims

1. An isolated double stranded RNA (dsRNA) comprising:
i) a first strand wherein said first strand is 19-80 nucleotides in length, and
ii) a second strand, wherein said second strand is 19-35 nucleotides in length, wherein with reference to any one of Figures 1A, 1B, 5A and 5B, the first and second strands form a duplex in Region B of 15-35 base pairs in length;
wherein the first strand comprises a Region E at the 3' terminus, wherein Region E comprises a tetraloop; and
the dsRNA comprises a discontinuity between the 3' terminus of the first strand and the 5' terminus of the second strand,
wherein the the dsRNA decreases target gene expression in a mammalian cell.

2. The isolated dsRNA of claim 1, wherein the second strand forms a blunt end with the 5' terminus of the first strand or comprises a 3' overhang consisting of 1, 2, 3, or 4 nucleotides.

3. The isolated dsRNA of any one of claims 1-2, wherein the tetraloop comprises ribonucleotides, deoxyribonucleotides, modified nucleotides, or combinations thereof.

4. The isolated dsRNA of any one of claims 1-3, wherein said tetraloop has a nucleic acid sequence selected from the group consisting of UNCG, GNRA, CUUG, d(GNNA), d(GNAB), d(CNNG), d(TNCG), UUCG, GAAA, d(GTTA), and d(TTCG).

5. The isolated dsRNA of any one of claims 1-4, wherein the tetraloop is flanked at the 5' end by a nucleic acid sequence selected from the group consisting of C, CC, G, and GG, and wherein the tetraloop is flanked at the 3' end by a nucleic acid sequence that duplexes with a nucleic sequence selected from the group consisting of C, CC, G, and GG or a nucleic acid sequence selected from the group consisting of C, CC, G, and GG.

6. The isolated dsRNA of any one of claims 1-5, wherein the second strand is dephosphorylated at the 5' terminus.

7. The isolated dsRNA of any one of claims 1-6, wherein the first strand is phosphorylated at the 5' terminus.

8. The isolated dsRNA of any one of claims 1-7, wherein the second strand duplexes to a target RNA along at least 19-23 nucleotides of the length of the second strand.

9. The isolated dsRNA of any one of claims 1-8, wherein the dsRNA, when introduced into a mammalian cell, reduces target gene expression in comparison to a reference dsRNA.

10. The isolated dsRNA of any one of claims 1-9, further comprising one or more modified nucleotides.

11. The isolated dsRNA of claim 10, wherein the modified nucleotide has a modification selected from the group consisting of 2'-O-methyl, 2'-methoxyethoxy, 2' fluoro, 2'-allyl, 2'-O-[2-(methylamino)-2-oxoethyl], 4'-thio, 4'-CH₂-O-2'-bridge, 4'-(CH₂)₂-O-2'-bridge, 2'-LNA, and 2'-O-(N-methylcarbamate).

12. The isolated dsRNA of claim 10, wherein the first strand comprises a modified nucleotide in Region C distal to the tetraloop or at all positions in Region C distal to the tetraloop.

13. The isolated dsRNA of claim 10, wherein the first strand comprises a deoxyribonucleotide or modified nucleotide in Region C proximal to the tetraloop at position 1, 2, or 1 and 2 from the 3' end of the first strand in Region C.

14. The isolated dsRNA of any one of claims 10-13, wherein the modified nucleotides are ribonucleotides having a 2'-O-methyl modification.

15. The isolated dsRNA of any one of claims 1-14, wherein the dsRNA enhances cleavage by Dicer in comparison to a reference dsRNA.

16. The isolated dsRNA of claim 1, wherein said second strand is 19-23 nucleotides in length.

17. The isolated dsRNA of claim 1, wherein the first strand is 35-40 nucleotides in length and the second strand is 21-24 nucleotides in length.

18. The isolated dsRNA of claim 1, wherein said first strand is 35-39 nucleotides in length, wherein nucleotides 11-16 from the 3' terminus of the first strand form a duplex with nucleotides 1-6 from the 3' terminus of the first strand, and where nucleotides 7-10 from the 3' terminus of the first strand form a tetraloop; and wherein the second strand is 16 nucleotides shorter in length than the first strand, and where all the nucleotides beginning from the 3' terminus of the second strand form a duplex with the same number of nucleotides beginning at the 5' terminus of the first strand.

19. The isolated dsRNA of claim 1, wherein said duplex is 19-26 nucleotides in length.

## Patentansprüche

1. Isolierte doppelsträngige RNA (dsRNA), umfassend:
i) einen ersten Strang, wobei der erste Strang 19-80 Nukleotide lang ist, und
ii) einen zweiten Strang, wobei der zweite Strang 19-35 Nukleotide lang ist, wobei der erste und der zweite Strang in Bezug auf eine der Figuren 1A, 1B, 5A und 5B ein Duplex in der Region B mit einer Länge von 15-35 Basenpaaren bilden;
wobei der erste Strang eine Region E am 3'-Terminus umfasst, wobei Region E einen Tetraloop umfasst; und
die dsRNA eine Diskontinuität zwischen dem 3'-Terminus des ersten Strangs und dem 5'-Terminus des zweiten Strangs aufweist,
wobei die dsRNA die Targetgenexpression in einer Säugerzelle verringert.

2. Isolierte dsRNA nach Anspruch 1, wobei der zweite Strang ein glattes Ende mit dem 5'-Terminus des ersten Strangs bildet oder aus einem 3'-Überhang aus 1, 2, 3 oder 4 Nukleotiden besteht.

3. Isolierte dsRNA nach einem der Ansprüche 1-2, wobei der Tetraloop Ribonukleotide, Desoxyribonukleotide, modifizierte Nukleotide oder Kombinationen davon umfasst.

4. Isolierte dsRNA nach einem der Ansprüche 1-3, wobei der Tetraloop eine Nukleinsäuresequenz aufweist, ausgewählt aus der Gruppe, bestehend aus UNCG, GNRA, CUUG, d(GNNA), d(GNAB), d(CNNG), d(TNCG), UUCG, GAAA, d(GTTA) und d(TTCG).

5. Isolierte dsRNA nach einem der Ansprüche 1-4, wobei der Tetraloop am 5'-Ende durch eine Nukleinsäuresequenz flankiert ist, ausgewählt aus der Gruppe, die aus C, CC, G und GG besteht, und wobei der Tetraloop am 3'-Ende durch eine Nukleinsäuresequenz flankiert ist, die mit einer Nukleinsäuresequenz, ausgewählt aus der Gruppe, die aus C, CC, G und GG besteht, oder einer Nukleinsäuresequenz, ausgewählt aus der Gruppe, die aus C, CC, G und GG besteht, duplexiert.

6. Isolierte dsRNA nach einem der Ansprüche 1-5, wobei der zweite Strang am 5'-Terminus dephosphoryliert ist.

7. Isolierte dsRNA nach einem der Ansprüche 1-6, wobei der erste Strang am 5'-Terminus phosphoryliert ist.

8. Isolierte dsRNA nach einem der Ansprüche 1-7, wobei der zweite Strang an einer Target-RNA entlang mindestens 19-23 Nukleotiden der Länge des zweiten Strangs duplexiert.

9. Isolierte dsRNA nach einem der Ansprüche 1-8, wobei die dsRNA, wenn sie in eine Säugerzelle eingeführt wird, die Targetgenexpression im Vergleich zu einer Referenz-dsRNA reduziert.

10. Isolierte dsRNA nach einem der Ansprüche 1-9, die weiter ein oder mehrere modifizierte Nukleotide umfasst.

11. Isolierte dsRNA nach Anspruch 10, wobei das modifizierte Nukleotid eine Modifizierung aufweist, ausgewählt aus der Gruppe, bestehend aus 2'-O-Methyl, 2'-Methoxyethoxy, 2' Fluor, 2'-Allyl, 2'-O-[2-(Methylamino)-2-oxoethyl], 4'-Thio, 4'-CH₂-O-2'-Brücke, 4'-(CH₂)₂-O-2'-Brücke, 2'-LNA und 2'-O-(N-Methylcarbamat).

12. Isolierte dsRNA nach Anspruch 10, wobei der erste Strang ein modifiziertes Nukleotid in Region C distal des Tetraloops oder an allen Positionen in Region C distal des Tetraloops umfasst.

13. Isolierte dsRNA nach Anspruch 10, wobei der erste Strang ein Desoxyribonukleotid oder modifiziertes Nukleotid in Region C proximal zum Tetraloop an Position 1, 2 oder 1 und 2 vom 3'-Ende des ersten Strangs in Region C umfasst.

14. Isolierte dsRNA nach einem der Ansprüche 10-13, wobei die modifizierten Nukleotide Ribonukleotide mit einer 2'-O-Methylmodifizierung sind.

15. Isolierte dsRNA nach einem der Ansprüche 1-14, wobei die dsRNA die Spaltung durch Dicer im Vergleich zu einer Referenz-dsRNA verstärkt.

16. Isolierte dsRNA nach Anspruch 1, wobei der zweite Strang 19-23 Nukleotide lang ist.

17. Isolierte dsRNA nach Anspruch 1, wobei der erste Strang 35-40 Nukleotide lang ist und der zweite Strang 21-24 Nukleotide lang ist.

18. Isolierte dsRNA nach Anspruch 1, wobei der erste Strang 35-39 Nukleotide lang ist, wobei die Nukleotide 11-16 vom 3'-Terminus des ersten Strangs ein Duplex mit den Nukleotiden 1-6 vom 3'-Terminus des ersten Strangs bilden, und wobei die Nukleotide 7-10 vom 3'-Terminus des ersten Strangs ein Tetraloop bilden; und wobei der zweite Strang 16 Nukleotide kürzer als der erste Strang ist, und wobei alle Nukleotide, beginnend vom 3'-Terminus des zweiten Strangs, ein Duplex mit der gleichen Anzahl von Nukleotiden, beginnend am 5'-Terminus des ersten Strangs, bilden.

19. Isolierte dsRNA nach Anspruch 1, wobei das Duplex 19-26 Nukleotide lang ist.

## Revendications

1. ARN double brin (ARNds) isolé comprenant :
i) un premier brin dans lequel ledit premier brin est d'une longueur de 19 à 80 nucléotides, et
ii) un second brin, dans lequel ledit second brin est d'une longueur de 19 à 35 nucléotides,
dans lequel en référence à une quelconque des figures 1A, 1B, 5A et 5B, les premier et second brins forment un duplex dans une région B d'une longueur de 15 à 35 paires de base ;
dans lequel le premier brin comprend une région E au niveau de la terminaison 3', dans lequel une région E comprend une tétraboucle ; et
l'ARNds comprend une discontinuité entre la terminaison 3' du premier brin et la terminaison 5' du second brin,
dans lequel l'ARNds diminue une expression de gène cible dans une cellule de mammifère.

2. ARNds isolé selon la revendication 1, dans lequel le second brin forme une extrémité franche avec la terminaison 5' du premier brin ou comprend une extension en 3' composée de 1, 2, 3, ou 4 nucléotides.

3. ARNds isolé selon l'une quelconque des revendications 1 et 2, dans lequel la tétraboucle comprend des ribonucléotides, des désoxyribonucléotides, des nucléotides modifiés, ou des combinaisons de ceux-ci.

4. ARNds isolé selon l'une quelconque des revendications 1 à 3, dans lequel ladite tétraboucle présente une séquence d'acides nucléiques sélectionnée parmi le groupe constitué d'UNCG, de GNRA, de CUUG, de d(GNNA), de d(GNAB), de d(CNNG), de d(TNCG), d'UUCG, de GAAA, de d(GTTA), et de d(TTCG).

5. ARNds isolé selon l'une quelconque des revendications 1 à 4, dans lequel la tétraboucle est flanquée au niveau de l'extrémité 5' d'une séquence d'acides nucléiques sélectionnée parmi le groupe constitué de C, CC, G, et GG, et dans lequel la tétraboucle est flanquée au niveau de l'extrémité 3' d'une séquence d'acides nucléiques qui se duplexe avec une séquence nucléique sélectionnée parmi le groupe constitué de C, CC, G, et GG ou une séquence d'acides nucléiques sélectionnée parmi le groupe constitué de C, CC, G, et GG.

6. ARNds isolé selon l'une quelconque des revendications 1 à 5, dans lequel le second brin est déphosphorylé au niveau de la terminaison 5'.

7. ARNds isolé selon l'une quelconque des revendications 1 à 6, dans lequel le premier brin est phosphorylé au niveau de la terminaison 5'.

8. ARNds isolé selon l'une quelconque des revendications 1 à 7, dans lequel le second brin se duplexe à un ARN cible le long d'au moins 19 à 23 nucléotides de la longueur du second brin.

9. ARNds isolé selon l'une quelconque des revendications 1 à 8, dans lequel l'ARNds, lorsqu'introduit dans une cellule de mammifère, réduit une expression de gène cible en comparaison d'un ARNds de référence.

10. ARNds isolé selon l'une quelconque des revendications 1 à 9, comprenant en outre un ou plusieurs nucléotides modifiés.

11. ARNds isolé selon la revendication 10, dans lequel le nucléotide modifié présente une modification sélectionnée parmi le groupe constitué de 2'-O-méthyl, 2'-méthoxyéthoxy, 2' fluoro, 2'-allyl, 2'-O-[2-(méthylamino)-2-oxoéthyl], 4'-thio, pont-4'-CH₂-O-2'-, pont-4'-(CH₂)₂-O-2'-, 2'-LNA, et 2'-O-(N-méthylcarbamate).

12. ARNds isolé selon la revendication 10, dans lequel le premier brin comprend un nucléotide dans une région C distale à la tétraboucle ou à toutes les positions dans une région C distale à la tétraboucle.

13. ARNds isolé selon la revendication 10, dans lequel le premier brin comprend un désoxyribonucléotide ou un nucléotide modifié dans une région C proximale à la tétraboucle à une position 1, 2, ou 1 et 2 à partir de l'extrémité 3' du premier brin dans une région C.

14. ARNds isolé selon l'une quelconque des revendications 10 à 13, dans lequel les nucléotides modifiés sont des ribonucléotides présentant une modification 2'-O-méthyl.

15. ARNds isolé selon l'une quelconque des revendications 1 à 14, dans lequel l'ARNds augmente un clivage par Dicer en comparaison d'un ARNds de référence.

16. ARNds isolé selon la revendication 1, dans lequel ledit second brin est d'une longueur de 19 à 23 nucléotides.

17. ARNds isolé selon la revendication 1, dans lequel le premier brin est d'une longueur de 35 à 40 nucléotides et le second brin est d'une longueur de 21 à 24 nucléotides.

18. ARNds isolé selon la revendication 1, dans lequel ledit premier brin est d'une longueur de 35 à 39 nucléotides, dans lequel des nucléotides 11 à 16 de la terminaison 3' du premier brin forment un duplex avec des nucléotides 1 à 6 de la terminaison 3' du premier brin, et où des nucléotides 7 à 10 de la terminaison 3' du premier brin forment une tétraboucle ; et dans lequel le second brin est plus court de 16 nucléotides en longueur que le premier brin, et où tous les nucléotides en partant de la terminaison 3' du second brin forment un duplex avec le même nombre de nucléotides en partant de la terminaison 5' du premier brin.

19. ARNds isolé selon la revendication 1, dans lequel ledit duplex est d'une longueur de 19 à 26 nucléotides.
